# EUROPEAN PATENT SPECIFICATION

(11) **EP 3 967 696 B1**
(45) Date of publication and mention of the grant of the patent: **01.07.2026**
(21) Application number: 20802185.7
(22) Date of filing: 07.05.2020
(51) Int. Cl.: C07D 487/04, A61K 31/519, A61K 31/41, A61P 35/00

(54) **COMPOUND USED AS KINASE INHIBITOR AND APPLICATION THEREOF**
ALS KINASE-INHIBITOR VERWENDETE VERBINDUNG UND ANWENDUNG DAVON
COMPOSÉ UTILISÉ COMME INHIBITEUR DE KINASE ET SON APPLICATION

(30) Priority: 08.05.2019 CN 201910379104
(43) Date of publication of application: 16.03.2022
(73) Proprietor: TYK Medicines, Inc., Huzhou, Zhejiang 313100 (CN)
(72) Inventor: LI, Jun, Huzhou, Zhejiang 313100 (CN); NIU, Chengshan, Huzhou, Zhejiang 313100 (CN); LIANG, Apeng, Huzhou, Zhejiang 313100 (CN); WU, Yusheng, Huzhou, Zhejiang 313100 (CN)
(74) Representative: dompatent
(86) International application number: PCT/CN2020/089067
(87) International publication number: WO 2020/224626

(56) References cited:
- WO-A1-2020/114499
- CN-A- 102 596 957
- CN-A- 104 619 708
- CN-A- 107 207 514
- CN-A- 111 171 019
- DE-A1- 102005 042 742
- KATAYAMA, RYOHEI ET AL.: "The new-generation selective ROS1/NTRK inhibitor DS-6051b overcomes crizotinib resistant ROS1-G2032R mutation in preclinical models", NATURE COMMUNICATIONS, vol. 10, no. 1,, 9 August 2019 (2019-08-09), XP055751643

## Description

### TECHNICAL FIELD

The invention relates to the technical field of medicine, in particular to a compound used as a kinase inhibitor, a preparation method thereof, and a use for preparing a medicament for treating diseases mediated by kinase such as ROS1, NTRK, ALK, etc.

### BACKGROUND OF THE INVENTION

Tropomyosin receptor kinase (TRK) family belongs to transmembrane receptor tyrosine kinases (RTKs), which are involved in regulating synaptic growth and function maintenance, memory generation and development, and protecting neurons from damage, etc. in mammalian nervous system. TRK kinase is a kind of nerve growth factor receptor. Its family consists of Tropomyosin-related kinase A (TRKA), Tropomyosin-related kinase B (TRK B) and Tropomyosin-related kinase C (TRK C), which are highly homologous and encoded by NTRK 1, NTRK 2 and NTRK 3 genes, respectively. Complete TRK kinase consists of extracellular domain, transmembrane domain and intracellular domain. Like other RTKs, the extracellular domain of TRK kinase binds with corresponding ligands to form dimer, which can cause autophosphorylation of intracellular domain of TRK kinase to activate its kinase activity and further activate downstream signal transduction pathway. TRK kinase affects cell proliferation, differentiation, metabolism and apoptosis through downstream pathways such as Ras/MAPK, PI3K/AKT and PLC γ. When the NTRKs gene is fused or mutated, the extracellular receptor is altered or eliminated (Greco, A. et. al, Mol. Cell. Biol. 1995, 15, 6118; Oncogene 1998, 16, 809), but the fused or mutated TRK protein is in a highly activated kinase activity state without ligand binding, which can continuously activate the downstream signal transduction pathway, causing the regulation disorder of the downstream signal pathway of TRK kinase, inducing cell proliferation and promoting the occurrence and development of tumor. NTRKs gene fusion occurs in a variety of solid tumors in adults and children, including breast cancer, colorectal cancer, non-small cell lung cancer, papillary thyroid cancer, Spitz-like melanoma, glioma and various sarcomas, etc. In common cancers, such as non-small cell lung cancer and colorectal cancer, the incidence of NTRK gene fusion is lower, about 1%-3%, but in some rare cancers, such as infantile fibrosarcoma and secretory breast cancer, the incidence of NTRK gene fusion can reach more than 90%. The earliest TPM3-TRKA fusion protein was found in colon cancer cells. Later, more types of NTRK fusion proteins such as CD74-NTRKA, MPRIP-NTEKA, QKI-NTRKB, ETV6-NTRKC, BTB1-NTRKC, etc. were found in different clinical tumor patients such as breast cancer, non-small cell lung cancer, papillary thyroid cancer, Spitz-like melanoma, glioma, etc. Therefore, in recent years, NTRK fusion protein has become an effective anti-cancer target, and has become a hot spot in the research and development of anti-cancer drugs. With the further understanding of TRK kinase in recent years, more TRK fusion protein types and mutation types have been found (Russo, M. et. al Cancer Discovery, 2016, 6, 36; Drilon, A. et. al, Annals of Oncology, 2016, 27, 920), so it is urgent to develop new NTRK inhibitors with better activity and wider effects in clinic, so as to solve the tumor treatment problems caused by these NTRK protein fusion or mutation.

ROS1 (c-ros oncogene 1 receptor kinase) is a tyrosine protein kinase encoded by ROS1 proto-oncogene in human body. It is located on chromosome 6q22. 1 and belongs to the tyrosine kinase insulin receptor gene. It is composed of intracellular tyrosine kinase active region, transmembrane region and extracellular region, and encodes chimeric protein with tyrosine kinase activity. The basic structure consists of extracellular N-terminal ligand binding region (amino acid 1-1861), transmembrane region (amino acid 1862-1882) and intracellular C-terminal tyrosine kinase active region (amino acid 1883-2347) consisting of 464 amino acids. When ROS1 gene rearranges, the extracellular region is lost, and the transmembrane region and intracellular tyrosine kinase region are retained. The rearrangement sites mainly occur in exons 32 ~ 36 of ROS1 gene. ROS1 gene mutation mainly occurs in lung cancer patients, and the proportion of patients is 1%-2%. In NSCLC, ROS1 gene mainly fuses with SLC34A2 and CD74, and continuously activates ROS1 tyrosine kinase region and downstream JAK/STAT, PI3K/AKT, RAS/MAPK signaling pathways, thus causing tumor occurrence. It has been proved in a large number of literatures and clinically that diseases caused by ROS1 overactivation, especially cancer, can be treated by inhibiting the activity of mutated ROS1 kinase. At present, crizotinib and entrotinib are on the market for the treatment of ROS1 positive non-small cell lung cancer, both of which belong to the first generation of small molecule ROS1 inhibitors. However, during the treatment of crizotinib or entrotinib, drug resistance and disease progression will occur in about 15 months. Among drug-resistant patients, the most common drug-resistant mutation is solvent front mutation such as G2032R. For drug-resistant patients, there are no therapeutic drugs on the market at present. Therefore, it is urgent to develop new inhibitors against ROS1, especially new ROS1 inhibitors that are resistant to the first generation of ROS1 inhibitors such as crizotinib or entrotinib, for clinical treatment.

2-5% of NSCLC patients are anaplastic lymphoma kinase (ALK) rearrangements, a receptor protein tyrosine phosphokinase in the insulin receptor superfamily. At first, people found ALK in the form of an activated fusion oncogene in anaplastic large cell lymphoma, and then continuous studies found the fusion form of ALK in various cancers, including systemic dysplasia, inflammatory myofibroblastic carcinoma, non-small cell lung cancer, etc. The mutation and abnormal activity of ALK in a variety of cancers have made it a drug target for the treatment of ALK-positive cancers. At present, there are many ALK kinase inhibitors on the market. With the clinical application of these drugs, patients will have drug resistance mutations. If G1202R and other drug resistance mutations occur, these drugs will lose their efficacy.

In recent years, with the further understanding of ROS1, NTRK, ALK and other kinases, and the increase of clinical drug-resistant patients, it is urgent to develop new tyrosine kinase inhibitors with better activity and wider effects in clinic, so as to solve the treatment problems of tumors caused by the fusion or mutation of ROS1, NTRK, ALK and other kinases.

DE 10 2005 042 742 A1 describes new inhibitors for kinases which are substituted imidazo[1,2b]pyridazines, methods for producing such inhibitors, intermediates for producing such inhibitors and uses of such inhibitors.

CN 107207514 A discloses novel chemical compounds. The compounds can be used as an inhibitor of Trk and are useful in the treatment of pain, cancer, inflammation, neurodegenerative disease and certain infectious diseases.

CN 102596957 A discloses inhibitors of Trk kinases and are useful in the treatment of diseases which can be treated with a Trk kinase inhibitor such as pain, cancer, inflammation, neurodegenerative diseases and certain infectious diseases.

CN 104619708 A discloses compounds and their use for treating immunological disorders, cardiovascular disease, cancer, and other diseases, disorders or conditions associated with PI3Kdelta.

### SUMMARY OF THE INVENTION

The invention is defined in the claims. The invention provides a novel, efficient and broad-spectrum kinase inhibitor capable of simultaneously acting on carcinogenic proteins such as NTRK, ALK and/or ROS1.

In the first aspect of the invention, a compound is provided as defined in claim 1:

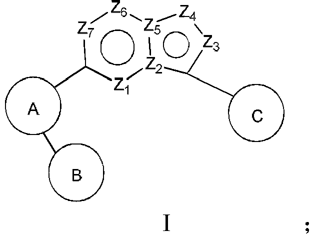

Preferred embodiments are defined in the dependent claims.

Disclosed, with the coumpounds of claim 6 being according to the invention, are the compound, or the tautomer, or the mesomer, the racemate and the mixture of mesomer and racemate, or the enantiomer, the diastereomer and the mixture of enantiomer and diastereomer, or the pharmaceutically acceptable salt, or the deuterated compound thereof, wherein the compound represented by formula I is optionally selected from the following compounds:

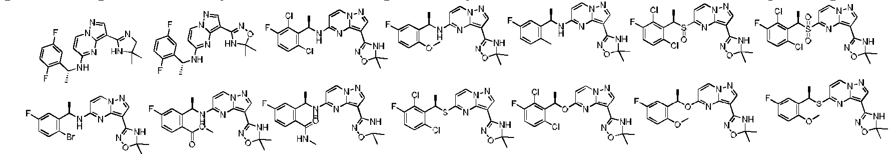

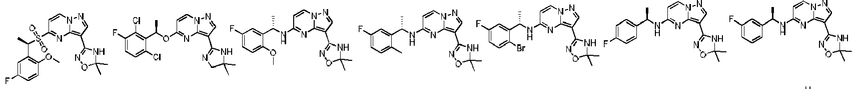

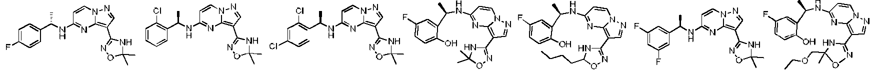

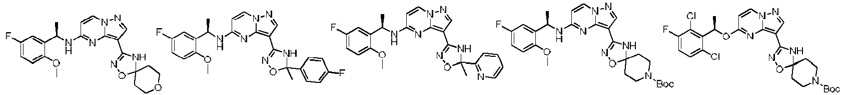

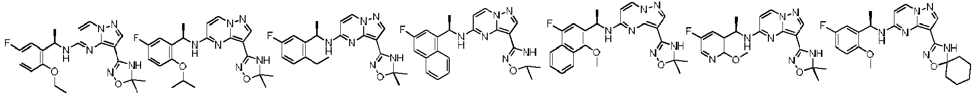

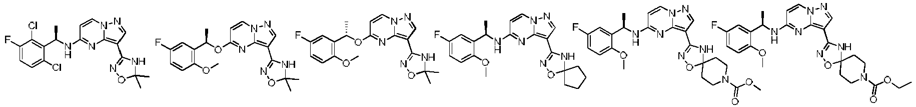

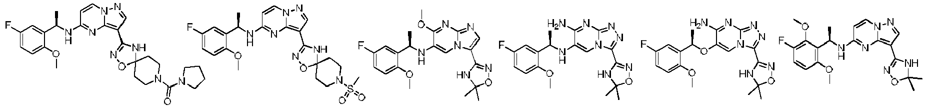

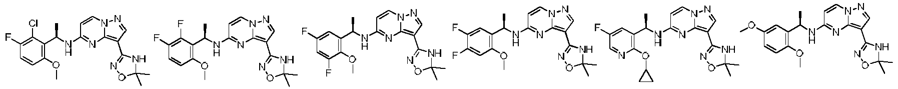

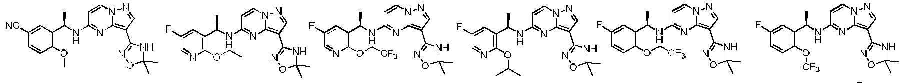

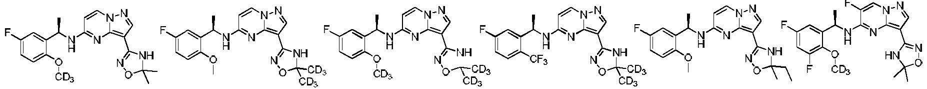

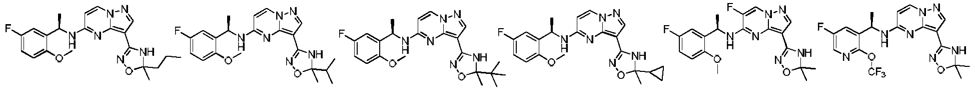

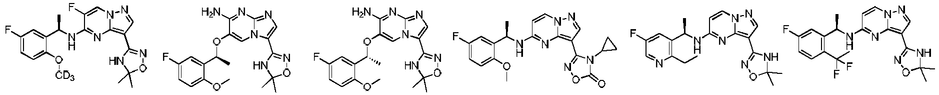

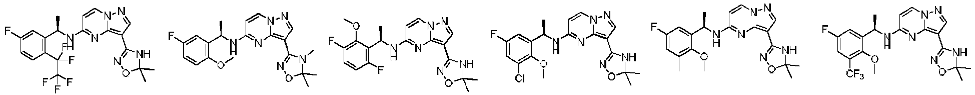

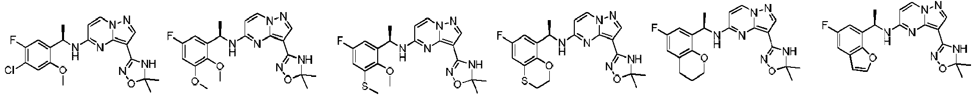

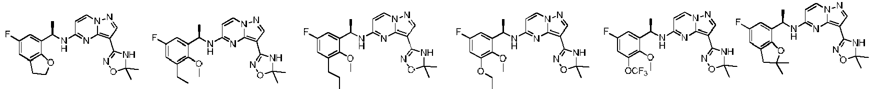

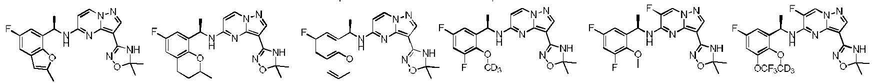

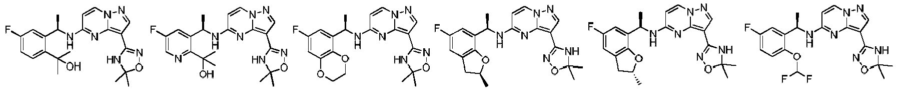

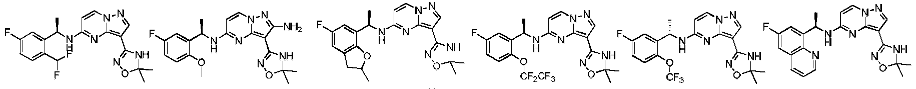

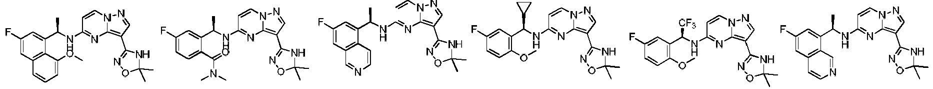

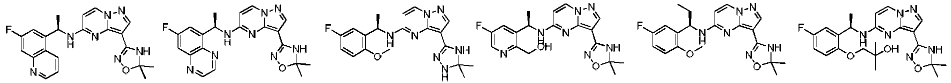

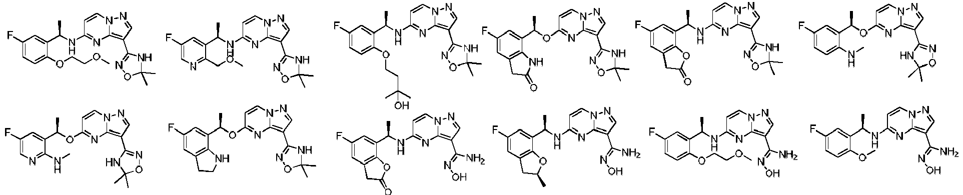

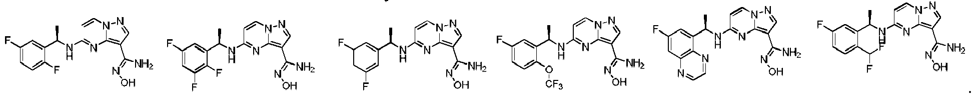

In another preferred embodiment, the compound represented by formula I is selected from the compound shown in the example of the present invention.

In the second aspect of the invention, a pharmaceutically acceptable salt of the compound of formula I is provided, wherein the pharmaceutically acceptable salt is an inorganic acid salt or an organic acid salt, wherein the inorganic acid salt is selected from the group consisting of hydrochloride, hydrobromide, hydroiodate, sulfate, bisulfate, nitrate, phosphate and acid phosphate; the organic acid salt is selected from formate, acetate, trifluoroacetate, propionate, pyruvate, hydroxyacetate, oxalate, malonate, fumarate, maleate, lactate, malate, citrate, tartrate, methanesulfonate, ethanesulfonate, hydroxyethanesulfonate, benzenesulfonate, salicylate, picrate, glutamate, ascorbate, camphorate, camphor sulfonate.

In the third aspect of the invention, a pharmaceutical composition comprising a therapeutically effective amount of the compound of the first aspect, or the tautomer, or the mesomer, the racemate and the mixture of mesomer and racemate, or the enantiomer, the diastereomer and the mixture of enantiomer and diastereomer, or the pharmaceutically acceptable salt, or the deuterated compound thereof, and one or more pharmaceutically acceptable carriers, diluents or excipients is provided.

In the fourth aspect of the invention, a use of the compound of the first aspect, or the tautomer, or the mesomer, the racemate and the mixture of mesomer and racemate, or the enantiomer, the diastereomer and the mixture of enantiomer and diastereomer, or the pharmaceutically acceptable salt, or the deuterated compound thereof or the pharmaceutical composition comprising the compound represented by formula I in the preparation of a medicament for preventing and/or treating the diseases related to pathological characteristics mediated by ROS1, NTRK, and ALK, etc is provided.

In another preferred embodiment, the diseases related to pathological characteristics mediated by ROS1, NTRK, and ALK, etc. include cancer, sarcoma and pain.

In another preferred embodiment, the cancer is any one of breast cancer, cervical cancer, colon cancer, lung cancer, stomach cancer, rectal cancer, pancreatic cancer, brain cancer, skin cancer, oral cancer, prostate cancer, bone cancer, kidney cancer, ovarian cancer, bladder cancer, liver cancer, fallopian tumor, peritoneal tumor, melanoma, glioma, glioblastoma, head and neck cancer, mastoid nephroma, leukemia, lymphoma, myeloma and thyroid tumor.

The pharmaceutical composition provided by the invention can be made into a suitable dosage form for application. These dosage forms include those suitable for oral, rectal, topical, intraoral, and other non-parenteral administration (for example, subcutaneous, intramuscular and intravenous, etc.)

The pharmaceutical composition of the present invention can be formulated, quantified and administered in a manner consistent with medical practice specifications. The "effective amount" of the compound to be administered depends on factors such as the specific condition to be treated, the individual to be treated, the cause of the condition, the target of the drug and the manner of administration.

### DETAILED DESCRIPTION OF THE INVENTION

After extensive and in-depth research, the inventor of the present invention accidentally discovered a new compound having excellent inhibitory activity against ROS1, NTRK and ALK and their drug-resistant mutations, especially against drug-resistant mutations, and having better pharmacodynamics and pharmacokinetic properties and lower toxic and side effects. It has the potential to be developed into an effective drug for drug-resistant patients that is urgently needed in clinical practice.

### Term

Unless otherwise stated, the following terms used in this application (including the specification and claims) have the definitions given below.

"Alkyl" refers to a monovalent linear or branched saturated hydrocarbon group containing 1 to 12 carbon atoms composed only of carbon and hydrogen atoms. "Alkyl" is preferably an alkyl of 1 to 6 carbon atoms, that is, a C₁-C₆ alkyl, more preferably a C₁-C₄ alkyl. Examples of alkyl include but are not limited to methyl, ethyl, propyl, isopropyl, isobutyl, sec-butyl, tert-butyl, amyl, n-hexyl, octyl and dodecyl etc. In the present invention, the alkyl is also intended to include a deuterated alkyl, examples of which include, but are not limited to CD₃, CD₂CD₃ and CD₂CD₂CD₃.

"Alkoxy" refers to the formula -OR or -R'-OR, wherein R is an alkyl as defined herein, and R' is an alkylene. Examples of alkoxy include but are not limited to methoxy, ethoxy, isopropoxy, tert-butoxy, -CH₂O-CH₃, -CH₂CH₂-O-CH₃, -CH₂-O-CH₂CH₃ and the like.

"Halogen (Halo)" refers to fluorine, chlorine, bromine or iodine substituent.

"Haloalkyl" refers to an alkyl as defined herein in which one or more hydrogen is replaced by the same or different halogens. The "haloalkyl" is preferably a halogenated C₁-C₆ alkyl, more preferably a halogenated C₁-C₄ alkyl. Examples of the halogenated alkyl include -CH₂Cl, -CH₂CF₃, -CH₂CCl₃ and perfluoroalkyl (e.g., -CF₃-, -CF₂CF₃), etc.

"Haloalkoxy" refers to the formula -OR, wherein, R is a halogenated alkyl as defined herein. Examples of haloalkoxy include but are not limited to trifluoromethoxy, difluoromethoxy, and 2, 2, 2-trifluoroethoxy, etc.

"Cycloalkyl" refers to a monovalent saturated carbocyclic group consisting of a mono- or bicyclic ring having 3-12 (C₃-C₁₂), preferably 3-10(C₃-C₁₀), more preferably 3-6 ring atoms (C₃-C₆). The cycloalkyl may optionally be substituted with one or more substituents, wherein each substituent is independently a hydroxyl, alkyl, alkoxy, halogen, haloalkyl, amino, monoalkylamino or dialkylamino. Examples of cycloalkyl include but are not limited to cyclopropyl, cyclobutyl, cyclopentyl, cyclohexyl and cycloheptyl, etc.

"Cycloalkoxy" refers to the formula -OR, wherein R is a cycloalkyl as defined herein. Examples of cycloalkyloxy include cyclopropyloxy, cyclobutyloxy, cyclopentyloxy and cyclohexyloxy, etc.

"Acyl" refers to the formula -C(O)R, wherein R is an alkyl or alkylamino as defined herein. "Acyl" is preferably -C(O)C₁-C₆ alkyl, -C(O)NH₂, -C(O)NHC₁-C₆ alkyl, -C(O)N(C₁-C₆ alkyl)₂, more preferably -C(O)C₁-C₃ alkyl, -C(O)NH₂, -C(O)NHC₁-C₃ alkyl, -C(O)N(C₁-C₃ alkyl)₂, examples of acyl include acetyl, n-propionyl, isopropionyl, n-butyryl, isobutyryl, tert-butyryl, -C(O)NH₂, -C(O)NHCH₃ and -C(O)N(CH)₃)₂, etc.

"Alkylamino" refers to the formula -NRaRb, wherein Ra and Rb are the same or different, and is each independently H or alkyl as defined herein.

Ester refers to the formula -C(O)OR, wherein R is an alkyl as defined herein. The ester is preferably -C(O)OC₁-C₆ alkyl, more preferably -C(O)OC₁-C₄ alkyl, examples of ester include -C(O)OMe, -C(O)OEt and -C(O)O-C(CH₃)₃, etc.

Sulfonyl refers to formula -S(O)₂-R, wherein R is an alkyl as defined herein. The sulfonyl is preferably -S(O)₂-C₁-C₆ alkyl, examplarily comprises -S(O)₂-Me and -S(O)₂-Et, etc.

Sulfinyl refers to the formula -SO-R, wherein R is an alkyl as defined herein. The sulfinyl is preferably -SO-C₁-C₆ alkyl, examplarily comprises -SO-Me and -SO-Et, etc.

"Alkylthio" refers to the formula -SRa, wherein Ra is H or alkyl as defined herein.

"Cycloalkylamino" refers to the formula -NRaRb, wherein Ra is H, an alkyl as defined herein or a cycloalkyl as defined herein, and Rb is a cycloalkyl as defined herein; or Ra and Rb together with the N atoms attached to themform a 3-6-membered N-containing heterocyclic group, such as tetrahydropyrrolyl.

"Heterocyclyl" refers to a completely saturated or partially unsaturated cyclic group (including but not limited to, for example, 3-7-membered monocyclic, 6-11-membered bicyclic, or 8-16-membered tricyclic system) in which at least one heteroatom is present in a ring having at least one carbon atom. Each heteroatom-containing heterocyclic ring has 1, 2, 3, or 4 heteroatoms selected from the group consisting of nitrogen, oxygen, or sulfur atoms, wherein the nitrogen or sulfur atoms may be oxidized or the nitrogen atoms may be quaternized. Heterocycloalkyl refer to completely saturated heterocyclyl. Heterocyclyl can be attached to the residue of any heteroatom or carbon atom of the ring or ring molecule. Typical monocyclic heterocyclyls include, but are not limited to azetidinyl, pyrrolidyl, oxetanyl, pyrazolinyl, imidazolinyl, imidazolidinyl, oxazolidinyl, isoxazolidinyl, thiazolidinyl, isothiazolidinyl, tetrahydrofuryl, piperidyl, piperazinyl, 2-oxoppiperazinyl, 2-oxo piperidyl, 2-oxopyrrolidyl, hexahydroazepinyl, 4-piperidone, tetrahydropyranyl, morpholinyl, thiomorpholinyl, thiomorpholinylsulfoxide, thiomorpholinylsulfone, 1,3-dioxane and tetrahydro-1,1-dioxythienyl, etc.. A polycyclic heterocyclyl includes spiro, fused, and bridged heterocyclyls. The spiro, fused, and bridged heterocyclyls involved are optionally connected with other groups by single bond, or are further fused with other cycloalkyl, heterocyclyl, aryl and heteroaryl by any two or more atoms of the ring.

"Aryl" refers to aromatic cyclic hydrocarbon groups with 1-5 rings, especially monocyclic and bicyclic groups. Any aromatic ring having two or more aromatic rings (bicyclic, etc.), the aromatic rings of aryl may be connected by single bond (such as biphenyl) or fused (such as naphthalene, anthracene, etc.). The aryl is preferably a C6-C12 aryl and refers to an aromatic cyclic hydrocarbon group containing 6, 7, 8, 9, 10, 11 or 12 ring carbon atoms. Examples of aryl (especially monocyclic and bicyclic groups) include but are not limited to phenyl, biphenyl or naphthyl. Aryl can be fused with heterocyclic groups through a single bond or any two adjacent ring C atoms, for example: benzotetrahydrofuranyl, benzotetrahydropyranyl, benzodioxanyl and 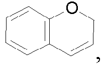 etc.

"Heteroaryl" refers to monocyclic, bicyclic, or tricyclic aromatic ring containing 5 to 12 ring atoms (5-12 membered), and containing at least 1 (e.g. 1, 2 or 3) ring heteroatoms selected from N, O or S, and the remaining ring atoms are C. It should be clear that the connection point of heteroaryl should be located on the heteroaromatic ring. Heteroaryl is preferred to have 5-8 ring atoms (5-8 membered), more preferably have 5-6 ring atoms (5-6 membered). Examples of heteroaryl include but are not limited to imidazolyl, aoxazolyl, isoxazolyl, thiazolyl, isothiazolyl, oxadiazolyl, thiadiazolyl, pyrazinyl, thienyl, furanyl, pyranyl, pyridyl, pyrrolyl, pyrazolyl, pyrimidinyl, quinolinyl, isoquinolinyl, benzofuranyl, benzothienyl, benzothiopyranyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinazinyl, naphthyridinyl, pterridinyl, carbazolyl, azepinyl, diazepinyl and acridinyl, etc.

"Polysubstituted" means being substituted by two or more substituents.

In the present invention, unless other stated, the alkyl, alkoxy, cycloalkyl, heterocyclyl, aryl, heteroaryl and other groups include substituted alkyl, alkoxy, cycloalkyl, heterocyclyl, aryl, heteroaryl, etc., the substituents such as (but not limited to) halogen, hydroxyl, cyano , acyl, sulfonyl, ester, sulfinyl, alkyl, cycloalkyl, heterocyclyl, aryl, heteroaryl, acyl, ester, etc.

"Deuterated compound" refers to the compound obtained by replacing one hydrogen atom (H) or multiple hydrogen atoms (H) with deuterium atoms (D) in a compound.

### Active ingredient

As use herein, the terms "compounds of the invention" or "active ingredients of the invention" are used interchangeably, and refers to a compound of formula I, or the pharmaceutically acceptable salt, or the deuterated compound thereof as defined in claim 1.

Disclosed, but only claimed as far as covered by claim 1 are the compound of formula I, or the tautomer, or the mesomer, the racemate and the mixture of mesomer and racemate, or the enantiomer, the diastereomer and the mixture of enantiomer and diastereomer, or the pharmaceutically acceptable salt, or the deuterated compound thereof has the following structure, 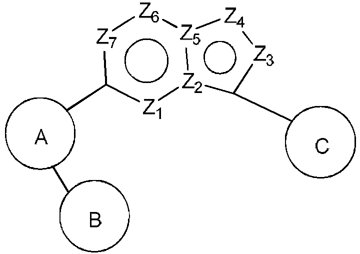 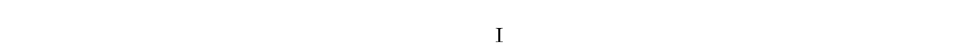 wherein, A, B, C, Z₁, Z₂, Z₃, Z₄, Z₅, Z₆ and Z₇ are as defined above.

Preferably, the compound of formula I, or the tautomer, or the mesomer, the racemate and the mixture of mesomer and racemate, or the enantiomer, the diastereomer and the mixture of enantiomer and diastereomer, or the pharmaceutically acceptable salt, or the deuterated compound thereof has a structure represented by formula III, 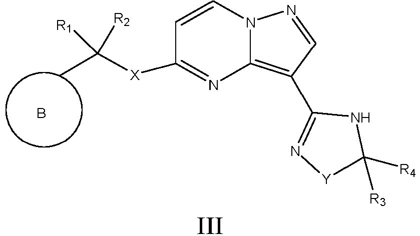 wherein,
B, X, Y, R₁, R₂, R₃ and R₄ are as defined above.

The salt that the compound in the present invention may be formed are also within the scope of the present invention. Unless otherwise stated, the compound in the present invention is understood to include its salt. The term "salt" as used herein refers to a salt formed in the form of acid or base from inorganic or organic acid and base. Further, when the compound in the present invention contains a base fragment which includes, but is not limited to pyridine or imidazole, when contains an acid fragment which includes, but is not limited to carboxylic acid. The zwitter-ion that may form "inner salt" is included within the scope of the term "salt". Pharmaceutically acceptable (i.e., non-toxic, physiologically acceptable) salt is preferred, although other salts are also useful and may be used, for example, in the separation or purification steps of the preparation process. The compound of the present invention may form a salt, for example, compound I is reacted with a certain amount (such as an equivalent amount) of an acid or base, and precipitated in a medium, or freeze-dried in aqueous solution.

Base fragment contained in the compounds in the present invention includes but is not limited to amines or pyridine or imidazole rings, which may form salt with organic or inorganic acid. Typical acids that can form salts include hydrochloride, hydrobromide, hydroiodate, sulfate, bisulfate, nitrate, phosphate and acid phosphate; the organic acid salt is selected from formate, acetate, trifluoroacetate, propionate, pyruvate, hydroxyacetate, oxalate, malonate, fumarate, maleate, lactate, malate, citrate, tartrate, methanesulfonate, ethane sulfonate, hydroxyethanesulfonate, benzenesulfonate, salicylate, picrate, glutamate, ascorbate, camphorate, camphor sulfonate, etc.

Acidic fragments that may be contained in some compounds of the invention includes, but not limited to carboxylic acid, which may form salts with various organic or inorganic bases. Salt formed by typical base includes ammonium salt, alkali metal salt (such as sodium, lithium and potassium salts), alkaline earth metal salt (such as calcium and magnesium salts), and salt formed by organic bases (such as organic amines), such as benzathine, dicyclohexylamine, hydrabamine ( salt formed with N,N- bis (dehydroabietyl) ethylenediamine), *N*-methyl-D-glucanamine, N-methyl-D-glucoamide, tert-butyllamine, and the salt formed with amino acids such as arginine, lysine, etc.. Basic nitrogen-containing groups can form quaternary ammonium salts with halides, such as small molecular alkyl halides (such as chlorides, bromides and iodides of methyl, ethyl, propyl and butyl), dialkyl sulfate (such as dimethyl, diethyl, dibutyl, and dipentyl sulfates), long chain halides (such as chlorides, bromides and iodides of decyl, dodecyl, tetradecyl, and tetradecyl), aralkyl halides (such as bromides of benzyl and phenyl), etc..

The prodrug and solvate of the compound in the present invention are also included within the scope of the present invention. The term "prodrug" herein refers to a compound resulting from the chemical transformation of a metabolic or chemical process to produce a compound, salt, or solvate in the present invention for the treatment of an associated disease. The compounds of the invention include solvates such as hydrates.

Compound, salt or solvate in the present invention, may be present in tautomeric forms such as amide and imino ether. All of these tautomers are part of the present invention.

Stereisomers of all compounds (e.g., those asymmetric carbon atoms that may be present due to various substitutions), include their enantiomeric forms and non-enantiomed forms, all belong to the protection scope of the present invention. The independent stereoisomer in the present invention may not coexist with other isomers (e.g., as a pure or substantially pure optical isomer with special activity), or may be a mixture (e.g., racemate), or a mixture formed with all other stereoisomers or a part thereof. The chiral center of the present invention has two configurations of S or R, which is defined by International Union of Pure and Applied Chemistry (IUPAC) in 1974. The racemization form can be solved by physical methods, such as fractional crystallization, or separation crystallization by derivation into diastereomers, or separation by chiral column chromatography. Individual optical isomer can be obtained from racemate by appropriate methods, including but not limited to conventional methods, such as recrystallization after salting with optically active acids.

Weight content of compound in the present invention obtained by preparation, separation and purification in turn is equal to or greater than 90%, such as equal to or greater than 95%, equal to or greater than 99% ("very pure" compound), and listed in the description of the text. In addition, the "very pure" compound of the present invention is also part of the present invention.

All configuration isomers of the compound of the present invention are within the scope, whether in mixture, pure or very pure form. The definition of the compound of the present invention comprises cis (Z) and trans (E) olefin isomers, and cis and trans isomers of carbocyclic and heterocyclic.

In the entire specification, the groups and substituents can be selected to provide stable fragments and compounds.

Specific functional groups and chemical term definitions are described as follows in detail. For the purposes of the present invention, the chemical elements are consistent with Periodic Table of the Elements, CAS version, Handbook of Chemistry and Physics, 75th Ed. The definition of a particular functional group is also described therein. In addition, the basic principles of Organic Chemistry as well as specific functional groups and reactivity described in "Organic Chemistry", Thomas Sorrell, University Science Books, Sausalito: 1999, the entire content of which is incorporated herein by reference.

Some compounds of the present invention may exist in specific geometric or stereoisomer forms. The present invention covers all compounds, including their cis and trans isomers, R and S enantiomers, diastereomers, (D) type isomers, (L) type isomers, racemic mixtures and other mixtures. In addition, asymmetric carbon atom can represent substituent, such as alkyl. All isomers and mixtures thereof are included in the present invention.

According to the invention, mixtures of isomers may contain a variety ratio of isomers. For example, mixtures with only two isomers may have the following combinations: 50:50, 60:40, 70:30, 80:20, 90:10, 95:5, 96:4, 97:3, 98:2, 99:1, or 100:0, all ratios of the isomers are within the scope of the present invention. Similar ratios readily understood by those of ordinary skill in the art and ratios for mixtures of more complex isomers are also within the scope of the present invention.

The invention also includes isotope labeled compounds, which are disclosed herein equivalent to the original compounds. However, in practice, it usually occurs when one or more atoms are replaced by atoms with a different atomic weight or mass number. Examples of compound isotopes that may be listed in the present invention include hydrogen, carbon, nitrogen, oxygen, phosphorus, sulfur, fluorine and chlorine isotopes, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl. The compound, or enantiomer, diastereomer, isomer, or pharmaceutically acceptable salt or solvate, wherein the compound containing isotopes or other isotope atoms of above compound are all within the scope of the invention. Some isotope-labeled compounds in the present invention, such as the radioactive isotopes of ³H and ¹⁴C, are also included and are useful in experiments on the tissue distribution of drugs and substrates. Tritium (³H) and Carbon-14 (¹⁴C), which are relatively easy to prepare and detect, are the preferred choice. In addition, heavier isotope substitutions such as deuterium, i.e. ²H, have advantages in certain therapies due to their good metabolic stability, such as increased half-life or reduced dosage in vivo, and thus may be preferred in certain situations. Isotope-labeled compounds can be prepared by conventional methods through substituting readily available isotope-labeled reagents for non-isotopic reagents, and can be prepared using the disclosed scheme shown in the Example.

If the synthesis of a specific enantiomer of the compound of the invention is to be designed, it can be prepared by asymmetric synthesis, or derivatized with chiral adjuvant, separating the resulting diastereomeric mixture and removing the chiral adjuvant to obtain a pure enantiomer. In addition, if a molecule contains a basic functional group, such as an amino acid, or an acidic functional group, such as a carboxyl group, a diastereomer salt can be formed with a suitable optically active acids or bases, which can be separated by conventional means, such as crystallization or chromatography, to obtain a pure enantiomer.

As described herein, the compound in the present invention may be substituted with any number of substituents or functional groups to extend its scope. In general, whether the term "substituted" appears before or after the term "optional", the general formula that includes substituents in the compound of the present invention means the substitution of a specified structural substituent for a hydrogen radical. When multiple locations in a particular structure are replaced by multiple specific substituents, each location of the substituents can be the same or different. The term "substituted" as used herein includes all substitution that allows organic compounds to be substituted. Broadly speaking, the allowable substituents include non-cyclic, cyclic, branched, non-branched, carbocyclic and heterocyclic, aromatic ring and non-aromatic organic compounds. In the present invention, for example, heteroatom nitrogen, its valence state may be supplemented by a hydrogen substituent or by any permitted organic compound described above. Furthermore, the invention is unintentionally limited to the substituted organic compounds. The present invention considers that a combination of substituents and variable groups is good for the treatment of diseases in the form of stable compounds. The term "stable" herein refers to a stable compound which is sufficient for maintaining the integrity of the compound structure within a sufficiently long time, preferably in a sufficiently long time, which is hereby used for the above purposes.

The metabolites of the compounds of the present application and their pharmaceutically acceptable salts, and prodrugs that can be converted into the compounds of the present application and their pharmaceutically acceptable salts thereof in vivo, also included in the claims.

### Preparation method

The compound of the invention may be conveniently prepared by optionally combining the various synthetic methods described in this specification or known in the art, such a combination may be easily performed by a skilled person in the art to which the invention belongs.

Generally, in the preparation process, each reaction is usually carried out in an inert solvent at -60 °C to 100 °C, preferably -60 °C to 80 °C. The reaction time is usually 0.1-60 hours, preferably 0.5-48 hours.

The preferred synthetic route is as follows: 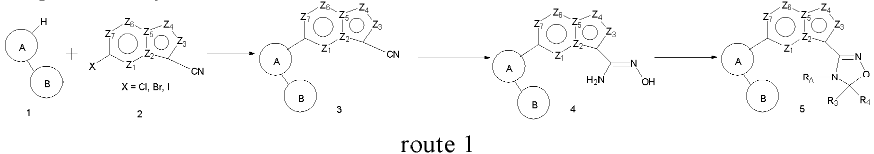 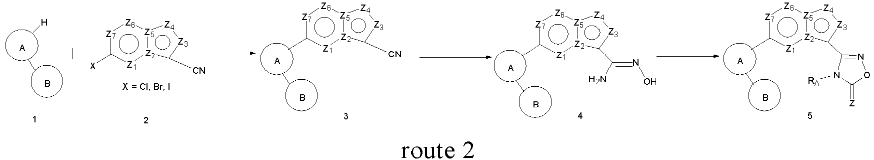 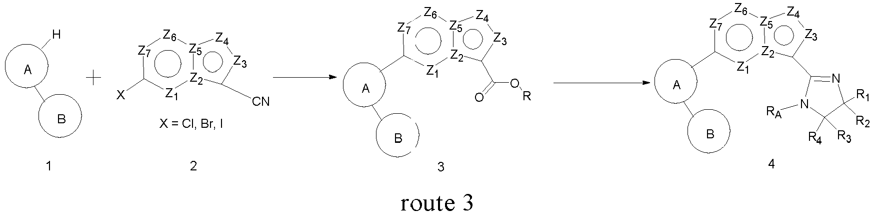
wherein Z is O; R is C1-C6 alkyl;
A, B, C, Z₁, Z₂, Z₃, Z₄, Z₅, Z₆, Z₇, R₃, R₄ and R_{A} are as defined above;
wherein, in route 1: (1) compound 1 and compound 2 undergone nucleophilic substitution reaction in an inert solvent (such as ethanol and methanol) under the action of base (such as sodium carbonate, potassium carbonate, sodium hydroxide, triethylamine and pyridine, etc.) to give compound 3; (2) compound 3 reacted with hydroxylamine hydrochloride in an inert solvent (such as ethanol and methanol) under the action of a base (such as sodium carbonate, potassium carbonate, sodium hydroxide, triethylamine, pyridine, etc.) to give compound 4; (3) compound 4 reacted with dimethoxy acetonide in an inert solvent (e.g. 1, 2-dichloroethane and/or glacial acetic acid) to give the final product 5.
in route 2: (1) compound 1 and compound 2 undergone nucleophilic substitution reaction in an inert solvent (such as ethanol and methanol) under the action of base (such as sodium carbonate, potassium carbonate, sodium hydroxide, triethylamine and pyridine, etc.) to give compound 3; (2) compound 3 reacted with hydroxylamine hydrochloride in an inert solvent (such as ethanol and methanol) under the action of a base (such as sodium carbonate, potassium carbonate, sodium hydroxide, triethylamine, pyridine, etc.) to give compound 4; (3) compound 4 reacted with dimethoxy acetonide in an inert solvent (e.g. 1, 2-dichloroethane and/or glacial acetic acid) to give the final product 5.
in route 3: (1) compound 1 and compound 2 undergone nucleophilic substitution reaction in an inert solvent (such as toluene) under the action of base (such as sodium tert-butoxide, potassium tert-butoxide, sodium hydride, potassium hydride, potassium carbonate, cesium carbonate, potassium phosphate, potassium hydroxide, sodium hydroxide, etc.) to give compound 3; (2) compound 3 reacted with 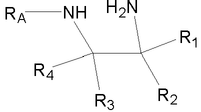 in the presence of trimethyl aluminum in an inert solvent (such as toluene) to give final product 4.

The starting materials of the present invention are known and commercially available, or can be synthesized according to the literature reported in the art.

### Pharmaceutical composition and method of administration

The pharmaceutical compositions of the present invention are used to prevent and/or treat the following diseases: inflammation, cancer, cardiovascular disease, infection, immunological disease, metabolic disease.

The compounds of the present invention can be used in combination with other drugs known to treat or improve similar conditions. When administered in combination, the original administration for the drug can remain unchanged, while compound of the present invention may be administered simultaneously or subsequently. Pharmaceutical composition containing one or more known drugs and the compound of the present invention may be preferred when administered in combination with one or more other drugs. The drug combination also includes administering the compound of the present invention and other one or more known drugs at overlapping time. When the compound of the present invention is combined with other one or more drugs, the dose of the compound of the present invention or known drug may be lower than that of their individual use.

The dosage forms of the pharmaceutical composition of the prensent invention include (but are not limited to): injection, tablet, capsule, aerosol, suppository, pellicle, pill, liniment for external use, controlled release or sustained-release or nano formulation.

The pharmaceutical composition of the present invention comprises a compound of the present invention or a pharmaceutically acceptable salt and a pharmaceutically acceptable excipient or carrier with safe and effective amount. In which, "safe and effective amount" refers to the amount of compound is sufficient to significantly improve the condition, not to produce severe side effects. Typically, the pharmaceutical composition contains 1-2000 mg of the compound of the present invention per dosage, and preferrably contains 10-1000 mg of the compound of the present invention per dosage. Preferably, "one dosage" is a capsule or a pill.

"Pharmaceutically acceptable carrier" refers to one or more compatible solid or liquid filler or gel substances, which are suitable for human use, and must be sufficiently pure and of sufficiently low toxicity. "Compatible" herein refers to each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc..

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectant, such as, glycerol; (d) disintegrating agent, such as agar, calcium carbonate, potato starch or tapioca starch, alginic acid, certain composite silicates, and sodium carbonate; (e) dissolutionretarding agents, such as paraffin; (f) absorption accelerators, such as quaternary ammonium compounds; (g) wetting agents, such as cetyl alcohol and glyceryl monostearate; (h) adsorbents, for example, kaolin; and (i) lubricants such as talc, calcium stearate, magnesium stearate, solid polyethylene glycol, lauryl sodium sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents such as water or other solvents, solubilizers and emulsifiers known in the art, such as ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The dosage forms for topical administration of compounds of the invention include ointments, powders, patches, aerosol, and inhalants. The active ingredients are mixed with physiologically acceptable carriers and any preservatives, buffers, or propellant that may be required if necessary, under sterile conditions.

Compounds of the present invention can be administrated alone, or in combination with other treatment means or therapeutic drugs.

When the pharmaceutical compositions are used, a safe and effective amount of compound of the present invention is administrated to a mammal (such as human) in need thereof, wherein the dose of administration is a pharmaceutically effective dose. For a person weighed 60 kg, the daily dose is usually 1-2000 mg, preferably 10-1000mg. Of course, the particular dose should also depend on various factors, such as the route of administration, patient healthy status, which are all within the skills of an experienced physician.

The present invention also provides a preparation method of pharmaceutical composition comprising the step of mixing a pharmaceutically acceptable carrier with the compound or the pharmacically acceptable salt, stereoisomer, solvate or prodrug thereof of the present invention, thus forming the pharmaceutical composition.

The invention also provides a treatment method comprising the steps of administering the compound, or pharmaceutically acceptable salt, stereoisomer, solvate or prodrug thereof, or administering the pharmaceutical composition of the invention to a subject in need thereof to selectively inhibit fusion mutations and drug resistance mutations of ROS1, NTRK and ALK, etc.

### The invention has the following main advantages:

(1) The compound of the invention has good inhibition ability to ROS1, NTRK and ALK kinase, especially excellent activity to drug-resistant mutation of these targets;
(2) The compound of the invention has better pharmacodynamics, pharmacokinetic properties and lower toxic and side effects;
(3) The compound of the invention has great potential to be developed into an effective drug for drug-resistant patients urgently needed clinically at present.

The technical solution of the present invention will be further described below, but the scope of protection of the present invention is not limited thereto.

Some specific examples are listed below for explanation.

### Example 1

### Synthetic route:

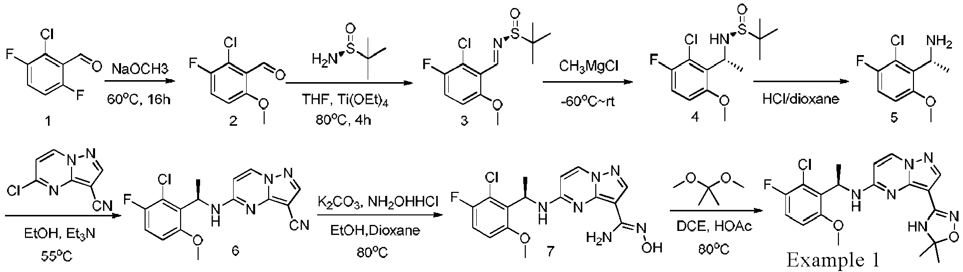

### Reaction steps:

(1) Synthesis of compound 2: 100mL single-neck flask, condenser tube, argon protection. Compound 1 (5.2 g) was weighed, and methanol (50ml) and tetrahydrofuran (25ml) were added, the temperature was raised to 60 °C under argon protection, 1M/L sodium methoxide solution (self-made) in methanol (32ml) was slowly added dropwise, finished in 1 hour and then stirred overnight at 60 °C. The next day, the solvent was evaporated, water and ethyl acetate were added for extraction, and then extracted with ethyl acetate again. The organic phases were combined, dried, evaporated and purified by column chromatography to obtain 4.21 g of an oily product. ¹H NMR (400 MHz, CDCl₃) δ 10.48 (d, *J=* 1.0 Hz, 1H), 7.31 (dd, *J* = 9.2, 8.2 Hz, 1H), 6.88 (dd, *J=* 9.2, 3.7 Hz, 1H), 3.92 (s, 3H).
(2) Synthesis of compound 3: A 250mL single-neck flask with a sealed condensing tube above was filled with compound 2 (4.01 g), (R)-tert-butyl sulfinamide (3.87 g, 1.5 eq), tetraethyl titanate (9.73 g, 2.0 eq) and tetrahydrofuran (100mL), stirred overnight at 80 °C and cooled on the next day. A large amount of saturated brine and ethyl acetate were added for extraction, the aqueous phase was extracted with dichloromethane once again. The organic phases were combined, dried, evaporated and purified by column chromatography to obtain 4.73 g of oily product, ¹H NMR (400 MHz, CDCl₃) δ 8.93 (s, 1H), 7.23 (dd, *J* = 9.1, 8.4 Hz, 1H), 6.85 (dd, *J =* 9.2, 3.9 Hz, 1H), 3.88 (s, 3H), 1.30 (s, 9H).
(3) Synthesis of compound 4: compound 3 (4.73 g) and tetrahydrofuran (200ml) were added into a 250mL three-neck flask, protected with argon and stirred at room temperature for 10min, then cooled to -10 °C, then 3M methyl magnesium chloride (25ml, 3eq) in tetrahydrofuran was added, and the reaction was slowly raised to room temperature and stirred overnight. The next day, TLC monitoring showed that the reaction was completed. Water and ethyl acetate were added for extraction, and then extracted with ethyl acetate again. The organic phases were combined, dried, evaporated and purified by column chromatography to obtain 4.525g of a solid product. ¹H NMR (400 MHz, CDCl₃) δ 7.01 (td, *J* = 9.2, 8.4 Hz, 1H), 6.76 (ddd, *J* = 9.1, 6.9, 4.1 Hz, 1H), 5.33-4.39 (m, 2H), 3.87 (d, *J=* 6.2 Hz, 3H), 1.57 (dd, *J =* 56.9, 7.0 Hz, 3H), 1.17 (d, *J* = 28.1 Hz, 9H).
(4) Synthesis of compound 5: compound 4 (4.525 g) and hydrochloric acid/dioxane (150ml) were added in a 500ml single-neck flask. Stirred at room temperature for 4 hours, monitored by TLC, and the raw materials were reacted completely. The solvent was evaporated directly, water was added, and then pH was adjusted to 9-10 with sodium carbonate aqueous solution. Extracted with ethyl acetate, extracted twice, dried and concentrated to obtain 2.86 g of a pale yellow oily product.
(5) Synthesis of compound 6: compound 5 (1.06 g), 5-chloropyrazolopyrimidin-3-carbonitrile (0.93 g, 1.0 eq), ethanol (60ml) and triethylamine (1.581 g, 3.0 eq) were added into a 100mL single-neck flask connected with a condenser tube, then stirred at room temperature for 10 min under the protection of argon gas, and then reacted overnight at 55 °C. The next day, TLC monitoring showed that the reaction was completed, and direct suction filtration was carried out to obtain 0.93 g of powder solid product. ¹H NMR (400 MHz, DMSO) δ 8.57 (d, *J =* 7.6 Hz, 1H), 8.46 (d, *J =* 7.3 Hz, 1H), 8.23 (s, 1H), 7.26 (t, *J =* 9.0 Hz, 1H), 7.02 (dd, *J =* 9.2, 4.3 Hz, 1H), 6.59 (d, *J=* 7.6 Hz, 1H), 5.82 (q, *J =* 7.1 Hz, 1H), 3.89 (s, 3H), 1.56 (d, *J =* 7.2 Hz, 3H).
(6) Synthesis of compound 7: compound 6 (0.93 g), anhydrous potassium carbonate (1.12 g, 3eq), hydroxylamine hydrochloride (0.563 g, 3eq), ethanol (40ml) and dioxane (20ml) were added into a 100ml single-neck flask and reacted overnight at 80 °C. The next day, TLC monitoring showed that the reaction was completed. The solvent was directly evaporated, water and ethyl acetate were added. The water phase was extracted with dichloromethane once. The organic phases were combined, dried, evaporated and purified by column chromatography to obtain 0.411 g of pure product, ¹H NMR (400 MHz, DMSO) δ 9.02 (s, 1H), 8.46 (d, *J =* 7.6 Hz, 1H), 8.13 (d, *J =* 7.5 Hz, 1H), 7.88 (s, 1H), 7.26 (t, *J =* 9.0 Hz, 1H), 7.04 (dd, *J=* 9.2, 4.3 Hz, 1H), 6.44 (d, *J=* 7.6 Hz, 1H), 5.89-5.61 (m, 3H), 3.87 (s, 3H), 1.54 (d, *J =* 7.2 Hz, 3H).

**Synthesis of Compound Example 1:** compound 7 (0.411 g), dimethoxy acetonide (0.457 g, 4eq), 1,2-dichloroethane (15ml) and glacial acetic acid (7.5 ml) were added, stirred at 80 °C for 4 hours, TLC monitoring showed that the reaction was completed. The solvent was directly evaporated, then water and dichloromethane were added for extraction, dried, evaporated and purified by column chromatography to obtain 170mg of final product. ¹H NMR (400 MHz, CDCl₃) δ 8.25-8.12 (m, 2H), 7.05 (dd, *J =* 9.1, 8.2 Hz, 1H), 6.80 (dd, *J* = 9.1, 4.0 Hz, 1H), 6.08 (t, *J =* 30.1 Hz, 4H), 3.91 (s, 2H), 1.58 (t, *J =* 5.8 Hz, 8H).

### Example 2

### Synthetic route:

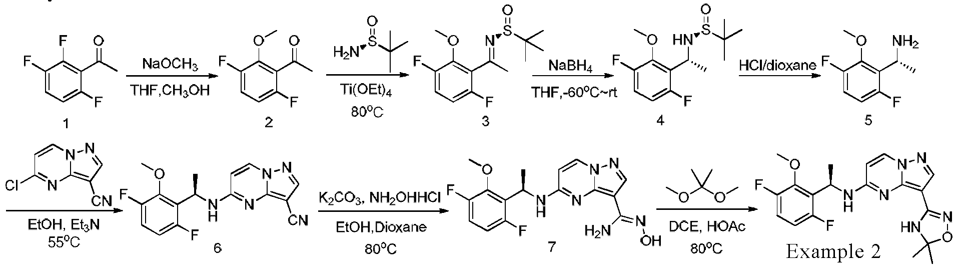

### Reaction steps:

(1) Synthesis of compound 2: 500mL three-neck flask was connected to a thermometer and a condenser tube, argon gas protection. Compound 1 (14.77 g) was weighed, methanol (200ml) and tetrahydrofuran (85ml) were added, the temperature was raised to 60 °C under argon protection, and 1M/L sodium methoxide solution (self-made) in methanol (85ml) was slowly added dropwise, finished in 1 hour. Then stirred overnight at 60 °C. The next day, the solvent was evaporated, water and ethyl acetate were added for extraction, and then extracted again with ethyl acetate to obtain 12g of an oily product.
(2) Synthesis of compound 3: compound 2 (12g), (R)-tert-butyl sulfinamide (19.52 g, 2.5 eq), tetraethyl titanate (36.8 g, 2.5 eq) and tetrahydrofuran (300ml) were added into a 500mL single-neck flask with a sealed condensing tube above. Stirred overnight at 80 °C, and cooled down the next day. A large amount of saturated brine and ethyl acetate were added for extraction, the aqueous phase was extracted with dichloromethane once again. The organic phases were combined, dried, evaporated and purified by column chromatography to obtain 3.0g of an oily product,
(3) Synthesis of compound 4: compound 3 (3.0 g) and tetrahydrofuran (200ml) were added into a 500mL single-neck flask, and stirred at room temperature for 10min under argon protection, then cooled to -60 °C with dry ice, and sodium borohydride (1.2 g, 3.0 eq) was added. The reaction was slowly raised to room temperature and stirred overnight. The next day, TLC monitoring showed that the reaction was completed. Water and ethyl acetate were add for extraction, and then extracted again with ethyl acetate. The organic phases were combined, dried, evaporated and purified by column chromatography to obtain 2.25 g of an oily product.
(4) Synthesis of compound 5: compound 4 (2.25 g) and hydrochloric acid/dioxane (50ml) were added in a 100mL single-neck flask, stirred at room temperature for 4 hours, monitored and the raw materials were reacted completely. The solvent was evaporated directly, water was added, and then pH was adjusted to 9-10 with sodium carbonate aqueous solution. Extracted with ethyl acetate twice, dried and concentrated to obtain 1.8g of a pale yellow oily product.
(5) Synthesis of compound 6: compound 5 (0.92g), 5-chloropyrazolopyrimidin-3-carbonitrile (0.81g, 1.1 eq), ethanol (40ml) and triethylamine (1.25g, 3.0 eq) were added into a 100mL single-neck flask connected with a condenser tube, then stirred at room temperature for 10 min under the protection of argon gas, and then reacted overnight at 55 °C. The next day, TLC monitoring showed that the reaction was completed, then directly evaporated, and water and ethyl acetate were added for extraction. The organic phases were combined, dried, evaporated, and purified by column chromatography to obtain 0.95 g of an oily product.
(6) Synthesis of compound 7: compound 6 (0.95 g), anhydrous potassium carbonate (0.8 g, 2eq), hydroxylamine hydrochloride (0.4 g, 2eq), ethanol (40ml) and dioxane (20ml) were added into a 100ml single-neck flask. Then reacted overnight at 80 °C, and the next day, TLC monitoring showed that the reaction was completed. The solvent was directly evaporated, water and ethyl acetate were added for extraction, and the water phase was extracted with dichloromethane once again. The organic phases were combined, dried, evaporated and purified by column chromatography to obtain 0.4 g of pure product. ¹H NMR (400 MHz, DMSO) δ 9.02 (s, 1H), 8.46 (d, *J =* 7.6 Hz, 1H), 8.23 (d, *J =* 7.1 Hz, 1H), 7.89 (s, 1H), 7.20 (ddd, *J=* 11.1, 9.2, 5.2 Hz, 1H), 6.98 (td, *J=* 9.6, 3.8 Hz, 1H), 6.40 (d, *J =* 7.6 Hz, 1H), 5.78 (d, *J* = 11.3 Hz, 2H), 5.55-5.33 (m, 1H), 3.91 (d, *J=* 1.7 Hz, 3H), 1.59 (d, *J* = 7.1 Hz, 3H).

Synthesis of compound Example 2: compound 7 (0.3g), dimethoxy acetonide (0.345g, 4eq), 1, 2-dichloroethane (10 mL) and glacial acetic acid (7.5 mL) were added, stirred at 80 °C for 4 hours. TLC monitoring showed that the reaction was completed. The solvent was directly evaporated, then water and dichloromethane were added. Dried, evaporated and purified by column chromatography to obtain 130mg of final product. ¹H NMR (400 MHz, CDCl₃) δ 8.20 (s, 1H), 8.15 (d, *J=* 7.6 Hz, 1H), 6.97 (ddd, *J=* 10.8, 9.2, 5.3 Hz, 1H), 6.75 (td, *J* = 9.4, 3.7 Hz, 1H), 6.34 (s, 1H), 6.06 (d, *J=* 7.5 Hz, 1H), 5.79-5.59 (m, 2H), 4.03 (d, *J* = 1.8 Hz, 2H), 1.72-1.64 (m, 5H), 1.60 (s, 3H).

### Example 3

### Synthetic route:

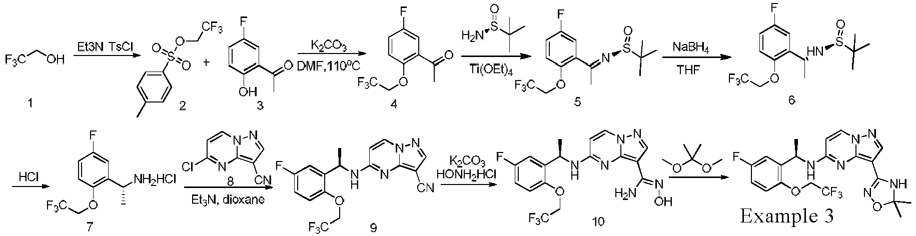

### Reaction steps:

(1) Synthesis of compound 2: 500mL three-neck flask was connected to a thermometer and a condenser tube, argon gas protection. Compound 1 (9.65 g) was weighed, dichloromethane (350ml) and p-toluenesulfonyl chloride (23.84 g, 1.3 eq) were added, the temperature was reduced to 0 °C under argon protection, triethylamine (29.24 g, 3.0 eq) was slowly added dropwise and finished in 10min, and then stirred overnight at room temperature. The next day, water and dichloromethane were added, and extracted again with dichloromethane, dried, evaporated and purified by column chromatography to obtain 20g of product.
(2) Synthesis of compound 4: 500mL three-neck flask was connected to a thermometer and a condenser tube, argon gas protection. Compound 2 (20g) was weighed, N, N-dimethylformamide (350ml) and compound 3 (12.13 g, 1eq) were added, and anhydrous potassium carbonate (54.33 g, 5eq) was added. The temperature was raised to 60 °C under argon and stirred overnight. The next day, water and ethyl acetate were added, extracted again with ethyl ester, dried, evaporated, and purified by column chromatography to obtain 13g of an oily product. Yield: 70.3%. ¹H NMR (400 MHz, CDCl₃) δ 7.51 (dd, *J=* 8.8, 3.3 Hz, 1H), 7.19 (ddd, *J=* 9.0, 7.2, 3.3 Hz, 1H), 6.88 (dd, *J=* 9.0, 3.9 Hz, 1H), 4.43 (q, *J* = 7.9 Hz, 2H), 2.63 (s, 3H).
(3) Synthesis of compound 5: compound 4 (13g), (R)-tert-butyl sulfinamide (13.33g, 1.5 eq), tetraethyl titanate (25.13g, 2.0 eq) and tetrahydrofuran (300mL) were added into a 500mL single-neck flask with a sealed condensing tube above, stirred overnight at 80 °C and cooled the next day. A large amount of saturated brine and ethyl acetate were added for extraction, the aqueous phase was extracted with dichloromethane once again, the organic phases were combined, dried, evaporated and purified by column chromatography to obtain 9.6g of an oily product, yield: 51.6%.
(4) Synthesis of compound 6: compound 5 (9.6g) and tetrahydrofuran (150ml) were added into a 250mL single-neck flask, and stirred at room temperature for 10min under argon protection, then cooled to -60 °C with dry ice, and sodium borohydride (3.23g, 3 eq) was added. The reaction was slowly raised to room temperature and stirred overnight. The next day, detected by TLC. Saturated ammonium chloride aqueous solution and ethyl acetate were added, and extracted once again with ethyl acetate. The organic phases were combined, dried, evaporated and purified by column chromatography to obtain 0.9g of an oily product. ¹H NMR (400 MHz, CDCl₃) δ 7.05 (dd, *J =* 8.8, 3.1 Hz, 1H), 6.94 (ddd, *J =* 8.9, 7.7, 3.1 Hz, 1H), 6.78 (dd, *J=* 9.0, 4.3 Hz, 1H), 4.67 (p, *J* = 6.8 Hz, 1H), 4.47-4.32 (m, 2H), 3.79 (d, *J=* 6.9 Hz, 1H), 1.50 (d, *J=* 6.8 Hz, 3H), 1.21 (s, 9H).
(5) Synthesis of compound 7: compound 6 (0.9g) and hydrochloric acid/dioxane (50ml) were added in a 100mL single-neck flask, stirred at room temperature for 4 hours, detected by TLC and the raw materials were reacted completely. The solvent was directly evaporated to obtain 0.865 g of pale yellow solid.
(6) Synthesis of compound 9: compound 7 (0.865 g), 5-chloropyrazolopyrimidin-3-carbonitrile (0.562 g, 1eq), ethanol (40ml) and triethylamine (0.96 g, 3eq) were added into a 100ml single-neck flask. Connected to a condenser tube and argon protection, stirred at room temperature for 10min, and then reacted overnight at 55 °C. The next day, directly evaporated, water and ethyl acetate were added for extraction, dried, evaporated and purified by column chromatography to obtain 0.988 g of an oily product.
(7) Synthesis of compound 10: compound 9 (0.988 g), anhydrous potassium carbonate (1.08 g, 3eq), hydroxylamine hydrochloride (0.544 g, 3eq), ethanol (40ml) and dioxane (20ml) were added into a 100ml single-neck flask and reacted overnight at 80 °C. The next day, detected by TLC. The solvent was directly evaporated, water and ethyl acetate were added for extraction, and the water phase was extracted with dichloromethane once again. The organic phases were combined, dried, evaporated and purified by column chromatography to obtain 0.65g of pure product, yield: 60.5%. ¹H NMR (400 MHz, DMSO) δ 9.00 (s, 1H), 8.55 (dd, *J=* 38.2, 7.8 Hz, 1H), 8.28 (d, *J=* 6.5 Hz, 1H), 7.92 (d, *J=* 33.3 Hz, 1H), 7.18-7.02 (m, 3H), 6.47 (dd, *J =* 77.2, 7.8 Hz, 1H), 5.63 (s, 2H), 5.44-5.27 (m, 1H), 5.08-4.74 (m, 2H), 1.42 (d, *J =* 6.9 Hz, 3H).

**Synthesis of compound Example 3:** compound 10 (0.65 g), dimethoxy acetonide (0.656 g, 4eq), 1, 2-dichloroethane (15ml) and glacial acetic acid (7.5 ml) were added to a reaction flask and stirred at 80 °C for 4 hours. The solvent was directly evaporated, water and dichloromethane were added for extraction, dried, evaporated and purified by column chromatography to obtain 180mg of the final product. The HPLC purity was 99%. ¹H NMR (400 MHz, CDCl₃) δ 8.29-8.10 (m, 2H), 7.03 (dd, *J =* 8.6, 2.9 Hz, 1H), 7.00-6.92 (m, 1H), 6.83 (dd, *J* = 9.0, 4.2 Hz, 1H), 6.08 (d, *J=* 7.6 Hz, 1H), 5.79 (s, 1H), 5.51 (d, *J=* 5.5 Hz, 1H), 5.25 (s, 1H), 4.53-4.29 (m, 2H), 1.62 (s, 3H), 1.59 (s, 3H), 1.47 (s, 3H).

### Example 4

### Synthetic route:

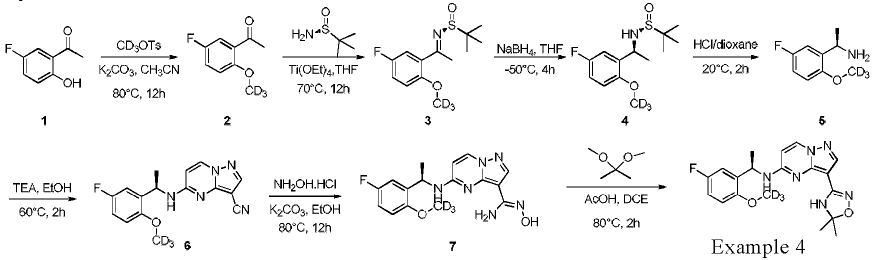

### Reaction steps:

(1) Synthesis of compound 2: compound 1 (6.6 g, 42.8 mmol, 1eq) was dissolved in acetonitrile (100 mL) solution, and CD₃OTS (9.72 g, 51.4 mmol, 1.2 eq) and K₂CO₃ (8.88 g, 64.2 mmol, 1.5 eq) were added, and reacted at 80°C for 12 h. After the raw materials were reacted completely, water and ethyl acetate were added for extraction, and washed with brine for three times. The organic phases were combined, dried over anhydrous sodium sulfate, and the organic phase was evaporated to obtain the compound 2 (7.0 g, 40.9 mmol, 95.5% yield).
(2) Synthesis of compound 3: compound 2 (6.0 g, 35.1 mmol, 1.0 eq) was dissolved in 60 mL dry THF solution, and (R)-(+)-tert-butyl sulfinamide (8.5 g, 70.1 mmol, 2eq) and Ti (OEt)₄(16.0 g, 70.1 mmol, 2eq) were added, and reacted at 70°C for 12 h. After the raw materials were reacted completely, water and ethyl acetate were added for extraction, and washed with brine for three times. The organic phases were combined, dried over anhydrous sodium sulfate, and the organic phase was evaporated and purified by column chromatography (petroleum ether: ethyl acetate= 4: 1) to obtain compound 3 (8.0 g, 29.1 mmol, yield 83.2%).
(3) Synthesis of compound 4: compound 3 (3.0 g, 10.9 mmol, 1.0 eq) was dissolved in 30 mL dry THF solution, NaBH₄ (1.24 g, 32.8 mmol, 3eq) was added under -50 °C, and the reaction was continued for 4 h under -50°C. After the raw materials were reacted completely, saturated ammonium chloride aqueous solution was added to quench, extracted with ethyl acetate and washed with brine for three times. The organic phases were combined, dried over anhydrous sodium sulfate. The organic phase was evaporated and purified by column chromatography (petroleum ether: ethyl acetate= 8: 1) to obtain compound 4 (1 g, 3.62 mmol, yield 33.1%).
(4) Synthesis of compound 5: 4M dioxane hydrochloride (10 mL) was added into compound 4 (1.0 g, 3.62 mmol, 1eq) under ice bath, and continued to react at 0°C for 1 h. After the raw materials were reacted completely, saturated sodium carbonate aqueous solution was added to quench, extracted with ethyl acetate, and washed with brine for three times. The organic phases were combined, dried over anhydrous sodium sulfate. The organic phases were evaporated to obtain compound 5 (0.5 g, yellow oily liquid, yield: 80.2%).
(5) Synthesis of compound 6: compound 5 (500 mg, 2.9 mmol, 1eq) was dissolved in ethanol (8 mL), and then the compound 5a (622 mg, 3.48 mmol, 1.2 eq) and triethylamine (881 mg, 8.71 mmol, 2 eq) were added. Then the temperature was raised to 60 ° C to react for 2 h. After the raw materials were reacted completely, the solvent was evaporated and purified by column chromatography (petroleum ether: ethyl acetate= 2: 1) to obtain compound 6 (0.75 g, 2.39 mmol, yield 82.2%).
   MS: 300 (M + H +).
(6) Synthesis of compound 7: compound 6 (700 mg, 2.23 mmol, 1eq) was dissolved in ethanol (10 ml), then hydroxylamine hydrochloride (310 mg, 4.45 mmol, 2 eq) and potassium carbonate (616 mg, 4.45 mmol, 2 eq) were added, then the reaction temperature was raised to 80 ° C to react for 12 h. After the raw materials were reacted completely, water and ethyl acetate were added for extraction, and washed with brine for three times. The organic phases were combined, dried over anhydrous sodium sulfate. The organic phase was evaporated and purified by column chromatography (dichloromethane: methanol=50: 1) to obtain compound 7 (700 mg, 2.02 mmol, yield 90.5%).

**Synthesis of compound Example 4:** compound 7 (400 mg, 1.15 mmol, 1eq) was dissolved in 5 mL acetic acid and 1, 2-dichloroethane (5 mL), and then compound 7a (480 mg, 4.61 mmol, 4 eq) was added, and then the temperature was raised to 80 ° C to react for 2 h. After the raw materials were reacted completely, saturated sodium carbonate aqueous solution was added to quench, ethyl acetate were added for extraction, and washed with brine for three times. The organic phases were combined, dried over anhydrous sodium sulfate. The organic phase was evaporated and purified by column chromatography (petroleum ether: ethyl acetate= 0: 1) to obtain compound Example 4 (300 mg, white solid,0.77 mmol, yield: 67.2%). ¹H NMR (400 MHz, CDCl₃) δ 8.17 (d, *J* = 7.6 Hz, 1H), 8.15 (s, 1H), 6.98 (dd, *J =* 8.8, 3.3 Hz, 1H), 6.95 - 6.88 (m, 1H), 6.86 (dd, *J=* 8.8, 4.4 Hz, 1H), 6.17 (d, *J =* 6.4 Hz, 1H), 5.95 (s, 1H), 5.86 (d, *J* = 5.4 Hz, 1H), 1.62 (s, 3H), 1.54 (d, *J=* 6.9 Hz, 3H), 1.44 (s, 3H).

### Example 5

### Synthetic route:

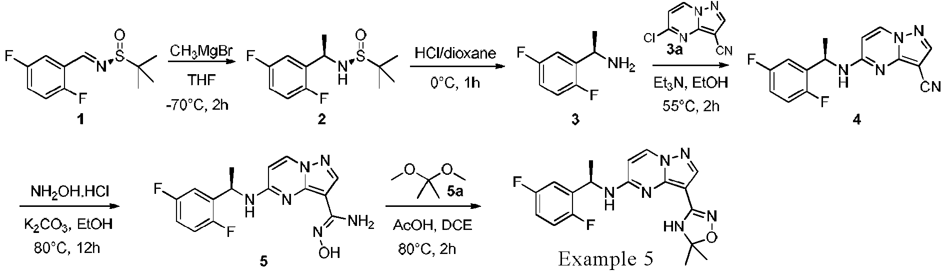

### Reaction steps:

(1) Synthesis of compound 2: compound 1 (8 g, 32.6 mmol, 1eq) was dissolved in dry tetrahydrofuran (60 mL), and magnesium methyl bromide solution (21 mL, 65.2 mmol, 3 M, 2eq) was added dropwise at -70°C. After addition was completed, the reaction was continued to react for 2 h. After the raw materials were reacted completely, saturated ammonium chloride aqueous solution was added to quench, extracted with ethyl acetate and washed with brine for three times. The organic phases were combined, dried over anhydrous sodium sulfate. The organic phase was evaporated and purified by column chromatography (petroleum ether: ethyl acetate= 4: 1) to obtain compound 2 (2 g, yellow solid, yield 23.5%).
(2) Synthesis of compound 3: 4M dioxane hydrochloride (10 mL) was added into compound 2 (1.5 g, 5.74 mmol, 1eq) under ice bath, and reacted at 0°C for 1 h. After the raw materials were reacted completely, saturated sodium carbonate aqueous solution was added to quench, extracted with ethyl acetate, and washed with brine for three times. The organic phases were combined, dried over anhydrous sodium sulfate. The organic phases were evaporated to obtain compound 3 (0.8 g, yellow oily liquid, yield: 88.7 %).
(3) Synthesis of compound 4: compound 3 (200 mg, 1.27 mmol, 1eq) was dissolved in ethanol (4 mL), and then compound 3a (272 mg, 1.53 mmol, 1.2 eq) and triethylamine (257 mg, 2.55 mmol, 2 eq) were added. Then the temperature was raised to 55 ° C to react for 2 h. After the raw materials were reacted completely, the solvent was evaporated, and purified by column chromatography (petroleum ether: ethyl acetate= 2: 1) to obtain compound 4 (150 mg, white solid, yield 39.4 %).
   MS: 300 (M + H +).
(4) Synthesis of compound 5: compound 4 (150 mg, 0.5 mmol, 1eq) was dissolved in ethanol (2 ml), then hydroxylamine hydrochloride (70 mg, 1.0 mmol, 2 eq) and potassium carbonate (138 mg, 1.0 mmol, 2 eq) were added, then the reaction temperature was raised to 80 °C to react for 12 h. After the raw materials were reacted completely, water and ethyl acetate were added for extraction, and washed with brine for three times. The organic phases were combined, dried over anhydrous sodium sulfate. The organic phase was evaporated and purified by column chromatography (dichloromethane: methanol=50: 1) to obtain compound 5 (50 mg, brown oily liquid, yield 30.0 %).

**Synthesis of compound Example 5:** compound 5 (50 mg, 0.15 mmol, 1eq) was dissolved in acetic acid (0.5 mL) and 1, 2-dichloroethane (0.5 mL), and then compound 5a (62 mg, 0.6 mmol, 4 eq) was added. Then the temperature was raised to 80 °C to react for 2 h. After the raw materials were reacted completely, saturated sodium carbonate aqueous solution was added to quench, extracted with ethyl acetate, and washed with brine for three times. The organic phases were combined, dried over anhydrous sodium sulfate. The organic phase was evaporated and purified by column chromatography (petroleum ether: ethyl acetate= 0: 1) to obtain Example 5 (20 mg, white solid, 35.7% yield). ¹H NMR (400 MHz, CDCl₃) δ 8.21 (d, *J=* 7.6 Hz, 1H), 8.18 (s, 1H), 7.09 - 6.99 (m, 2H), 6.92 (m, 1H), 6.16 (d, *J* = 7.6 Hz, 1H), 5.94 (s, 1H), 5.55 (d, *J=* 5.7 Hz, 1H), 5.38 (m, 1H), 1.64 (s, 3H), 1.60 (s, 3H), 1.50 (s, 3H). MS: 373 (M + H +).

### Example 6

### Synthetic route:

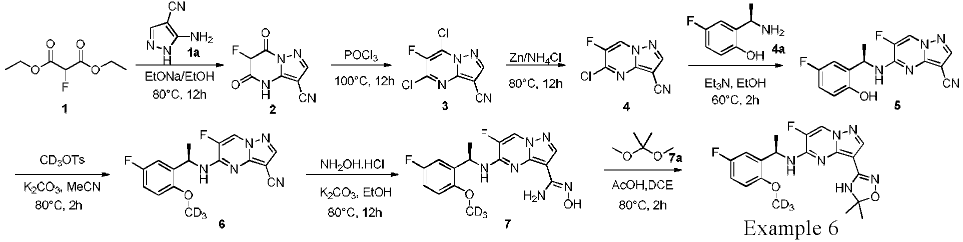

### Reaction steps:

(1) Synthesis of compound 2: compound 1a (10 g, 92.5 mmol, 1eq) and compound 1 (17.3 g, 97.1 mmol, 1.05 eq) were dissolved in 200 mL ethanol, EtONa (8.81 g, 129.5 mmol, 1.4 eq) was added, and the temperature was raised to 80 °C to react for 12 h. After the raw materials were reacted completely, the solvent was evaporated, water was added, pH was adjusted to 2-3 with 1M HCl, precipitate was precipitated. The precipitate was filtered and dried to obtain compound 2 (14 g, 72.12 mmol, yield 66.8%).
(2) Synthesis of compound 3: compound 2 (14 g, 72.12 mmol, 1eq) was added to POCl₃ (100 mL), and reacted at 100°C for 12h. After the raw materials were reacted completely, the solvent was evaporated, and purified by column chromatography to obtain compound 3 (2.2 g, 9.52 mmol, yield 13.2%).
(3) Synthesis of compound 4: compound 3 (2.2 g, 9.52 mmol, 1eq) was dissolved in ethanol (42 ml), tetrahydrofuran (14 ml) and water (28 ml), then Zn powder (3.11 g, 47.6 mmol, 5 eq) and NH₄Cl (2.04 g, 38.1 mmol, 4 eq) were added, then reacted at 20 °C for 10 minutes. After the raw materials were reacted completely, water and ethyl acetate were added for extraction, and washed with brine for three times. The organic phases were combined, dried over anhydrous sodium sulfate. The organic phase was evaporated and purified by column chromatography to obtain compound 4 (1.0 g, 5.09 mmol, yield 53.4%).
(4) Synthesis of compound 5: compound 4a (500 mg, 3.22 mmol, 1eq) was dissolved in ethanol (6 ml), then compound 4 (696 mg, 3.54 mmol, 1.1 eq) and triethylamine (978 mg, 9.67 mmol, 3 eq) were added. The temperature was raised to 60 °C to react for 2 h. After the raw materials were reacted completely, the solvent was evaporated, purified by column chromatography (petroleum ether: ethyl acetate= 2: 1) to obtain compound 5 (600 mg, white solid, yield 59.0%).
(5) Synthesis of Compound 6: Compound 5 (600 mg, 1.9 mmol, 1eq) was dissolved in 6 mL acetonitrile, then CD₃OTs (432 mg, 2.28 mmol, 1.2 eq) and potassium carbonate (395 mg, 2.85 mmol, 2 eq) were added, then heated to 80 °C to react for 2 h. After the raw materials were reacted completely, water and ethyl acetate were added for extraction, and washed with brine for three times. The organic phases were combined, dried over anhydrous sodium sulfate. The organic phase was evaporated and purified by column chromatography to obtain compound 6 (400 mg, 1.2 mmol, yield 63.2%).
(6) Synthesis of compound 7: compound 6 (250 mg, 0.75 mmol, 1eq) was dissolved in ethanol (5 ml), then hydroxylamine hydrochloride (105 mg, 1.5 mmol, 2 eq) and potassium carbonate (208 mg, 1.5 mmol, 2 eq) were added, then the reaction temperature was raised to 80 °C to react for 12 h. After the raw materials were reacted completely, water and ethyl acetate were added for extraction, and washed with brine for three times. The organic phases were combined, dried over anhydrous sodium sulfate. The organic phase was evaporated and and purified by column chromatography (dichloromethane: methanol=50: 1) to obtain compound 7 (250 mg, brown oily liquid, yield 91.0 %).

**Synthesis of Compound Example 6:** compound 7 (250 mg, 0.68 mmol, 1eq) was dissolved in acetic acid (2 ml) and 1, 2-dichloroethane (2 ml), then compound 7a (285 mg, 2.74 mmol, 4 eq) was added and the temperature was raised to 80 °C to react for 2 h. After the raw materials were reacted completely, saturated sodium carbonate aqueous solution was added to quench, extracted with ethyl acetate, and washed with brine for three times. The organic phases were combined, dried over anhydrous sodium sulfate. The organic phase was evaporated and purified by column chromatography (petroleum ether: ethyl acetate= 0: 1) to obtain Example 6 (50 mg, white solid, yield 18.0%). ¹H NMR (400 MHz, CDCl₃) δ 8.27 (d, *J* = 5.6 Hz, 1H), 8.18 (s, 1H), 6.94 (m, 2H), 6.88 (m, 1H), 5.87 (d, *J=* 6.0 Hz, 1H), 5.71 (s, 1H), 5.36 (m, 1H), 1.62 (m, 6H), 1.44 (s, 3H).

### Example 7

### Synthetic route:

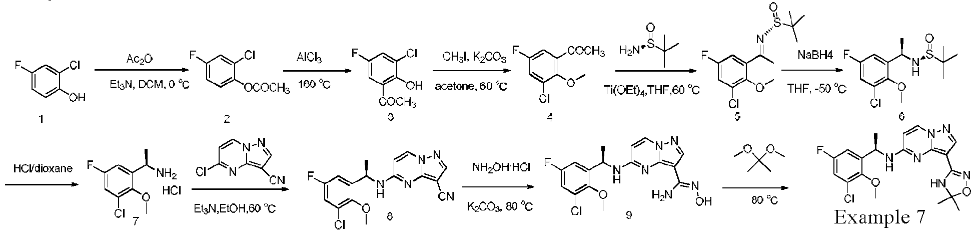

### Reaction steps:

(1) Synthesis of compound 2: 6 g of compound 1 and triethylamine (4.97 g, 1.2 eq) were dissolved in dichloromethane in a 100 mL three-neck flask, and acetyl chloride (3.86 g, 1.2 eq) was slowly added at 0 °C. The reaction was monitored by TLC until it was completed. Extracted with water and ethyl acetate, dried over anhydrous sodium sulfate, evaporated, and purified by column chromatography to obtain 7 g of compound 2. GC-MS [M] was 188.
(2) Synthesis of compound 3: compound 2 (7 g) and aluminum trichloride (14.86 g, 3 eq) were added into a 100 mL round bottom flask, the temperature was raised to 160 °C, the reaction was stirred for 1 h. TLC monitoring showed that the reaction was completed. Hydrochloric acid (6 mol/L) was added, extracted with ethyl acetate, dried over anhydrous sodium sulfate, evaporated and purified by column chromatography to obtain 6.16 g of compound 3. GC-MS [M] was 188.
(3) Synthesis of compound 4: compound 3 (2 g) and potassium carbonate (7.3 g, 5 eq) were dissolved in acetone in a 100 mL round bottom flask, methyl iodide (7.5 g, 5 eq) was added under stirring, the temperature was raised to 60 °C, and the reaction was monitored by TLC until it was completed. Extracted with ethyl acetate, dried over anhydrous sodium sulfate, evaporated, and separated by column chromatography to obtain 2.05 g of compound 4. LC-MS [M+1] was 203.
(4) Synthesis of compound 5: compound 4 (2.05 g) and R-tert-butyl sulfinamide (2.42 g, 2 eq) were dissolved in anhydrous tetrahydrofuran in a 100 mL round bottom flask, ethyl titanate (4.56 g, 2 eq) was added under stirring, and the temperature was raised to 60 °C. The reaction was monitored by TLC until it was completed. Water was added, suction-filtered, extracted with ethyl acetate, evaporated, and separated by column chromatography to obtain 2.63 g of compound 5. LC-MS [M+1] was 306.
(5) Synthesis of compound 6: compound 5 (2.63 g) was dissolved in anhydrous tetrahydrofuran in a 100 mL three-neck flask, sodium borohydride (0.98 g, 3 eq) was added at-50 °C, and the reaction was monitored by TLC until it was completed. After quenching with aqueous ammonium chloride solution, extracted with ethyl acetate. The organic phase was dried over anhydrous sodium sulfate, and separated by column chromatography to obtain 1.76 g of compound 6. LC-MS [M+1] was 308.
(6) Synthesis of compound 7: compound 6 (1.76 g) was added in a 100 ml round bottom flask, and dioxane hydrochloride (10 ml) was added. After stirring for 1 h, TLC showed that the reaction was completed. Filter cake 1.1 g compound 7 was obtained by suction filtration.
(7) Synthesis of compound 8: compound 7 (1.1 g) and 5-chloro-3-cyanopyrazolo [1, 5-α] pyrimidine (0.98 g, 1.2 eq) were dissolved in absolute ethanol in a 100 mL round bottom flask, and triethylamine (1.8 g, 4 eq) was added dropwise under stirring. The temperature was raised to 60 °C, and the reaction was monitored by TLC until it was completed. The solvent was evaporated and extracted with water and ethyl acetate. The organic phase was dried over anhydrous sodium sulfate and separated by column chromatography to obtain 1.35 g of compound 8. LC-MS [M+1] was 346.
(8) Synthesis of compound 9: compound 8 (1.35 g), hydroxylamine hydrochloride (1 g, 4 eq), potassium carbonate (2 g, 4 eq) and ethanol (10 ml) were added into a 100 ml round bottom flask. The temperature was raised to 80 °C, and the reaction was monitored by TLC until it was completed. The solvent was spin-dried and extracted with water and ethyl acetate. The organic phase was dried over anhydrous sodium sulfate and separated by column chromatography to obtain 0.73 g of compound 9. LC-MS [M+1] was 379.

**Synthesis of Example 7:** compound 9 (0.73 g) was dissolved in acetic acid (4 ml) and 1, 2-dichloroethane (4 ml) in a 100 ml round-bottom flask. 2, 2-dimethoxypropane (1 g, 5 eq) was added under stirring and the temperature was raised to 80 °C. The reaction was monitored by TLC until it was completed. An aqueous solution of sodium carbonate and ethyl acetate were added for extraction, and the organic phase was dried over anhydrous sodium sulfate and separated by column chromatography to obtain 0.36 g of Example 8. LC-MS [M+1] = 419. ¹H NMR (400 MHz, CDCl₃) δ 8.14 (dd, *J* = 7.7, 1.9 Hz, 2H), 7.07 (dt, *J =* 7.9, 3.9 Hz, 1H), 7.01 (dd, *J=* 7.4, 2.6 Hz, 1H), 6.33 - 6.21 (m, 2H), 5.58 (d, *J=* 5.7 Hz, 1H), 3.98 (s, 3H), 1.72 (s, 3H), 1.62 - 1.55 (m, 6H).

### Example 8

### Synthetic route:

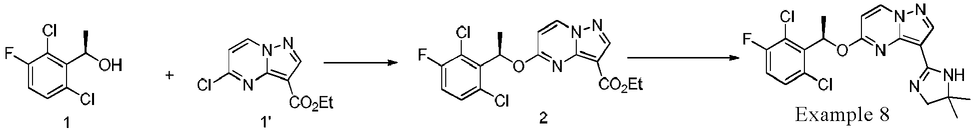

### Reaction steps:

(1) Synthesis of compound 2: sodium tert-butoxide (0.07 g, 1.5 eq) was dissolved in 5ml toluene, compound 1 (0.1 g, 1eq) was added at 0 °C, and compound 1' (ethyl 5-clopyrazolo [1, 5-a] pyrimidin-3-carboxylate) (0.13 g, 1.2 eq) was added to the reaction system after 5min. The temperature was gradually increased to rt to react for 2h. TLC monitoring showed that the reaction was completed, quenched with ammonium chloride solution, extracted with EA and dried, samples were mixed and purified by column chromatography to obtain 0.1 g compound 2 in a yield of 50%.

**Synthesis of compound Example 8:** 1, 2-diamino-2-methylpropane (1.5 eq) was dissolved in dry toluene (3ml). Trimethyl aluminum (5eq) was added dropwise at 0 °C under the protection of Ar, and then the temperature was raised to RT to react for 2h. Then compound 2 (0.1 g, 1eq) in toluene (3mL) was added dropwise at 0 °C. After reaction for 30min, the temperature was raised to 80 °C to react for 3h. The reaction was monitored by TLC until it was completed. Quenched with methanol, adjusted pH to be 8-9, extracted with EA and dried, and 20mg was obtained by separation on preparation plate with a yield of 20%. ¹H NMR (400 MHz, CDCl₃) δ 9.54 (s, 1H), 8.63 (d, *J* = 7.5 Hz, 1H), 7.36 - 7.28 (m, 2H), 7.13 (dd, *J=* 8.9, 7.9 Hz, 1H), 6.71 (d, *J=* 7.5 Hz, 1H), 6.61 (q, *J=* 6.8 Hz, 1H), 3.71 (dd, *J* = 27.5, 10.7 Hz, 2H), 1.86 (d, *J =* 6.9 Hz, 3H), 1.58 (d, *J =* 4.4 Hz, 6H).

### Example 9

### Synthetic route:

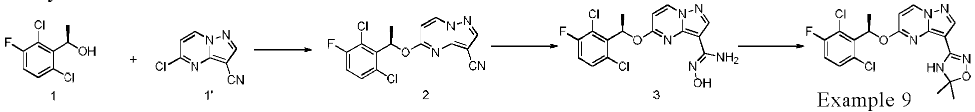

### Reaction steps:

(1) Synthesis of compound 2: sodium tert-butoxide (0.35 g, 1.5 eq) was dissolved in 25ml toluene, compound 1 (0.5 g, 1eq) was added at 0 °C, and compound 1' (0.51 g, 1.2 eq) was added to the reaction system after 5min. The temperature was gradually increased to rt to react for 2h. The reaction was monitored by TLC until it was completed. Quenched by ammonium chloride solution, extracted with EA and dried, samples were mixed and purified by column chromatography to obtain 0.7 g with a yield of 83%.
(2) Synthesis of compound 3: compound 2 (0.7 g, 1eq), hydroxylamine hydrochloride (0.28 g, 2eq) and potassium carbonate (0.56 g, 2eq) were successively added into absolute ethanol (7ml), and reacted overnight at 80 °C. After the reaction was completed, water and EA were added for extraction and dried, samples were mixed and purified by column chromatography to obtain 0.3 g with a yield of 39.5%. LCMS (384.0, 386.0).

**Synthesis of Compound Example 9:** compound 3 (0.1 g, 1eq) and 2, 2-dimethoxypropane (0.11 g, 4eq) were added into acetic acid (4ml) and reacted overnight at 50 °C. After the reaction was completed, the mixture was basified with sodium bicarbonate solution, extracted with EA, dried, samples were mixed and purified by column chromatography to obtain 0.07 g with a yield of 63.6%. ¹H NMR (400 MHz, CDCl₃) δ 8.43 (d, *J=* 7.5 Hz, 1H), 8.32 (s, 1H), 7.31 - 7.27 (m, 1H), 7.07 (dd, *J =* 8.8, 8.0 Hz, 1H), 6.59 (q, *J=* 6.9 Hz, 1H), 6.49 (d, *J* = 7.5 Hz, 1H), 5.61 (s, 1H), 1.81 (d, *J=* 6.9 Hz, 3H), 1.63 (s, 3H), 1.55 (s, 3H).

### Example 10

### Synthetic route:

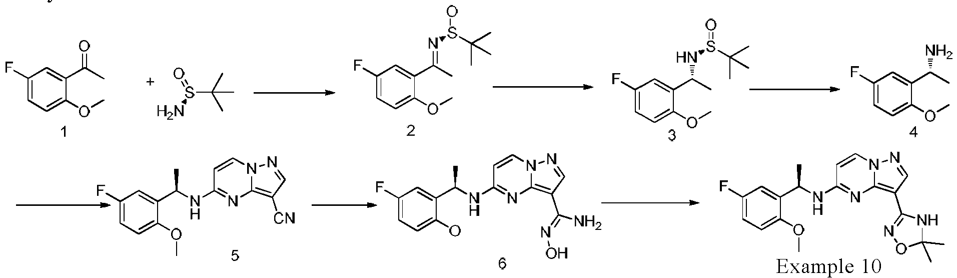

### Reaction steps:

(1) Synthesis of compound 2: compound 1 (5g, 1eq) was dissolved in THF (50ml), R-tert-butyl sulfinamide (7.25 g, 2eq) was added, then tetraethyl titanate (13.75 g, 2eq) was added to the reaction system, and reacted overnight at 60 °C. The reaction was monitored by TLC until it was completed. The samples were mixed and purified by column chromatography (PE: EA=10: 1-5: 1) to obtain 4.3 g of compound 2 with a yield of 53.7%.
(2) Synthesis of compound 3: compound 2 (4.4 g, 1eq) was dissolved in THF (35ml), sodium borohydride (1.85 g, 3eq) was added in batches at -50 °C, and then the temperature was gradually raised to RT to react for 5h. After the reaction was completed, water and EA were added for extraction, samples were mixed and purified by column chromatography to obtain 2.6 g+1g compound 3 (containing its diastereomers) with a yield of 59.1%.
(3) Synthesis of compound 4: compound 3 (0.8 g, 1eq) was added into 8ml 4M dioxane hydrochloride, reacted for 4h at RT. The reaction was monitored by TLC until it was completed. Sodium carbonate solution was added to adjust pH to be 9-10, extracted with EA, dried and evaporated to obtain 0.5 g of compound 4 with a yield of 98%.
(4) Synthesis of compound 5: compound 4 (0.5 g, 1eq) was added into 15ml absolute ethanol, followed by 5-clopyrazolo [1, 5-a] pyrimidin-3-cyano (0.58 g, 1.1 eq) and triethylamine (0.9 g, 3eq) and reacted overnight at 60 °C. The reaction was monitored by TLC until it was completed. PE was added and filtered to obtain 0.4 g with a yield of 43.5%.
(5) Synthesis of compound 6: compound 5 (0.4 g, 1eq), hydroxylamine hydrochloride (0.18 g, 2eq) and potassium carbonate (0.36 g, 2eq) were successively added into a mixture of absolute ethanol: dioxane = 2: 1 (15ml), and reacted overnight at 80 °C. After the reaction was completed, water and EA were added for extraction, dried, samples were mixed and purified by column chromatography to obtain 0.4 g with a yield of 91%.

**Synthesis of compound Example 10:** compound 6 (0.2 g, 1eq) and 2,2-dimethoxypropane (0.25 g, 4eq) were added into the mixed solvent (6ml) of acetic acid: 1, 2-dichloroethane = 1: 1, and reacted at 80 °C for 2h. After the reaction was completed, the mixture was basified with sodium bicarbonate solution, extracted with EA, dried, and the samples were mixed and purified by column chromatography to obtain 0.13 g with a yield of 59.1%. ¹H NMR (400 MHz, CDCl₃) δ 8.19 (d, *J =* 7.8 Hz, 2H), 6.92 (m, *J =* 13.3, 8.8, 3.7 Hz, 3H), 6.09 (d, *J=* 7.4 Hz, 1H), 5.87 (s, 1H), 5.47 (s, 1H), 5.29 (s, 1H), 3.91 (s, 3H), 1.62 (s, 3H), 1.56 (d, *J =* 6.7 Hz, 6H).

### Example 11

### Synthetic route:

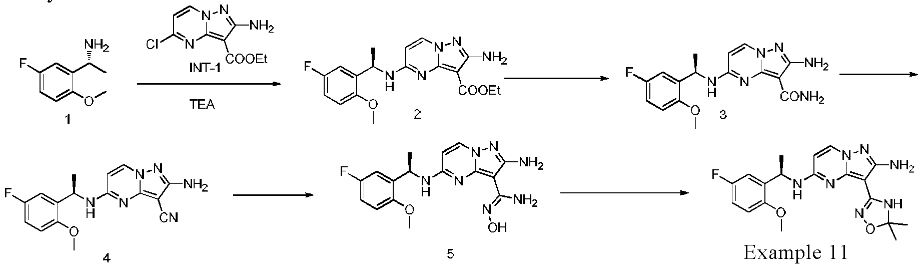

### Reaction steps:

(1) Synthesis of compound 2: 10ml of anhydrous ethanol was added to compound 1 (1.0 g, 1.5 eq), followed by INT-1 (947mg, 1.0 eq) and TEA (1.6 ml, 3.0 eq), respectively. After replacing with nitrogen, the reaction was carried out at 60 °C for 18h. TLC monitoring showed that the reaction was completed. Then the ethanol was evaporated. Then water (50ml) was added to the reaction system, and EA (50ml X3) was added for extraction. EA phases were combined, dried by adding anhydrous sodium sulfate, filtered, evaporated and purified by column chromatography to obtain 1.1 g of compound 2 (yield 86%).
(2) Synthesis of compound 3: 50ml toluene was added into a 150ml three-necked flask, cooled to -10 ~ 0 °C, then blew ammonia into toluene until saturated. Trimethyl aluminum (12.4 ml, 4.5 eq) was added dropwise at 0 °C, stirred at room temperature for 2h after addition. Then cooled down to 0 °C, compound 2 (1.1 g, 1.0 eq) in toluene was added dropwise, and the temperature was raised to 80 °C to react for 18h after addition. After TLC monitoring showed that the reaction was completed, filtrated, washed the cake with EA, and the filtrate was collected. Water was added to the filtrate, the liquid was separated, the organic phase was collected, anhydrous sodium sulfate was added to dry, filtered, evaporated and purified by column chromatography to obtain 600mg of compound 3 (yield 51%).
(3) Synthesis of compound 4: phosphorus oxychloride (10ml) was added to compound 3 (600mg, 1.0 eq), stirred at 80 °C for 5h, and TLC monitoring showed that the reaction was completed. The phosphorus oxychloride was evaporated, then pH was adjusted to be 7 ~ 8 with sodium bicarbonate aqueous solution, then EA (40x3) was added for extraction, separation. EA phases were combined, anhydrous sodium sulfate was added for drying, filtrated, evaporated and purified by column chromatography to obtain 120mg of compound 4 (yield 21%).
(4) Synthesis of compound 5: absolute ethanol (3ml) and 1, 4-dioxane (3ml) were added to compound 4 (80mg, 1.0 eq), then hydroxylamine hydrochloride (42.6 mg, 2.0 eq) and potassium carbonate (85mg, 2eq) were added, and replaced with nitrogen gas, reacted at 80 °C for 16h. TLC monitoring showed that the reaction was completed, filtered, evaporated and purified by column chromatography directly to obtain 70mg of compound 5 (yield 79%).

**Synthesis of Example 11:** 1ml of glacial acetic acid and 1, 2-dichloroethane (1ml) were added to compound 5 (70mg, 1.0 eq), then 2, 2-dimethoxypropane (81mg, 4eq) was added, and replaced with nitrogen gas, reacted at 80 °C for 1h. TLC monitoring showed that the reaction was completed, the solvent was evaporated, then sodium bicarbonate aqueous solution was added to the system, pH was adjusted to be 7 ~ 8, then EA (10mlX3) was added for extraction. EA phases were combined, anhydrous sodium sulfate was added for drying, filtered, evaporated and purified by column chromatography to obtain 15mg (yield 19%). ¹H NMR (400 MHz, CDCl₃) δ 7.91 (d, *J =* 7.4 Hz, 1H), 7.02 - 6.82 (m, 3H), 5.84 (d, *J=* 7.3 Hz, 1H), 5.78 (s, 1H), 5.24 - 5.13 (m, 1H), 4.93 (s, 2H), 3.90 (s, 3H), 3.75 (t, *J=* 6.7 Hz, 1H), 1.61 (s, 3H), 1.53 (d, *J =* 6.7 Hz, 6H).

### Example 12

### Synthetic route:

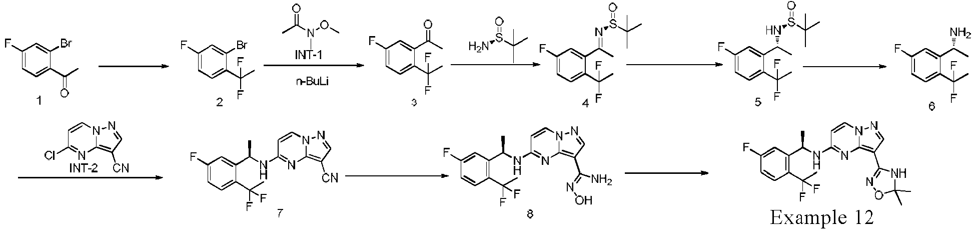

### Reaction steps:

(1) Synthesis of compound 2: BAST (23g, 1.5 eq) was added to compound 1 (15g, 1.0 eq), replaced with nitrogen gas, and reacted at 70 °C for 18h. The raw material monitored by TLC disappeared, then water (100ml) was added to the reaction system, followed by ether (100ml) for extraction, and the ether phase was collected, then 10% citric acid aqueous solution was added for washing and separation. Then sodium bicarbonate aqueous solution was added for washing and separation, and brine was added for washing once, then the organic phase was collected and dried, ether was evaporated at low temperature and purified by column chromatography using pure petroleum ether to obtain 9.6 g compound 2 (yield 58%).
(2) Synthesis of compound 3: anhydrous THF (50ml) was added into compound 2 (5g), cooled to -78 °C, then n-BuLi (10.08 ml, 1.2 eq) was slowly added dropwise, stirred at low temperature for 1h after addition. Then INT-1 (1.6 g, 1.2 eq) in THF was added dropwise, and reacted for 1h at the temperature after addition. TLC monitoring showed that the reaction was completed, ammonium chloride aqueous solution was added to the reaction system for quenching, then EA was added for extraction. EA phase was collected, dried over anhydrous sodium sulfate, filtered, evaporated and purified by column chromatography to obtain 680mg of compound 3 (yield 16%).
(3) Synthesis of compound 4: compound 3 (680mg, 1.0 eq) was dissolved in anhydrous THF (8ml), then R-tert-butyl sulfinamide (814.6 mg, 2eq) was added, followed by tetraethyl titanate (1.56 g, 2eq), and reacted at 60 °C for 2h. TLC monitoring showed that the reaction was completed, the reaction solution was poured into water, solids were precipitated, filtered, the filtrate was collected, extracted with water and EA, the EA phase was collected, dried over anhydrous sodium sulfate, evaporated and purified by column chromatography to obtain 900mg of compound 4 (yield 87%).
(4) Synthesis of compound 5: compound 4 (900mg, 1.0 eq) was dissolved in THF (10ml), cooled to -50 °C, sodium borohydride (224mg, 2eq) was added in batches, and then gradually raised to room temperature for 2h. TLC monitoring showed that the reaction was completed, water was added into the reaction system, then EA (30ml X3) was added for extraction, EA phase was collected, anhydrous sodium sulfate was added for drying, and evaporated and purified by column chromatography to obtain 115mg of compound 5 (yield 12.7%).
(5) Synthesis of compound 6: dioxane hydrochloride (2ml) was added to compound 5 (115mg, 1.0 eq), reacted at room temperature for 2h. TLC monitoring showed that the reaction was completed, the solvent was removed. Saturated sodium bicarbonate aqueous solution was added to adjust pH to be 7 ~ 8, then dichloromethane and methanol were added for extraction for many times. Organic phases were collected, dried over anhydrous sodium sulfate, filtered and evaporated to obtain 80mg compound 6 (yield 95%).
(6) Synthesis of compound 7: anhydrous ethanol (10 ml) was added into compound 6 (80mg, 1.0 eq), followed by INT-2 (84mg, 1.1 eq) and TEA (0.17ml, 3.0 eq), respectively. After replacing with nitrogen, the reaction was carried out at 60 °C for 18h. TLC monitoring showed that the reaction was completed, the ethanol was evaporated. Then water was added to the reaction system, and EA (10ml X3) was added for extraction. EA phases were combined, dried by adding anhydrous sodium sulfate, filtered, evaporated and purified by column chromatography to obtain 120mg of compound 7 (yield 88%).
(4) Synthesis of compound 8: absolute ethanol (1.2 ml) and 1, 4-dioxane (0.4ml) were added to compound 4 (120mg, 1.0 eq), then hydroxylamine hydrochloride (63.9mg, 2.0 eq) and potassium carbonate (127.5mg, 2.0eq) were added, and replaced with nitrogen gas, reacted at 80 °C for 16h. TLC monitoring showed that the reaction was completed, filtered, evaporated and purified by column chromatography directly to obtain 100mg of compound 8 (yield 76%).

Synthesis of Example 12: glacial acetic acid (1ml) and 1, 2-dichloroethane (1ml) were added to compound 8 (100mg, 1.0 eq), then 2, 2-dimethoxypropane (112mg, 4eq) was added, and replaced with nitrogen gas, reacted at 80 °C for 1h. TLC monitoring showed that the reaction was completed, the solvent was evaporated, then sodium bicarbonate aqueous solution was added to the system, pH was adjusted to be 7 ~ 8, then EA (20mlX3) was added for extraction. EA phases were combined, anhydrous sodium sulfate was added for drying, filtered, evaporated and purified by column chromatography to obtain 40mg of Example 14 (yield 36%). ¹H NMR (400 MHz, CDCl₃) δ 8.21 (d, *J =* 7.6 Hz, 1H), 8.19 (s, 1H), 7.50 (d, *J* = 7.0 Hz, 1H), 7.38 (s, 1H), 7.17 - 7.08 (m, 1H), 6.13 (d, *J =* 7.6 Hz, 1H), 5.96 (s, 1H), 5.49 (s, 1H), 5.43 (s, 1H), 1.87 (t, *J=* 18.2 Hz, 3H), 1.67 - 1.58 (m, 6H), 1.53 (s, 3H).

### Example 13

### Synthetic route:

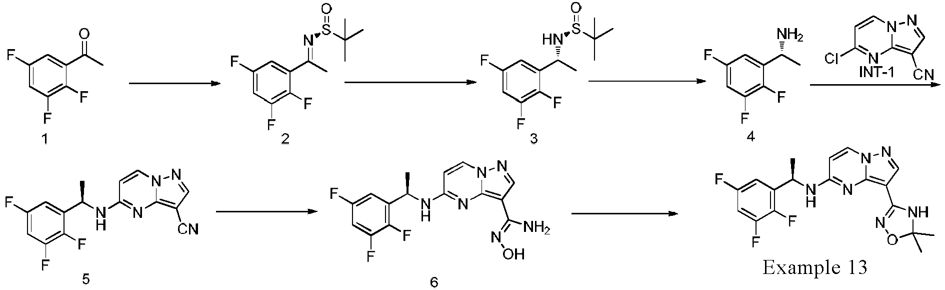

### Reaction steps:

(1) Synthesis of compound 2: compound 1 (3g, 1.0 eq) was dissolved in anhydrous THF (10ml), then R-tert-butyl sulfinamide (4.17g, 2.0eq) was added, followed by tetraethyl titanate (7.86g, 2.0eq), and reacted at 60 °C for 2h. TLC monitoring showed that the reaction was completed, the reaction solution was poured into water, solids were precipitated, filtered, the filtrate was collected, extracted with water and EA (150mgX3). The EA phase was collected, dried over anhydrous sodium sulfate, evaporated and purified by column chromatography to obtain 3.8g of compound 2 (yield 97%).
(2) Synthesis of compound 3: compound 2 (3.8g, 1.0 eq) was dissolved in THF (40ml), cooled to -50 °C, sodium borohydride (1.04g, 2.0eq) was added in batches, and then gradually raised to room temperature to react for 2h. TLC monitoring showed that the reaction was completed, water was added into the reaction system, then EA (100ml X3) was added for extraction. EA phase was collected, anhydrous sodium sulfate was added for drying, and evaporated and purified by column chromatography to obtain 1.2g of compound 3 (yield 31%).
(3) Synthesis of compound 4: dioxane hydrochloride was added into compound 3 (1.2g, 1.0 eq), reacted at room temperature for 2h. TLC monitoring showed that the reaction was completed, the solvent was removed. Saturated sodium bicarbonate aqueous solution was addedto adjust pH to be 7 ~ 8, then dichloromethane and methanol were added for extraction for many times. Organic phases were collected, dried over anhydrous sodium sulfate, filtered and evaporated to obtain 700mg compound 4 (yield 93%).
(4) Synthesis of compound 5: 10ml of anhydrous ethanol was added into compound 4 (700mg, 1.0 eq), followed by INT-1 (783mg, 1.1 eq) and TEA (1.2ml, 3.0 eq), respectively. After replacing with nitrogen, the reaction was carried out at 60 °C for 18h. TLC monitoring showed that the reaction was completed. Then the ethanol was evaporated. Then water was added to the reaction system, and EA (50ml X3) was added for extraction. EA phases were combined, dried by adding anhydrous sodium sulfate, filtered, evaporated and purified by column chromatography to obtain 900mg of compound 5 (yield 71%).
(5) Synthesis of compound 6: absolute ethanol (8ml) and 1, 4-dioxane (4ml) were added to compound 5 (900mg, 1.0 eq), then hydroxylamine hydrochloride (394.6mg, 2.0 eq) and potassium carbonate (783.6mg, 2.0eq) were added, and replaced with nitrogen gas, reacted at 80 °C for 16h. TLC monitoring showed that the reaction was completed, filtered, evaporated and purified by column chromatography directly to obtain 730mg of compound 6 (yield 74%).

Synthesis of Example 13: glacial acetic acid (7ml) and 1, 2-dichloroethane (7ml) were added to compound 8 (700mg, 1.0 eq), then 2, 2-dimethoxypropane (832mg, 4.0eq) was added, and replaced with nitrogen gas, reacted at 80 °C for 1h. TLC monitoring showed that the reaction was completed, the solvent was evaporated, then sodium bicarbonate aqueous solution was added to the system, pH was adjusted to be 7 ~ 8, then EA (30mlX3) was added for extraction. EA phases were combined, anhydrous sodium sulfate was added for drying, filtered, evaporated and purified by column chromatography to obtain 42mg (yield 5%). ¹H NMR (400 MHz, CDCl₃) δ 8.23 (d, *J=* 7.6 Hz, 1H), 8.19 (s, 1H), 6.85 (dd, *J=* 8.3, 4.9 Hz, 2H), 6.17 (d, *J =* 7.6 Hz, 1H), 5.85 (s, 1H), 5.60 (d, *J =* 5.6 Hz, 1H), 5.49 - 5.37 (m, 1H), 1.60 (d, *J =* 7.0 Hz, 6H), 1.51 (s, 3H).

### Example 14

### Synthetic route:

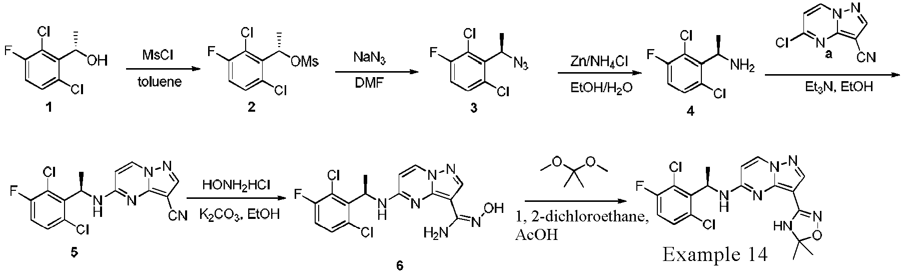

### Reaction steps:

(1) Synthesis of compound 2: 10 g of compound 1 and MsCl (7.1 g, 1.3 eq) were dissolved in toluene solvent in a 250 mL round bottom flask, then triethylamine (7.3 g, 1.5 eq) was added as base, and reacted at room temperature for 4 hours, TLC monitoring showed that the reaction was completed. The organic phase was extracted, dried, evaporated and purified by column chromatography using petroleum ether: ethyl acetate (10: 1) to obtain 13.1 g of pale yellow liquid compound 2.
(2) Synthesis of compound 3: compound 2 (13g) was placed in a 100 ml round bottom flask, DMF (60 ml) was added as solvent, followed by NaN₃ (5.9 g, 2.0 eq), reacted at 50 °C for 3.5 hours, monitored by TLC until it was completed. Extracted with water and ethyl acetate, the organic phase was dried and evaporated. Purified by column chromatography using petroleum ether: ethyl acetate (10: 1) to obtain 10.2 g of compound 3.
(3) Synthesis of compound 4: compound 3 (10.2 g) was placed in a 250 ml round bottom flask. Ethanol (102 ml) and water (34 ml) (3: 1) were added as a mixed solvent, then Zn (3.7 g, 1.3 eq) and NH₄Cl (5.85 g, 2.5 eq) were added, refluxed at 80 °C for 6 hours, the reaction was monitored and completed. The organic phase was filtered, extracted with ethyl acetate, dried and evaporated. Purified by column chromatography using petroleum ether: ethyl acetate (10: 1) to obtain 7.3 g of compound 4.
(4) Synthesis of compound 5: compound 4 (1.5 g) was placed in a 100 ml round-bottom flask, followed by compound a (1.56 g, 1.2 eq), triethylamine (3ml, 3eq) and ethanol (50ml) as solvent, refluxed, and the reaction was monitored and completed after about 2 hours. The small amount of ethanol was evaporated, water and ethyl acetate were added for extraction. Then purified by column chromatography using petroleum ether: ethyl acetate (3: 1) to obtain 2.1 g of compound 5.
(5) Synthesis of compound 6: compound 5 (1.0 g) was placed in a 100 ml round-bottom flask, then hydroxylamine hydrochloride (1.12 g, 5.6 eq) and anhydrous potassium carbonate (2.2 g, 5.6 eq) were added, then ethanol (50ml) was added as solvent, and refluxed overnight at 80 °C, and the reaction was monitored and completed. The small amount of ethanol was evaporated, water and ethyl acetate were added for extraction. Then purified by column chromatography using petroleum ether: ethyl acetate (3: 1) to obtain 0.6 g of compound 6.

**Synthesis of Example 14:** compound 6 (0.2 g) was weighed in a 50 ml round bottom flask, followed by 2, 2-dimethoxypropane (0.22 g, 4 eq), 1, 2-dichloroethane (4 ml) and acetic acid (4 ml) as a mixed solvent, refluxed at 80 °C for 2 hours, and the reaction was monitored and completed. A small amount of water was added, and saturated sodium bicarbonate was added to neutralize acetic acid in the reaction system, then extracted with dichloromethane. Then purified by column chromatography using dichloromethane: methanol (30: 1) to obtain 66 mg of the final compound. ¹H NMR (400 MHz, DMSO) δ: 8.59(d, *J =* 6.0 Hz, 1H), 8.55(d, *J =* 7.6 Hz, 1H), 7.97(s,1H), 7.52(brs, 1H), 7.40(t, *J =* 8.7Hz, 1H), 6.51(d, *J =* 7.6 Hz, 1H), 5.65-5.59(m, 1H),1.59(d, *J =* 7.2 Hz, 3H),1.48(s,3H), 1.39(s,3H).

### Example 15

### Synthetic route:

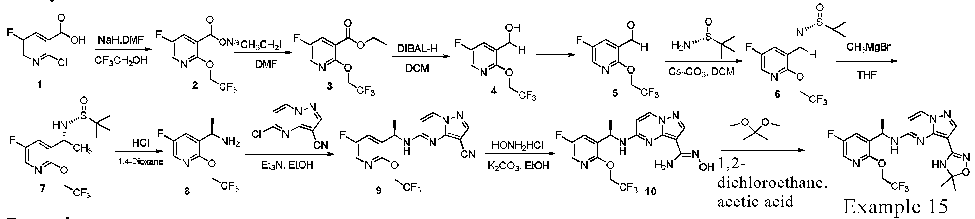

### Reaction steps:

(1) Synthesis of compound 2: DMF (20 ml) as reaction solvent was added into a 100 ml round bottom flask, then the temperature was cooled to 0 °C, NaH (1.71 g, 2.5 eq, 42.9 mmol) was slowly added, and the temperature was cooled for about 30 min after addition. 2-chloro-5-fluoronicotinic acid (3 g, 17.1 mmol) was added in batches, then the reaction was heated to room temperature to react for 4 hours, then raised to 75 °C overnight to obtain compound 2 which was used in the next step without further purification.
(2) Synthesis of compound 3: On the basis of compound 2, iodiethane (4.01 g, 1.5 eq, 25.7 mmol) was added dropwise, and then the reaction was stopped after half an hour. First, a large amount of DMF was removed by evaporation, and then extracted with ethyl acetate, dried over Na₂SO₄. The solvent was evaporated and purified by column chromatography using petroleum ether: ethyl acetate (20: 1) to obtain 1.3 g of compound 3.
(3) Synthesis of compound 4: compound 3 (1.3 g, 4.87 mmol) was placed in a 100 mL three-neck flask, DCM (20 mL) was added as the solvent under nitrogen protection, then the temperature was cooled to -78 °C. After stabilization, DIBAL-H (3.4 mL, 1.05 eq, 5.11 mmol) was added dropwise, and the temperature was kept at -78 °C for about 1 h. The reaction was monitored and completed. Water and methanol was added to quench the reaction to produce insoluble solid, a small amount of NaOH solution was added, and the solid was disappeared. The reaction was extracted with DCM, dried over Na₂SO₄. The solvent was evaporated and purified by column chromatography using petroleum ether: ethyl acetate (20: 1) to obtain 0.63 g of compound 4.
(4) Synthesis of compound 5: compound 4 (0.63 g, 2.8 mmol) was placed in a 100 mL round bottom flask, ethyl acetate (10 mL) was added as solvent, then IBX (1.88 g, 2.4 eq, 6.72 mmol) was added and reacted at 80 °C. The reaction was completed after about 2 hours. Then the reaction was suction filtered by a sand core funnel, washed with ethyl acetate. Filtrate was collected, and concentrated by evaporation to obtain 0.35 g of compound 5. The molecular weight of mass spectrum peak produced by liquid mass was 18 more than that of the compound, which meant binding one water and did not affect the next reaction.
(5) Synthesis of compound 6: compound 5 (0.35 g, 1.57 mmol) was added into a 100 ml round-bottom flask, then (R)-tert-butyl sulfinamide (0.29 g, 1.5 eq, 2.35 mmol) and cesium carbonate (0.36 g, 0.7 eq, 1.1 mmol) were weighed into the round-bottom flask, dichloromethane (10 mL) was added as reaction solvent, reacted at room temperature for about 2 h. The reaction was completed after about 2 hours, dichloromethane was added for extraction, and dried over Na₂SO₄. The solvent was evaporated and purified by column chromatography using petroleum ether: ethyl acetate (10: 1) to obtain 0.6 g of compound 6.
(6) Synthesis of compound 7: compound 6 (0.6 g, 1.84 mmol) was placed in a 100 mL three-neck flask, anhydrous THF (10 mL) was added as the reaction solvent, and the temperature was cooled to -20 °C under nitrogen protection. After the temperature was constant, magnesium methyl bromide (2.2 mL, 1.2 eq, 2.21 mmol) in tetrahydrofuran was slowly added dropwise, and then the temperature was increased to react. After overnight reaction, there was a large amount of raw material remaining, and then methylmagnesium bromide in tetrahydrofuran solution (2.2 mL) was added. After the temperature returned to room temperature, the reaction was monitored and completed. Saturated ammonium chloride aqueous solution was added to quench the reaction. Then extracted with ethyl acetate, dried over Na₂SO₄. The solvent was evaporated and purified by column chromatography using petroleum ether: ethyl acetate (1.5: 1) to obtain 0.2 g of compound 7.
(7) Synthesis of compound 8: compound 7 (0.2 g, 0.3 mmol) was added into a 50 mL round bottom flask, HCl/1, 4-dioxane (3 mL) and methanol (3 mL) were added. The reaction was completed at room temperature for about 1h, and NaHCO₃ solution was added to neutralize the reaction, then ethyl acetate was added for extraction, dried over Na₂SO₄. The solvent was evaporated to obtain 0.1 g of compound 8.
(8) Synthesis of compound 9: compound 8 (0.1 g, 0.42 mmol) was placed in a 50 mL round bottom flask, followed by chlorocyanate (90 mg, 1.2 eq, 0.51 mmol), triethylamine (0.13 g, 3eq) and ethanol (10 mL) as solvent, refluxed, and the reaction was monitored and completed after about 2 hours. The small amount of ethanol was evaporated, water and ethyl acetate were added for extraction. Then purified by column chromatography using petroleum ether: ethyl acetate (3: 1) to obtain 55 mg of compound 9.
(9) Synthesis of compound 10: compound 9 (55 mg, 0.15 mmol) was placed in a 50 ml round-bottom flask, followed by hydroxylamine hydrochloride (56 mg, 5.6 eq) and anhydrous potassium carbonate (113 mg, 5.6 eq), then ethanol (5 ml) was added as solvent, and refluxed at 80 °C, and the reaction was monitored and completed. The small amount of ethanol was evaporated, water and ethyl acetate were added for extraction. Then purified by column chromatography using petroleum ether: ethyl acetate (3: 1) to obtain 41 mg of compound 10.
(10) Synthesis of Example 15: compound 10 (41 mg, 0.1 mmol) was placed in a 50 ml round bottom flask, followed by 2, 2-dimethoxypropane (61.95 mg, 6 eq, 0.6 mmol), 1, 2-dichloroethane (2 mL) and acetic acid (2 mL) as a mixed solvent, refluxed at 80 °C for 2 hours, and the reaction was monitored and completed. A small amount of water was added, and saturated sodium bicarbonate was added to neutralize acetic acid in the reaction system, then extracted with dichloromethane. Then purified by column chromatography using dichloromethane: methanol (30: 1) to obtain 15 mg of the final compound.

### Example 16

### Synthetic route:

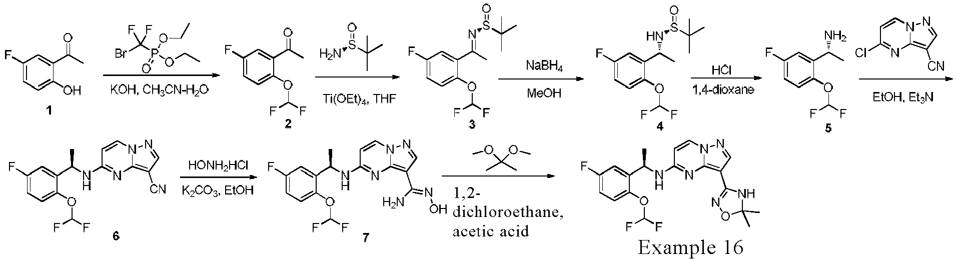

### Reaction steps:

(1) Synthesis of compound 2: compound 1 (5 g, 32.5 mmol) was added into a 250 mL three-neck flask. A mixture of acetonitrile and water (1: 1) (100 mL) was added as the reaction solvent. The temperature was cooled to -78 °C under nitrogen protection. During the cooling process, the reaction system was frozen to one piece at -50 °C. Then diethyl bromofluoromethyl phosphonate (17.3 g, 2 eq, 65 mmol) was slowly added dropwise. The temperature was raised to room temperature after addition, and stirred for about 4 hours. After the reaction was monitored and completed, ethyl acetate was added for extraction, dried over Na₂SO₄. The solvent was evaporatedand purified by column chromatography using petroleum ether: ethyl acetate (19: 1) to obtain 5.6 g of compound 2 which had no mass spectrum absorption peak.
(2) Synthesis of compound 3: compound 2 (3.04 g, 14.9 mmol) was added into a 100 mL round bottom flask, and (R)-tert-butyl sulfinamide (3.62 g, 2 eq, 29.8 mmol) and tetraethyl titanate (8.5 g, 2.5 eq, 37.3 mmol) were added. Anhydrous THF (20 mL) was used as reaction solvent, and reaction was carried out at 80 °C for about 4 hours. After the reaction was monitored and completed, a large amount of solid was produced in the reaction system after adding water, filtered through Celite and washed, then extracted with ethyl acetate, dried over Na₂SO₄. The solvent was evaporated and purified by column chromatography using petroleum ether: ethyl acetate (4: 1) to obtain 4.827 g of compound 3.
(3) Synthesis of compound 4: compound 3 (4.827 g, 15.7 mmol) was added into a 100 mL round bottom flask, anhydrous methanol (10 mL) was used as the solvent, and sodium borohydride (1.487 g, 2.5 eq, 39.3 mmol) was slowly added under ice bath. The temperature was raised and stirred for 30 min after addition. Water and ethyl acetate were slowly added for extraction after the reaction was completed, dried over Na₂SO₄. The solvent was evaporated and purified by column chromatography using petroleum ether: ethyl acetate (1.5: 1) to obtain 1.82 g of compound 4.
(4) Synthesis of compound 5: compound 4 (1.82 g, 0.3 mmol) was added into a 100 mL round bottom flask, HCl/1, 4-dioxane (5 mL) and methanol (5 mL) were added. The reaction was completed at room temperature for about 1h, and NaHCO₃ solution was added to neutralize the reaction, then ethyl acetate was added for extraction, dried over Na₂SO₄. The solvent was evaporated to obtain 1.4 g of compound 6.
(5) Synthesis of compound 6: compound 5 (0.5 g, 2.44 mmol) was placed in a 50 mL round bottom flask, followed by chlorocyanate (0.52 g, 1.2 eq, 2.93 mmol), triethylamine (0.65 mL, 2eq) and ethanol (10 mL) as solvent, refluxed, and the reaction was monitored and completed after about 2 hours. The small amount of ethanol was evaporated, water and ethyl acetate were added for extraction. Then purified by column chromatography using petroleum ether: ethyl acetate (3: 1) to obtain 0.53 g of compound 6.
(6) Synthesis of compound 7: compound 6 (0.53 g, 1.53 mmol) was placed in a 50 ml round-bottom flask, followed by hydroxylamine hydrochloride (0.59 g, 5.6 eq) and anhydrous potassium carbonate (1.18 g, 5.6 eq), then ethanol (10 ml) was added as solvent, and refluxed at 80 °C, and the reaction was monitored and completed. The small amount of ethanol was evaporated, water and ethyl acetate were added for extraction. Then purified by column chromatography using dichloromethane: methanol (30: 1) to obtain 420 mg of compound 7.

**Synthesis of Example 16:** compound 7 (420 mg, 1.1 mmol) was placed in a 50 ml round bottom flask, followed by 2, 2-dimethoxypropane (0.46 g, 4 eq, 4.44 mmol), 1, 2-dichloroethane (3 mL) and acetic acid (3 mL) as a mixed solvent, refluxed at 80 °C, and the reaction was monitored and completed. A small amount of water was added, and saturated sodium bicarbonate was added to neutralize acetic acid in the reaction system, then extracted with dichloromethane. Then purified by column chromatography using dichloromethane: methanol (30: 1) to obtain 26 mg of the final compound.

Examples 1-16 are summarized in Table 1-1 below:

**Table 1-1**

| Serial number | Structural formula | **Characterization data of compounds (MS/HNMR)** |
|---|---|---|
| Example 1 | 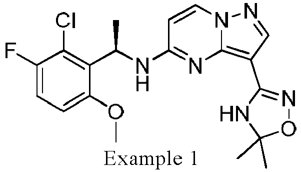 | ¹H NMR (400 MHz, CDCl₃) δ 8.25-8.12 (m, 2H), 7.05 (dd, *J* = 9.1, 8.2 Hz, 1H), 6.80 (dd, *J* = 9.1, 4.0 Hz, 1H), 6.08 (t, *J =* 30.1 Hz, 4H), 3.91 (s, 2H), 1.58 (t, *J* = 5.8 Hz, 8H). |
| Example 2 | 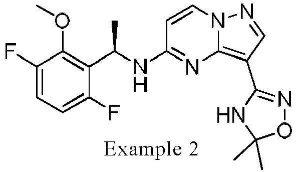 | ¹H NMR (400 MHz, CDCl₃) δ 8.20 (s, 1H), 8.15 (d, *J = 7.6* Hz, 1H), 6.97 (ddd, *J =* 10.8, 9.2, 5.3 Hz, 1H), 6.75 (td, *J* = 9.4, 3.7 Hz, 1H), 6.34 (s, 1H), 6.06 (d, *J =* 7.5 Hz, 1H), 5.79-5.59 (m, 2H), 4.03 (d, *J =* 1.8 Hz, 2H), 1.72-1.64 (m, 5H), 1.60 (s, 3H). |
| Example 3 | 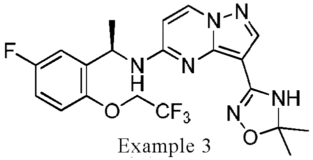 | ¹H NMR (400 MHz, CDCl₃) δ 8.29-8.10 (m, 2H), 7.03 (dd, *J =* 8.6, 2.9 Hz, 1H), 7.00-6.92 (m, 1H), 6.83 (dd, *J* = 9.0, 4.2 Hz, 1H), 6.08 (d, *J* = 7.6 Hz, 1H), 5.79 (s, 1H), 5.51 (d, *J =* 5.5 Hz, 1H), 5.25 (s, 1H), 4.53-4.29 (m, 2H), 1.62 (s, 3H), 1.59 (s, 3H), 1.47 (s, 3H). |
| Example 4 | 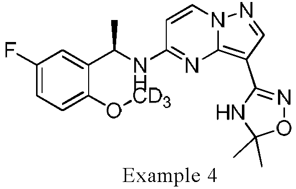 | ¹H NMR (400 MHz, CDCl₃) δ 8.17 (d, *J =* 7.6 Hz, 1H), 8.15 (s, 1H), 6.98 (dd, *J =* 8.8, 3.3 Hz, 1H), 6.95 - 6.88 (m, 1H), 6.86 (dd, *J* = 8.8, 4.4 Hz, 1H), 6.17 (d, *J =* 6.4 Hz, 1H), 5.95 (s, 1H), 5.86 (d, *J* = 5.4 Hz, 1H), 1.62 (s, 3H), 1.54 (d, *J* = 6.9 Hz, 3H), 1.44 (s, 3H). |
| Example 5 | 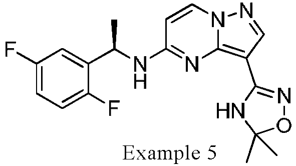 | ¹H NMR (400 MHz, CDCl₃) δ 8.21 (d, *J =* 7.6 Hz, 1H), 8.18 (s, 1H), 7.09 - 6.99 (m, 2H), 6.92 (m, 1H), 6.16 (d, *J* = 7.6 Hz, 1H), 5.94 (s, 1H), 5.55 (d, *J =* 5.7 Hz, 1H), 5.38 (m, 1H), 1.64 (s, 3H), 1.60 (s, 3H), 1.50 (s, 3H). MS: 373 (M + H +). |
| Example 6 | 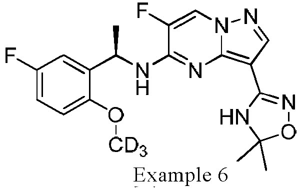 | ¹H NMR (400 MHz, CDCl₃) δ 8.27 (d, *J =* 5.6 Hz, 1H), 8.18 (s, 1H), 6.94 (m, 2H), 6.88 (m, 1H), 5.87 (d, *J =* 6.0 Hz, 1H), 5.71 (s, 1H), 5.36 (m, 1H), 1.62 (m, 6H), 1.44 (s, 3H). |
| Example 7 | 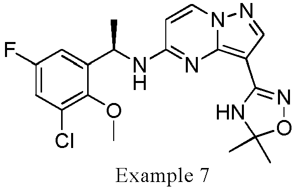 | ¹H NMR (400 MHz, CDCl₃) δ 8.14 (dd, *J* = 7.7, 1.9 Hz, 2H), 7.07 (dt, *J =* 7.9, 3.9 Hz, 1H), 7.01 (dd, *J* = 7.4, 2.6 Hz, 1H), 6.33 - 6.21 (m, 2H), 5.58 (d, *J* = 5.7 Hz, 1H), 3.98 (s, 3H), 1.72 (s, 3H), 1.62 - 1.55 (m, 6H). |
| Example 8 | 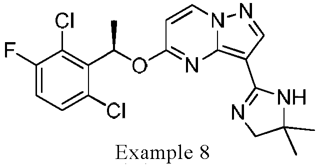 | ¹H NMR (400 MHz, CDCl₃) δ 9.54 (s, 1H), 8.63 (d, *J = 7.5* Hz, 1H), 7.36 - 7.28 (m, 2H), 7.13 (dd, *J =* 8.9, 7.9 Hz, 1H), 6.71 (d, *J* = 7.5 Hz, 1H), 6.61 (q, *J =* 6.8 Hz, 1H), 3.71 (dd, *J =* 27.5, 10.7 Hz, 2H), 1.86 (d, *J =* 6.9 Hz, 3H), 1.58 (d, *J* = 4.4 Hz, 6H). |
| Example 9 | 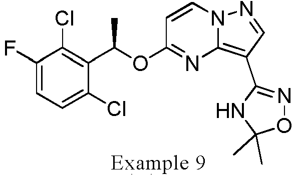 | ¹H NMR (400 MHz, CDCl₃) δ 8.43 (d, *J =* 7.5 Hz, 1H), 8.32 (s, 1H), 7.31 - 7.27 (m, 1H), 7.07 (dd, *J =* 8.8, 8.0 Hz, 1H), 6.59 (q, *J =* 6.9 Hz, 1H), 6.49 (d, *J =* 7.5 Hz, 1H), 5.61 (s, 1H), 1.81 (d, *J =* 6.9 Hz, 3H), 1.63 (s, 3H), 1.55 (s, 3H). |
| Example 10 | 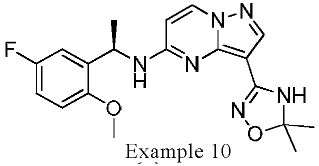 | ¹H NMR (400 MHz, CDCl₃) δ 8.19 (d, *J* = 7.8 Hz, 2H), 6.92 (m, *J* = 13.3, 8.8, 3.7 Hz, 3H), 6.09 (d, *J =* 7.4 Hz, 1H), 5.87 (s, 1H), 5.47 (s, 1H), 5.29 (s, 1H), 3.91 (s, 3H), 1.62 (s, 3H), 1.56 (d, *J =* 6.7 Hz, 6H). |
| Example 11 | 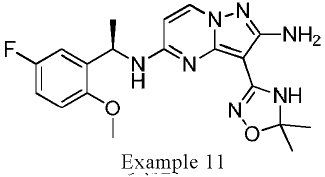 | ¹H NMR (400 MHz, CDCl₃) δ 7.91 (d, *J* = 7.4 Hz, 1H), 7.02 - 6.82 (m, 3H), 5.84 (d, *J =* 7.3 Hz, 1H), 5.78 (s, 1H), 5.24 - 5.13 (m, 1H), 4.93 (s, 2H), 3.90 (s, 3H), 3.75 (t, *J* = 6.7 Hz, 1H), 1.61 (s, 3H), 1.53 (d, *J =* 6.7 Hz, 6H). |
| Example 12 | 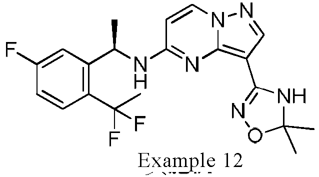 | ¹H NMR (400 MHz, CDCl₃) δ 8.21 (d, *J = 7.6* Hz, 1H), 8.19 (s, 1H), 7.50 (d, *J =* 7.0 Hz, 1H), 7.38 (s, 1H), 7.17 - 7.08 (m, 1H), 6.13 (d, *J =* 7.6 Hz, 1H), 5.96 (s, 1H), 5.49 (s, 1H), 5.43 (s, 1H), 1.87 (t, *J =* 18.2 Hz, 3H), 1.67 - 1.58 (m, 6H), 1.53 (s, 3H). |
| Example 13 | 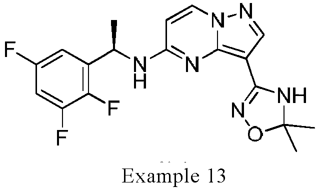 | ¹H NMR (400 MHz, CDCl₃) δ 8.23 (d, *J = 7.6* Hz, 1H), 8.19 (s, 1H), 6.85 (dd, *J =* 8.3, 4.9 Hz, 2H), 6.17 (d, *J =* 7.6 Hz, 1H), 5.85 (s, 1H), 5.60 (d, *J =* 5.6 Hz, 1H), 5.49 - 5.37 (m, 1H), 1.60 (d, *J* = 7.0 Hz, 6H), 1.51 (s, 3H). |
| Example 14 | 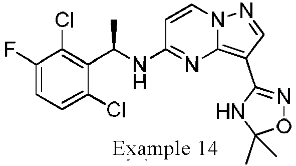 | ¹H NMR (400 MHz, DMSO) δ: 8.59(d, *J =* 6.0 Hz, 1H), 8.55(d, *J = 7.6* Hz, 1H), 7.97(s,1H), 7.52(brs, 1H), 7.40(t, *J =* 8.7Hz, 1H), 6.51(d, *J=* 7.6 Hz, 1H), 5.65-5.59(m, 1H),1.59(d, *J* = 7.2 Hz, 3H),1.48(s,3H), 1.39(s,3H). |
| Example 15 | 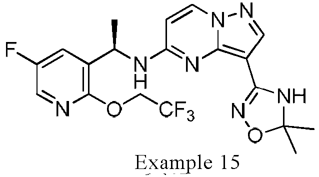 | [M + H]⁺ |
| | | 454.1 |
| Example 16 | 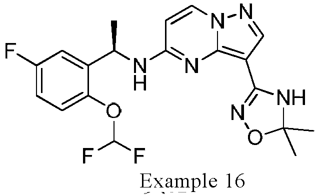 | [M + H]⁺ |
| | | 421.1 |

Meanwhile, with reference to the above examples, examples 17-83 were synthesized, as detailed in Table 1-2:

**Table 1-2**

| **Example** | **Structural formula of compound** | **Characterization data of compounds (MS/HNMR)** |
|---|---|---|
| **17** | 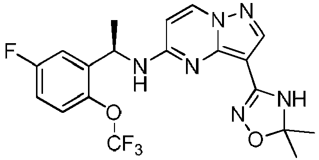 | ¹H NMR (400 MHz, CDCl₃) δ 8.30 - 8.16 (m, 2H), 7.32 - 7.23 (m, 1H), 7.12 (dd, *J =* 8.8, 3.1 Hz, 1H), 7.00 (ddd, *J* = 9.0, 7.5, 3.1 Hz, 1H), 6.12 (d, *J =* 7.6 Hz, 1H), 5.74 (s, 1H), 5.38 (m,, 2H), 1.60 (m, 3H), 1.57 (m, 6H). |
| **18** | 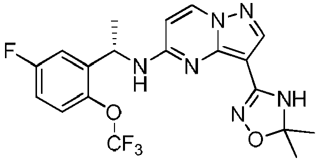 | ¹H NMR (400 MHz, CDCl₃) δ 8.28 - 8.11 (m, 2H), 7.27 (m, 1H), 7.12 (dd, *J =* 8.8, 3.0 Hz, 1H), 7.06 - 6.96 (m, 1H), 6.13 (d, *J =* 7.6 Hz, 1H), 5.75 (s, 1H), 5.46 (m, 1H), 5.37 (s, 1H), 1.69 - 1.53 (m, 9H). |
| **19** | 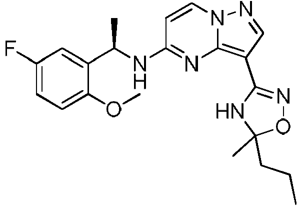 | ¹H NMR (400 MHz, CDCl₃) δ 8.18 (dd, *J* = 7.6, 1.1 Hz, 1H), 8.16 (s, 1H), 6.99 - 6.94 (m, 1H), 6.91 (dd, *J =* 7.8, 3.0 Hz, 1H), 6.86 (dd, *J =* 8.9, 4.4 Hz, 1H), 6.12 (d, *J =* 6.9 Hz, 1H), 5.88 (s, 1H), 5.66 (d, *J=* 6.5 Hz, 1H), 3.90 (d, *J* = 2.8 Hz, 3H), 1.64 (m, 2H), 1.55 (m, 6H), 1.41 (m, 2H), 1.00 (t, *J* = 7.4 Hz, 3H). |
| **20** | 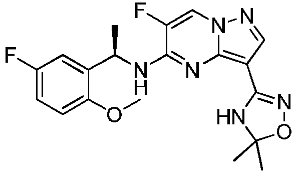 | ¹H NMR (400 MHz, CDCl₃) δ 8.27 (d, *J =* 5.6 Hz, 1H), 8.19 (s, 1H), 6.98 - 6.91 (m, 2H), 6.91 - 6.85 (m, 1H), 5.85 (d, *J =* 6.0 Hz, 1H), 5.71 (s, 1H), 5.36 (p, *J =* 6.8 Hz, 1H), 3.91 (s, 3H), 1.62 (m, 6H), 1.44 (s, 3H). |
| **21** | 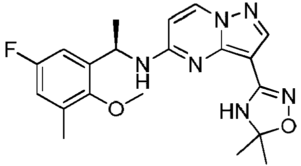 | ¹H NMR (400 MHz, CDCl₃) δ 8.18 (s, 1H), 8.16 (d, *J* = 7.6 Hz, 1H), 6.85 (dd, *J =* 8.8, 3.2 Hz, 1H), 6.79 (dd, *J* = 8.8,3.2 Hz, 1H), 6.26 (s, 1H), 6.11 (d, *J =* 7.6 Hz, 1H), 5.61 (d, *J* = 6.8 Hz, 1H), 3.85 (s, 3H), 2.32 (s, 3H), 1.67 (s, 3H), 1.59 (d, *J =* 6.8 Hz, 3H), 1.57 (s, 3H) |
| **22** | 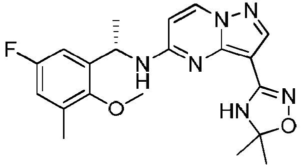 | ¹H NMR (400 MHz, CDCl₃) δ 8.17 (s, 1H), 8.14 (d, *J =* 7.6 Hz, 1H), 6.87 (dd, *J =* 8.8, 3.2 Hz, 1H), 6.78 (dd, *J =* 8.8, 3.2 Hz, 1H), 6.31 (s, 1H), 6.14 (d, *J =* 7.6 Hz, 1H), 5.80 (d, *J =* 6.8 Hz, 1H), 5.54 (s, 1H), 3.85 (s, 3H), 2.32 (s, 3H), 1.68 (s, 3H), 1.59 (d, *J =* 7.2 Hz, 6H). |
| **23** | 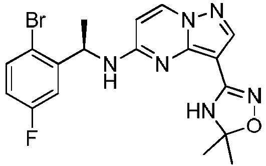 | ¹H NMR (400 MHz, CDCl₃) δ 8.21 (d, *J* = 7.6 Hz, 1H), 8.16 (s, 1H), 7.53 (dd, *J* = 8.8, 5.2 Hz, 1H), 7.13 (dd, *J* = 9.2, 3.2 Hz, 1H), 6.86 (m, 1H), 6.28 (d, *J =* 7.2 Hz, 1H), 6.12 (d, *J* = 4.8 Hz, 1H), 5.79 (s, 1H), 5.39 - 5.28 (m, 1H), 1.65 (s, 3H), 1.55 (d, *J* = 6.8 Hz, 3H), 1.43 (s, 3H). |
| **24** | 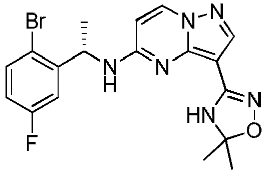 | ¹H NMR (400 MHz, CDCl₃) δ 8.23 (d, *J =* 7.6 Hz, 1H), 8.18 (s, 1H), 7.54 (dd, *J =* 8.8, 5.2 Hz, 1H), 7.10 (dd, *J =* 9.2, 3.2 Hz, 1H), 6.87 (m, 1H), 6.21 (d, *J =* 6.8 Hz, 1H), 5.71 (d, *J =* 6.4 Hz, 2H), 5.34 (s, 1H), 1.65 (s, 3H), 1.56 (d, *J =* 6.8 Hz, 4H), 1.43 (s, 3H). |
| **25** | 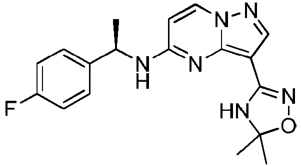 | ¹H NMR (400 MHz, CDCl₃) δ 8.19 (d, *J =* 7.6 Hz, 1H), 8.16 (s, 1H), 7.32 (dd, *J =* 8.8, 5.2 Hz, 2H), 7.04 (t, *J =* 8.8 Hz, 2H), 6.14 (d, *J =* 7.6 Hz, 1H), 5.67 (s, 1H), 5.57 (d, *J =* 4.4 Hz, 1H), 4.99 (s, 1H), 1.60 (s, 3H), 1.59 (d, *J = 7.2* Hz, 3H),1.41 (s, 3H). |
| **26** | 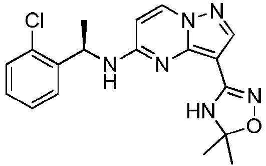 | ¹H NMR (400 MHz, CDCl₃) δ 8.18 (d, *J =* 7.6 Hz, 1H), 8.15 (s, 1H), 7.41 - 7.34 (m, 2H), 7.26 - 7.17 (m, 2H), 6.22 (d, *J =* 7.6 Hz, 1H), 5.83 (d, *J =* 5.6 Hz, 2H), 5.47 (s, 1H), 1.64 (s, 3H), 1.56 (d, *J =* 6.8 Hz, 3H), 1.42 (s, 3H). |
| **27** | 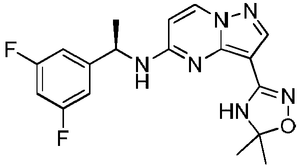 | ¹H NMR (400 MHz, CDCl₃) δ 8.21 (d, *J =* 7.6 Hz, 1H), 8.15 (s, 1H), 6.93 - 6.84 (m, 2H), 6.71 (m, 1H), 6.23 (d, *J* = 7.6 Hz, 1H), 5.86 (d, *J* = 4.8 Hz, 1H), 5.61 (s, 1H), 5.01 - 4.90 (m, 1H), 1.63 (s, 3H), 1.57 (d, *J =* 7.2 Hz, 3H), 1.39 (s, 3H). |
| **28** | 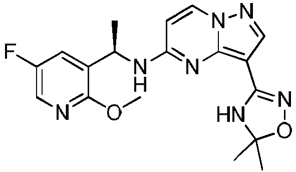 | ¹H NMR (400 MHz, CDCl₃) δ 8.14 (d, *J* = 7.6 Hz, 1H), 8.09 (s, 1H), 7.83 (d, *J =* 2.9 Hz, 1H), 7.24 (dd, *J =* 8.1, 2.9 Hz, 1H), 6.13 (d, *J =* 7.3 Hz, 1H), 5.72 (d, *J =* 5.9 Hz, 1H), 5.62 (s, 1H), 5.17 - 5.04 (m, 1H), 3.97 (s, 3H), 1.52 (s, 3H), 1.49 (d, *J =* 6.9 Hz, 3H), 1.33 (s, 3H). |
| **29** | 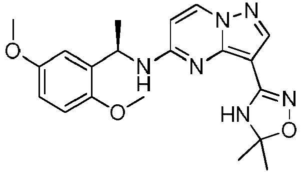 | ¹H NMR (400 MHz, CDCl₃) δ 8.15 (d, *J =* 7.8 Hz, 2H), 6.87 (d, *J* = 8.8 Hz, 1H), 6.83 (d, *J* = 3.0 Hz, 1H), 6.74 (dd, *J =* 8.8, 3.0 Hz, 1H), 6.10 (d, *J =* 7.5 Hz, 1H), 6.00 (s, 1H), 5.69 (t, *J =* 10.1 Hz, 1H), 5.30 (s, 1H), 3.88 (s, 3H), 3.73 (s, 3H), 1.63 (s, 3H), 1.55 (d, *J =* 6.8 Hz, 3H), 1.47 (s, 3H). |
| **30** | 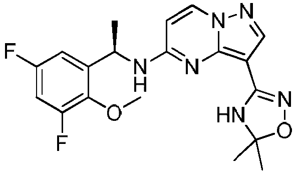 | ¹H NMR (400 MHz, CDCl₃) δ 8.16 (t, *J =* 3.8 Hz, 2H), 6.86 (ddd, *J =* 8.8, 2.9, 1.8 Hz, 1H), 6.75 (ddd, *J =* 11.1, 8.1, 3.0 Hz, 1H), 6.27 - 6.10 (m, 3H), 5.54 (s, 1H), 4.00 (s, 3H), 1.68 (s, 3H), 1.56 (d, *J =* 7.0 Hz, 3H), 1.53 (s, 3H). |
| **31** | 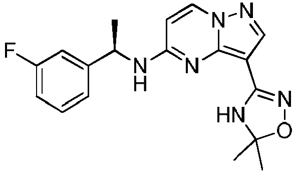 | ¹H NMR (400 MHz, CDCl₃) δ 8.21 (d, *J =* 7.6 Hz, 1H), 8.17 (s, 1H), 7.33 (td, *J =* 7.9, 5.9 Hz, 1H), 7.13 (d, *J =* 7.7 Hz, 1H), 7.08 - 7.01 (m, 1H), 6.97 (tdd, *J* = 8.5, 2.5, 0.8 Hz, 1H), 6.15 (d, *J* = 7.6 Hz, 1H), 5.61 (s, 1H), 5.48 (d, *J* = 4.5 Hz, 1H), 5.08 - 4.91 (m, 1H), 1.62 (s, 3H), 1.60 (s, 3H), 1.37 (s, 3H). |
| **32** | 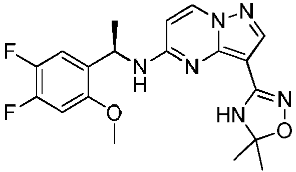 | ¹H NMR (400 MHz, CDCl₃) δ 8.19 (d, *J =* 7.6 Hz, 1H), 8.16 (s, 1H), 7.09 (dd, *J =* 10.7, 8.9 Hz, 1H), 6.76 (dd, *J =* 11.7, 6.5 Hz, 1H), 6.15 (d, *J =* 6.7 Hz, 1H), 5.89 (s, 1H), 5.74 (d, *J =* 6.4 Hz, 1H), 5.29 - 5.21 (m, 1H), 3.89 (s, 3H), 1.61 (s, 3H), 1.53 (d, *J =* 6.8 Hz, 3H), 1.47 (s, 3H). |
| **33** | 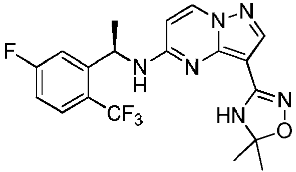 | ¹H NMR (400 MHz, CDCl₃) δ 8.19 (d, *J =* 4.3 Hz, 2H), 7.71 (dd, *J =* 8.7, 5.4 Hz, 1H), 7.37 (dd, *J =* 9.2, 1.3 Hz, 1H), 7.11 - 7.00 (m, 1H), 6.29 (d, *J =* 7.1 Hz, 1H), 6.05 (s, 1H), 5.80 (s, 1H), 5.44 (t, *J =* 8.9 Hz, 1H), 1.59 (s, 3H), 1.42 (s, 3H), 1.26 (s, 3H). |
| **34** | 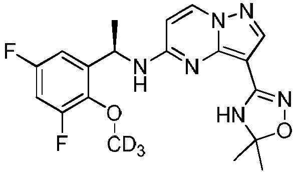 | ¹H NMR (400 MHz, CDCl₃) δ 8.16 (t, *J =* 3.8 Hz, 2H), 6.86 (ddd, *J =* 8.8, 2.9, 1.8 Hz, 1H), 6.75 (ddd, *J =* 11.1, 8.1, 3.0 Hz, 1H), 6.21 (d, *J* = 7.6 Hz, 1H), 6.17 (s, 1H), 6.11 (d, *J =* 4.9 Hz, 1H), 5.52 (s, 1H), 1.68 (s, 3H), 1.56 (d, *J =* 7.0 Hz, 3H), 1.53 (s, 3H). |
| **35** | 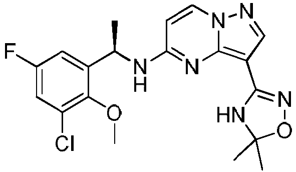 | ¹H NMR (400 MHz, CDCl₃) δ 8.14 (dd, *J =* 7.7, 1.9 Hz, 2H), 7.07 (dt, *J* = 7.9, 3.9 Hz, 1H), 7.03 - 6.99 (m, 1H), 6.49 (d, *J* = 45.3 Hz, 1H), 6.32 - 6.19 (m, 2H), 5.58 (d, *J =* 5.7 Hz, 1H), 3.98 (s, 3H), 1.72 (s, 3H), 1.61 - 1.56 (m, 6H). |
| **36** | 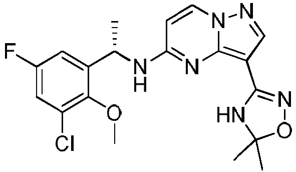 | ¹H NMR (400 MHz, CDCl₃) δ 8.18 (d, *J =* 8.1 Hz, 2H), 7.03 (dd, *J =* 7.7, 3.0 Hz, 1H), 6.98 (dd, *J =* 8.7, 3.1 Hz, 1H), 6.14 (t, *J =* 4.9 Hz, 2H), 5.76 (d, *J =* 6.4 Hz, 1H), 5.62 - 5.50 (m, 1H), 3.98 (s, 3H), 1.69 (s, 3H), 1.60 - 1.56 (m, 6H). |
| **37** | 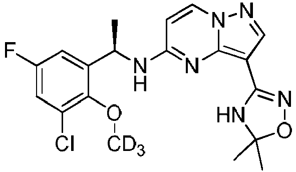 | ¹H NMR (400 MHz, CDCl₃) δ 8.15 (d, *J =* 7.5 Hz, 2H), 7.03 - 7.01 (m, 2H), 6.25 - 6.23 (m, 3H), 5.57 (d, *J =* 5.6 Hz, 1H), 1.71 (s, 3H), 1.58 (d, *J =* 7.0 Hz, 6H). |
| **38** | 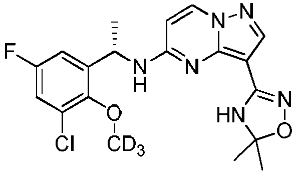 | ¹H NMR (400 MHz, CDCl₃) δ 8.16 (m, 2H), 7.03 (s, 1H), 7.01 - 7.00 (m, 1H), 6.19 (d, *J* = 7.5 Hz, 2H), 6.08 (d, *J =* 5.7 Hz, 1H), 5.62 - 5.51 (m, 1H), 1.70 (s, 3H), 1.60 - 1.56 (m, 6H). |
| **39** | 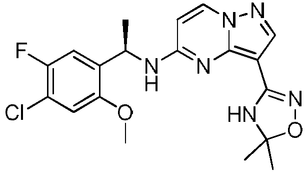 | ¹H NMR (400 MHz, CDCl₃) δ 8.19 (d, *J =* 7.6 Hz, 1H), 8.16 (s, 1H), 7.07 (d, *J =* 9.3 Hz, 1H), 6.93 (d, *J* = 5.9 Hz, 1H), 6.16 (d, *J* = 6.0 Hz, 1H), 5.84 (s, 1H), 5.78 (d, *J =* 6.1 Hz, 1H), 5.29 - 5.20 (m, 1H), 3.91 (s, 3H), 1.61 (s, 3H), 1.53 (d, *J =* 6.9 Hz, 3H), 1.45 (s, 3H). |
| **40** | 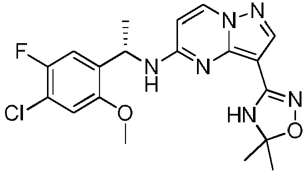 | ¹H NMR (400 MHz, CDCl₃) δ 8.20 (d, *J =* 7.6 Hz, 1H), 8.17 (s, 1H), 7.06 (d, *J = 9.3* Hz, 1H), 6.93 (d, *J* = 5.9 Hz, 1H), 6.13 (d, *J =* 6.7 Hz, 1H), 5.82 (s, 1H), 5.66 (d, *J =* 6.3 Hz, 1H), 5.28 - 5.18 (m, 1H), 1.61 (s, 3H), 1.54 (d, *J =* 6.9 Hz, 3H), 1.46 (s, 3H). |
| **41** | 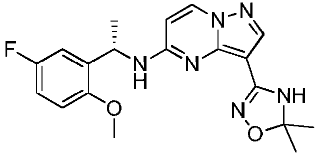 | ¹H NMR (400 MHz, CDCl₃) δ 8.19 (d, *J =* 7.7 Hz, 2H), 6.94 (m, 3H), 6.09 (d, *J* = 7.5 Hz, 1H), 5.87 (s, 1H), 5.47 (s, 1H), 5.30 (s, 1H), 3.91 (s, 3H), 1.62 (s, 3H), 1.56 (d, *J*= 3.4 Hz, 6H). |
| **42** | 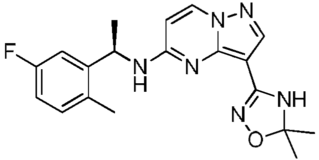 | ¹H NMR (400 MHz, CDCl₃) δ 8.26 - 8.13 (m, 2H), 7.16 (dd, *J =* 8.3, 5.7 Hz, 1H), 7.03 (dd, *J* = 9.9, 2.7 Hz, 1H), 6.87 (td, *J =* 8.3, 2.8 Hz, 1H), 6.13 (d, *J =* 6.8 Hz, 1H), 5.42 (d, *J =* 28.0 Hz, 2H), 5.12 (s, 1H), 2.44 (s, 3H), 1.61 (s, 3H), 1.58 (s, 3H), 1.54 (d, *J =* 6.8 Hz, 3H). |
| **43** | 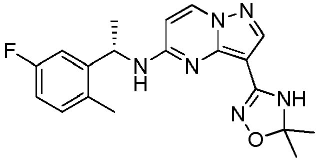 | ¹H NMR (400 MHz, CDCl₃) δ 8.28 - 8.10 (m, 2H), 7.16 (dd, *J =* 8.3, 5.7 Hz, 1H), 7.02 (dt, *J =* 9.3, 4.6 Hz, 1H), 6.87 (td, *J =* 8.2, 2.7 Hz, 1H), 6.11 (d, *J* = 7.1 Hz, 1H), 5.37 (d, *J* = 55.7 Hz, 2H), 5.12 (s, 1H), 2.44 (s, 3H), 1.61 (s, 3H), 1.56 (s, 3H), 1.54 (d, *J =* 6.9 Hz, 3H). |
| **44** | 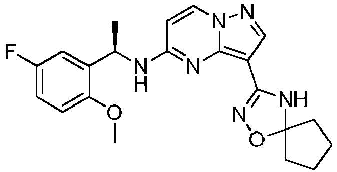 | ¹H NMR (400 MHz, CDCl₃) δ 8.20 (d, *J =* 7.1 Hz, 2H), 6.98 - 6.81 (m, 3H), 6.08 (d, *J =* 7.3 Hz, 1H), 5.93 (s, 1H), 5.46 (s, 1H), 5.28 (s, 1H), 3.89 (s, 3H), 2.27 - 1.64 (m, 8H), 1.26 (s, 3H). |
| **45** | 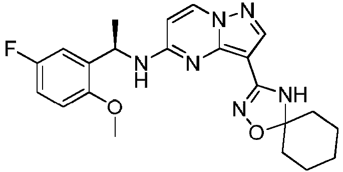 | ¹H NMR (400 MHz, CDCl₃) δ 8.19 (t, *J =* 3.8 Hz, 2H), 7.01 - 6.81 (m, 3H), 6.07 (d, *J =* 7.4 Hz, 2H), 5.55 (d, *J* = 6.8 Hz, 1H), 5.30 (s, 1H), 3.90 (s, 3H), 1.84 (ddd, *J =* 42.1, 36.8, 11.4 Hz, 8H), 1.42 (d, *J* = 4.6 Hz, 2H), 1.26 (s, 3H). |
| **46** | 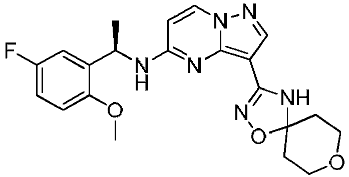 | ¹H NMR (400 MHz, CDCl₃) δ 8.26 - 8.13 (m, 2H), 7.02 - 6.84 (m, 3H), 6.17 - 6.02 (m, 2H), 5.46 (s, 1H), 5.32 (s, 1H), 3.93 (s, 3H), 3.83 (ddd, *J* = 19.8, 9.0, 5.2 Hz, 4H), 2.00 (ddd, *J =* 20.9, 13.3, 4.5 Hz, 4H), 1.26 (s,3H). |
| **47** | 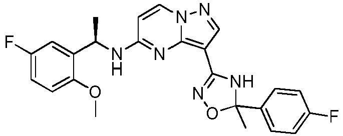 | ¹H NMR (400 MHz, CDCl₃) δ 8.26 - 8.15 (m, 2H), 7.73 - 7.60 (m, 1H), 7.39 (s, 1H), 7.08 (t, *J =* 8.7 Hz, 1H), 7.03 - 6.69 (m, 4H), 6.59 (s, 1H), 6.12 (d, *J =* 5.2 Hz, 1H), 5.38-5.24 (m, 2H), 3.69 (s, 3H), 1.53 (dd, *J =* 6.7, 3.7 Hz, 3H), 1.26 (s, 3H). |
| **48** | 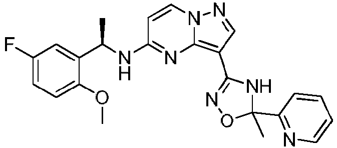 | ¹H NMR (400 MHz, CDCl₃) δ 8.55 (dd, *J =* 22.0, 4.5 Hz, 1H), 8.19 - 8.10 (m, 2H), 7.84 - 7.58 (m, 2H), 7.40 (d, *J =* 40.0 Hz, 1H), 7.21 (dd, *J =* 11.8, 5.9 Hz, 1H), 7.13 - 6.75 (m, 3H), 6.02 (d, *J =* 7.3 Hz, 1H), 5.82 (dd, *J* = 17.0, 10.2 Hz, 1H), 5.34 (s, 1H), 3.87 (t, *J* = 7.3 Hz, 3H), 1.95 (d, *J* = 12.4 Hz, 3H), 1.61 (dd, *J =* 11.8, 6.8 Hz, 3H). |
| **49** | 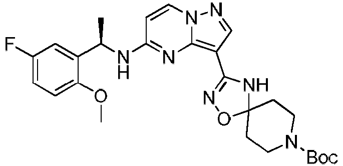 | ¹H NMR (400 MHz, CDCl₃) δ 8.17 (dd, *J =* 19.6, 6.8 Hz, 2H), 7.05 - 6.77 (m, 3H), 6.15 (d, *J =* 6.6 Hz, 1H), 6.03 (s, 1H), 5.62 (t, *J =* 9.1 Hz, 1H), 5.30 (s, 1H), 3.87 (s, 3H), 3.79 - 3.17 (m, 4H), 2.13 - 1.62 (m, 4H), 1.48 (d, *J =* 17.8 Hz, 9H), 1.26 (s, 3H). |
| **50** | 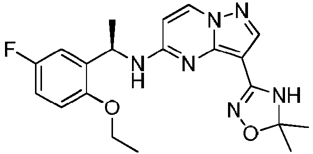 | ¹H NMR (400 MHz, CDCl₃) δ 8.25 - 8.16 (m, 2H), 6.98 - 6.82 (m, 3H), 6.08 (d, *J =* 7.6 Hz, 1H), 5.87 (s, 1H), 5.47 (s, 1H), 5.28 (s, 1H), 4.20 - 4.01 (m, 2H), 1.61 (s, 3H), 1.58 (s, 3H), 1.47 (t, *J* = 7.0 Hz, 6H). |
| **51** | 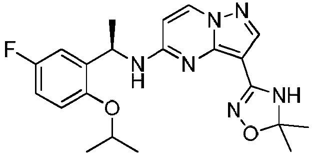 | ¹H NMR (400 MHz, CDCl₃) δ 8.26 - 8.13 (m, 2H), 6.98 - 6.81 (m, 3H), 6.05 (d, *J =* 7.6 Hz, 1H), 5.87 (s, 1H), 5.51 (s, 1H), 5.20 (s, 1H), 4.59 (dt, *J* = 12.1, 6.0 Hz, 1H), 1.62 (s, 3H), 1.57 (s, 6H), 1.41 (d, *J* = 6.1 Hz, 3H), 1.36 (d, *J* = 6.0 Hz, 3H). |
| **52** | 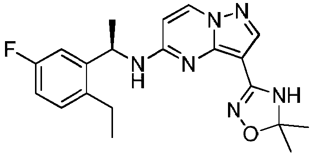 | ¹H NMR (400 MHz, CDCl₃) δ 8.21 (d, *J* = 6.8 Hz, 2H), 7.22 (dd, *J =* 8.5, 5.8 Hz, 1H), 7.07 (dd, *J =* 10.0, 2.7 Hz, 1H), 6.94 (td, *J* = 8.3, 2.7 Hz, 1H), 6.06 (d, *J* = 7.7 Hz, 1H), 5.62 (s, 1H), 5.28 (d, *J* = 19.7 Hz, 2H), 2.88 - 2.66 (m, 2H), 1.59 (d, *J =* 7.5 Hz, 6H), 1.43 (s, 3H), 1.31 (t, *J =* 7.6 Hz, 3H). |
| **53** | 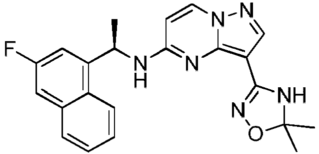 | ¹H NMR (400 MHz, CDCl₃) δ 8.22 (d, *J* = 7.6 Hz, 1H), 8.12 (s, 2H), 7.88 (dd, *J =* 6.0, 3.6 Hz, 1H), 7.65 - 7.54 (m, 2H), 7.35 (ddd, *J =* 20.2, 9.5, 2.5 Hz, 2H), 6.28 (s, 1H), 5.81 (s, 2H), 5.19 (s, 1H), 1.71 (d, *J =* 6.6 Hz, 3H), 1.22 (d, *J* = 31.4 Hz,6H). |
| **54** | 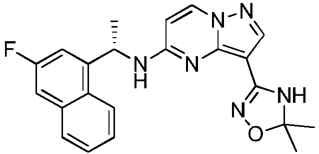 | ¹H NMR (400 MHz, CDCl₃) δ 8.24 (d, *J* = 7.6 Hz, 1H), 8.14 (s, 2H), 7.88 (dd, *J* = 6.0, 3.6 Hz, 1H), 7.59 (dd, *J* = 6.4, 3.3 Hz, 2H), 7.35 (ddd, *J =* 23.9, 9.5, 2.5 Hz, 2H), 6.24 (s, 1H), 5.84 (s, 1H), 5.60 (d, *J =* 4.1 Hz, 1H), 5.16 (s, 1H), 1.72 (d, *J* = 6.9 Hz, 3H), 1.22 (d, *J =* 32.7 Hz, 6H). |
| **55** | 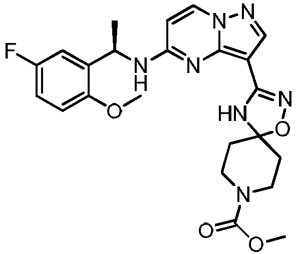 | ¹H NMR (400 MHz, CH₃Cl) δ: 8.20 (d, *J* = 7.6 Hz, 1H), 8.16 (s,1H), 6.94 (t, *J* = 10.7 Hz, 2H), 6.85 (s, 1H), 6.14 (d, *J =* 7.0 Hz, 1H), 6.05 (s, 1H), 5.61 (d,*J =* 5.9 Hz, 1H), 5.30 (s, 1H), 3.92 (s, 1H), 3.86 (s,3H), 3.75 (s, 3H), 3.39-3.31 (m, 2H), 2.08 (d, *J =* 12.6 Hz, 1H), 1.90-1.75 (m, 2H), 1.64 (s, 1H), 1.54 (d, *J =* 6.8 Hz, 3H). |
| **56** | 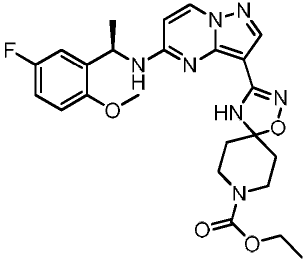 | ¹H NMR (400 MHz, CH₃Cl) δ: 8.21 (d, *J* = 7.6 Hz, 1H), 8.17 (s,1H), 6.93 (t,J = 9.3 Hz, 2H), 6.85 (d, *J* = 3.4 Hz, 1H), 6.12 (d, *J* = 7.0 Hz, 1H), 6.02 (s, 1H), 5.47 (s, 1H), 5.30 (s, 1H), 4.20- 4.10 (m, 2H), 3.96 (m, 2H), 3.86 (s, 3H), 3.39-3.27 (m, 2H), 2.09-2.04 (m, 1H), 1.91 (d, J = 11.5 Hz, 1H), 1.80-1.74 (m, 1H), 1.58 (s, 1H), 1.54 (d, *J =* 6.8 Hz, 3H), 1.33-1.28 (m, 3H). |
| **57** | 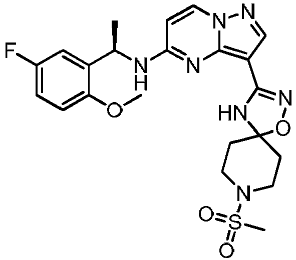 | ¹H NMR (400 MHz, CH₃Cl) δ: 8.21 (d, *J* = 7.6 Hz, 1H), 8.15 (s,1H), 6.94 (d,J = 6.7 Hz, 3H), 6.15 (d, *J* = 6.9 Hz, 1H), 6.06 (s, 1H), 5.51 (s, 1H), 5.30 (s, 1H), 3.94 (s, 3H), 3.85- 3.81 (m, 1H), 3.76-3.73 (m, 1H), 3.13-3.03 (m, 2H), 2.84 (s, 3H), 2.22-2.18 (m, 2H), 2.04-1.91 (m, 2H), 1.54 (d, J = 6.8 Hz, 2H), 1.41 (d, *J=* 9.2 Hz, 1H). |
| **58** | 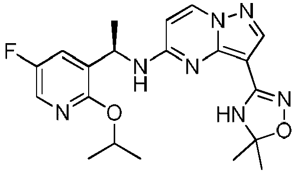 | [M + H]⁺ |
| | | 414.2 |
| **59** | 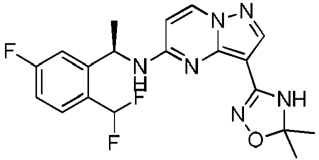 | [M + H]⁺ |
| | | 405.1 |
| **60** | 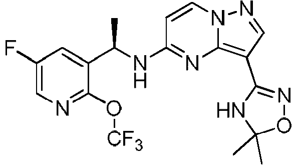 | [M + H]⁺ |
| | | 440.1 |
| **61** | 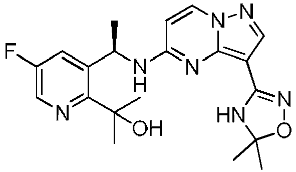 | [M + H]⁺ |
| | | 414.2 |
| **62** | 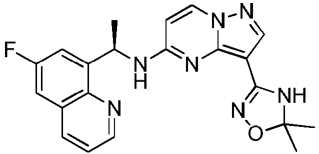 | [M + H]⁺ |
| | | 406.2 |
| **63** | 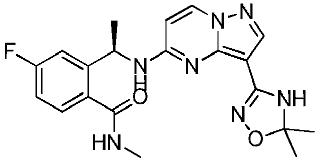 | [M + H]⁺ |
| | | 412.2 |
| **64** | 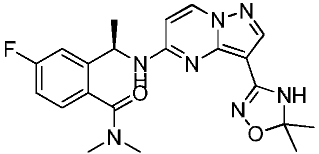 | [M + H]⁺ |
| | | 426.2 |
| **65** | 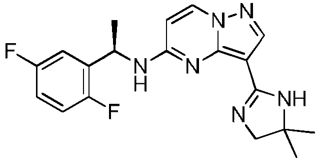 | [M + H]⁺ |
| | | 371.2 |
| **66** | 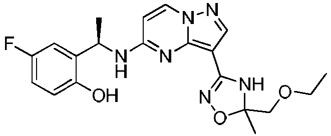 | [M + H]⁺ |
| | | 415.2 |
| **67** | 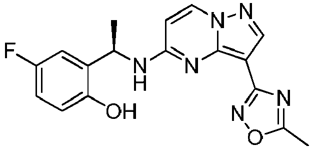 | [M + H]⁺ |
| | | 355.1 |
| **68** | 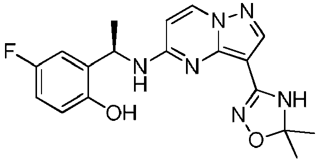 | [M + H]⁺ |
| | | 371.1 |
| **69** | 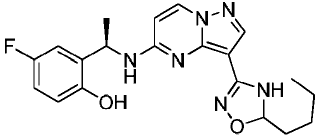 | [M + H]⁺ |
| | | 399.2 |
| **70** | 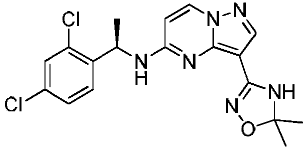 | [M + H]⁺ |
| | | 405.1/407.1 |
| **71** | 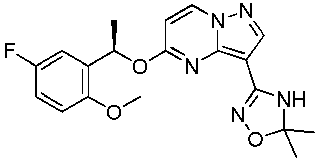 | [M + H]⁺ |
| | | 386.1 |
| **72** | 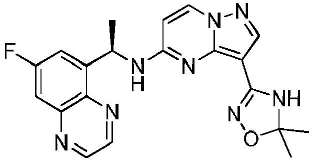 | [M + H]⁺ |
| | | 407.2 |
| **73** | 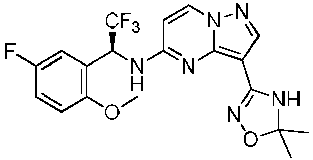 | [M + H]⁺ |
| | | 439.1 |
| **74** | 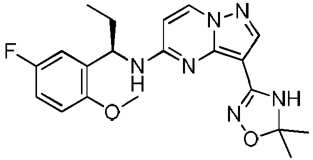 | [M + H]⁺ |
| | | 399.2 |
| **75** | 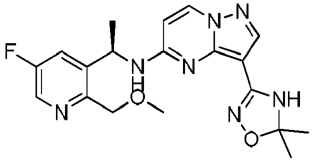 | [M + H]⁺ |
| | | 400.2 |
| **76** | 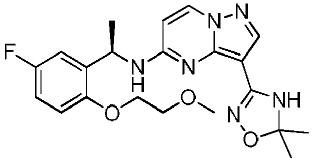 | [M + H]⁺ |
| | | 429.2 |
| **77** | 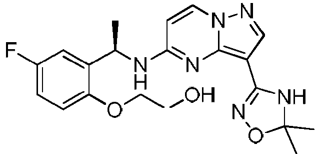 | [M + H]⁺ |
| | | 443.2 |
| **78** | 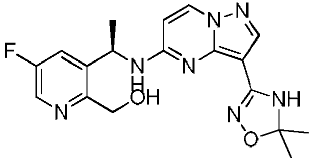 | [M + H]⁺ |
| | | 386.2 |
| **79** | 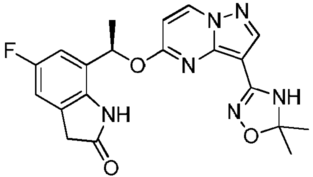 | [M + H]⁺ |
| | | 411.1 |
| **80** | 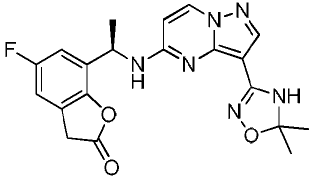 | [M + H]⁺ |
| | | 411.1 |
| **81** | 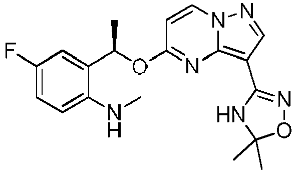 | [M + H]⁺ |
| | | 385.2 |
| **82** | 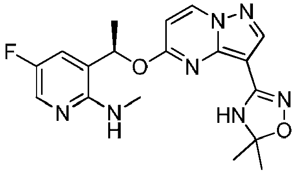 | [M + H]⁺ |
| | | 386.2 |
| **83** | 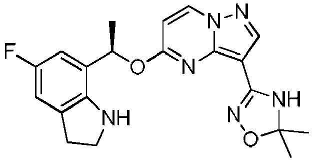 | [M + H]⁺ |
| | | 397.2 |

### Example 84 Example 25 and its enantiomers

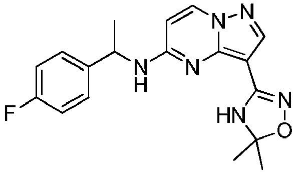

### Synthetic route of chiral amine intermediates:

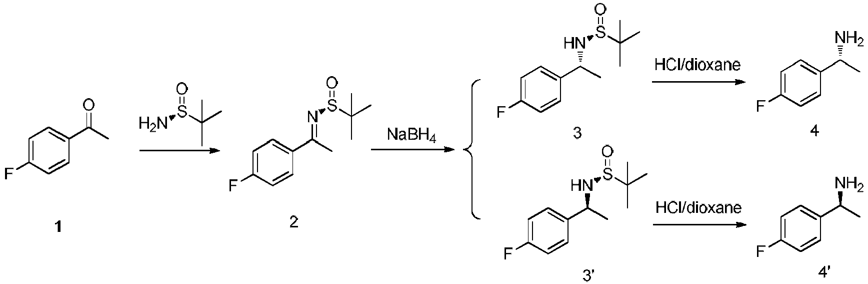

The synthesis of example 84 referred to the synthesis of chiral amine intermediate compound 4 in Example 12. p-fluoroacetophenone and (R)-tert-butyl sulfinamide were used as raw materials to obtain imine which was reduced with sodium borohydride and then a pair of diastereomer compound 3 and compound 3' were obtained. The two compounds were separated by column chromatography, and then the tert-butyl sulfinyl group was removed to obtain two chiral amine intermediates with R and S configuration. The two chiral amine intermediates were reacted separately to obtain a compound Example 84 i.e. R (i.e., Example 25) and S-configuration compounds. The two chiral amine intermediates were mixed to obtain Example 84, which was a racemate.

### Example 85 Resolution of Example 48

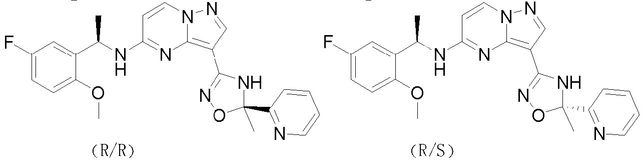

The two compounds were separated by preparation liquid phase under the following separation conditions:
Instruments: Waters2525 & Waters2767;
Column: Innoval ODS-2 (30 x 100 mm, 5 microns);
Flow rate: 15.0 ml/min, detection wavelength: 254 nm;
Solvent: Methanol, sample concentration was 12 mg/ml;
Injection volume: 0.5 ml, delay time: 24 seconds;
Threshold: 20,000, timetable: 2.00,
Mobile phase: A: water (containing 0.1% trifluoroacetic acid), B; methanol.
Gradient program:

| t (min) | Phase A | Phase B |
|---|---|---|
| 0 | 41 | 59 |
| 22 | 41 | 59 |
| 25 | 10 | 90 |
| 27 | 10 | 90 |
| 28 | 41 | 59 |
| 30 | 41 | 59 |

### Test Example 1: Inhibitory activity of the compounds of the invention against ROS1, NTRK and ALK and their drug-resistant kinases

Inhibition of protein kinase activity by compounds was carried out on the Radio-tagged HotSpot kinase experimental platform of Reaction Biology Corporation. Fresh reaction solution (20 mM HEPESpH 7.5, 10 mM MgCl₂, 1 mM EGTA, 0.02% Brij35, 0.02 mg/ml BSA, 0.1 mM Na₃VO₄, 2 mM DTT, 1% DMSO) containing corresponding substrates was prepared, cofactor and kinase to be tested were added into the above solution and mixed gently. Echo550 pipetting system was used to add the test compound DMSO solution to each well (the blank control group was added with the corresponding volume of DMSO), then 33P-ATP (with a final specific activity of 0.01 µCi/µL) was added to start the reaction. The reaction solution was incubated at room temperature for 120 minutes. Transferred the incubated reaction solution to P81 ion exchange chromatographic paper (Whatman # 3698-915), eluted with 0.75% phosphoric acid solution, and the amount of radioactive phosphorylated substrate remaining on the chromatographic paper was detected.

Table 2 showed the inhibitory activity IC₅₀ Value of the compounds of the present invention against ROS1, NTRK and ALK and the drug-resistant kinases thereof, wherein a < 0.5 nM, 0.5 nM≤B≤5.0 nM, 5.0 nM < C < 50 nM, 50 nM≤D≤500 nM, E > 500 nM;

**Table 2**

| Example | ROS1 (IC₅₀/ nM) | ROS1 (G2032 R) (IC₅₀/ nM) | TRK A (IC₅₀/ nM) | TRKA (G677 C) (IC₅₀/ nM) | TRKA (G595 R) (IC₅₀/ nM) | TRK B (IC₅₀/ nM) | TRK C (IC₅₀/ nM) | ALK (IC₅₀/ nM) | ALK (G1202 R) (IC₅₀/ nM) |
|---|---|---|---|---|---|---|---|---|---|
| 4 | A | A | A | C | C | A | A | B | C |
| 5 | A | A | B | C | C | A | A | B | C |
| 9 | A | | A | D | | B | A | D | |
| 10 | A | A | A | B | B | A | A | C | B |
| 14 | B | B | B | C | C | B | B | | C |
| 23 | B | B | B | C | C | B | B | | C |
| 24 | | | E | | | | | | |
| 25 | | | D | | | | | | |
| 25 race mate | | | D | | | | | | |
| 26 | B | | B | | | B | B | C | |
| 27 | B | | B | | | B | B | C | |
| 28 | A | A | A | C | B | A | A | C | B |
| 41 | | | D | | | | | | |
| 42 | B | | B | | | B | B | C | |
| 43 | | | D | | | | | | |
| 44 | | | | B | | | | | |
| 45 | | | | C | | | | | |
| 46 | | | | C | | | | | |
| 47 | | | | D | | | | | |
| 48 | | | | D | | | | | |
| 50 | A | A | B | B | B | A | A | C | B |
| 51 | A | A | B | C | C | A | A | C | B |
| 52 | A | B | B | D | C | B | A | D | C |
| 55 | A | A | B | | B | A | A | | |
| 54 | A | A | B | | B | B | A | | |
| 57 | A | A | B | | B | B | A | | |
| 65 | A | A | B | | B | B | A | | |
| 66 | A | A | B | | B | B | A | | |
| 67 | B | C | C | | C | B | B | | |
| 68 | A | A | A | | A | A | A | | |
| 69 | A | A | A | | A | A | A | | |
| 71 | B | B | B | | B | B | B | | |
| Staurosporine | 0.246 | 13.0 | 2.11 | | 5.3 | 0.473 | 0.106 | | |

The kinase activity test shows that the series compounds of the present invention have good inhibitory activity on ROS1, NTRK and ALK and the drug-resistant mutations thereof, especially the inhibitory activity on drug-resistant mutations is better.

The compounds of the present invention have better inhibitory activity against one or more of ROS1, NTRK and ALK and the drug-resistant mutations thereof than that of currently clinically available drugs.

Most of the compounds of the invention have better or equivalent activity against one or more of ROS1, NTRK and ALK and the drug-resistant mutations thereof than current clinically available drugs.

The compounds of the invention have great potential for use in the treatment of diseases mediated by ROS1, NTRK, ALK and the like.

### Test Example 2: Inhibition of cell proliferation by compounds

The experiment of inhibiting cell proliferation by compounds was carried out in Hefei Zhongkeprecedo Biomedical Technology Co., Ltd.. The Ba/F3 engineered cell line stably transfected with different kinase genes was recovered with RPMI 1640 medium (Biological Industries, Israel) + 10% fetal bovine serum (Biological Industries, Israel) + 1% double antibody (Penicillin Streptomycin solution, Coring, USA) and cultivated two generations. The logarithmic growth phase cell suspension was taken, and 2000 cells/well were inoculated on 96-well white cell culture plate (Corning 3917, NY, USA) with a volume of 95 µ L per well. 5 µ L of 20 × DMSO solution of the compound to be tested was added into the culture plate containing 95 µ L of cell suspension. The blank control group was added with corresponding volume of DMSO, mixed well, and incubated in a 5% CO₂ incubator at 37°C for 72 hours. CellTiter-Glo was used to detect cell viability.

Table 3 showed the inhibitory activity IC₅₀ Value of the compounds of the present invention against ROS1, NTRK and ALK or their drug-resistant mutant Ba/F3 engineered cell lines.

**Table 3**

| Exam ple | Ba/F3-CD74-ROS1 (IC₅₀/nM) | Ba/F3-CD74-ROS1-G2032R (IC₅₀/nM) | Ba/F3-LMNA-NTRK1 (IC₅₀/nM) | Ba/F3-LMNA-NTRK1-G595R (IC₅₀/nM) | Ba/F3-TEL-ALK-G1202R (IC₅₀/nM) |
|---|---|---|---|---|---|
| 1 | | | | 457.9 | |
| 2 | | | | 112.6 | |
| 3 | 2.8 | 10.3 | 2.1 | 3.3 | |
| 6 | 6.0 | 43.7 | 2.0 | 2.8 | |
| 9 | 26.2 | 154.8 | 100.5 | 890.1 | |
| 10 | 2.8 | 7.9 | 1.3 | 2.2 | 87.5 |
| 13 | 5.2 | 45.9 | 5.1 | 12.5 | |
| 15 | 4.9 | 34.8 | 3.8 | 8.6 | |
| 17 | 2.8 | 23.5 | 2.3 | 5.1 | |
| 19 | | 139.8 | | | |
| 20 | | 43.6 | | | |
| 29 | | | | 386.6 | |
| 30 | 2.2 | 16.3 | 5.8 | 7.0 | |
| 31 | | 54.4 | | 3.5 | |
| 32 | 52.1 | | 45.8 | 161.2 | |
| 33 | 15.7 | 120.5 | 25.9 | 386.5 | |
| 44 | 6.9 | 59.2 | 14.2 | 13.9 | |
| 53 | 8.4 | 81.1 | 18.2 | 30.1 | |
| 55 | 6.6 | 47.9 | 26.7 | 25.4 | |
| 57 | 4.5 | 38.9 | 26.2 | 45.3 | |
| 71 | 32.2 | 185 | 76.9 | 109.7 | |

The cell activity test shows that the series compounds of the present invention have good inhibitory activity against ROS1, NTRK and ALK and their drug-resistant mutant Ba/F3 engineered cell lines, especially the inhibitory activity against drug-resistant mutations is better. The compounds of the invention have good inhibitory activity against ROS1, NTRK and ALK and their drug-resistant mutant Ba/F3 engineering cell lines, and most of the compounds of the invention have excellent activity against ROS1, NTRK and ALK and their drug-resistant mutant Ba/F3 engineering cell lines, and they have great potential to be applied to the treatment of diseases mediated by ROS1, NTRK and ALK and the like.

It should be understood that after reading the above teaching, many variations and modifications may be made by the skilled in the art, and these also fall within the scope as defined by the appended claims.

## Claims

1. A compound represented by formula I, or a tautomer thereof, or a pharmaceutically acceptable salt thereof, or a deuterated compound thereof: 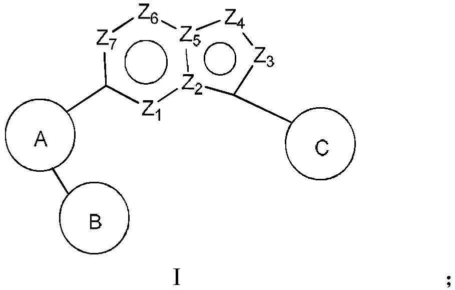 in formula I:
A is 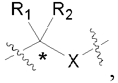 wherein * denotes R configuration, and X is independently selected from the group consisting of NR₆, O, CR₁R₂, S, S(O) and S(O)₂;
B is selected from the group consisting of phenyl, 5-6 membered heteroaryl; wherein, H on any carbon atom of the phenyl and 5-6 membered heteroaryl is optionally substituted by the following substituents: halogen, hydroxy, amino, cyano, C₁-C₆ alkyl, C₃-C₆ cycloalkyl, C₁-C₆ alkylamino, C₁-C₆ alkoxy, C₃-C₆ cycloalkoxy, monosubstituted or polysubstituted C₁-C₆ alkyl, monosubstituted or polysubstituted C₁-C₆ alkoxy, monosubstituted or polysubstituted C₃-C₆ cycloalkyl and monosubstituted or polysubstituted C₃-C₆ cycloalkoxy; the substituents of the monosubstituted or polysubstituted C₁-C₆ alkyl, the monosubstituted or polysubstituted C₁-C₆ alkoxy, the monosubstituted or polysubstituted C₃-C₆ cycloalkyl and the monosubstituted or polysubstituted C₃-C₆ cycloalkoxy are independently selected from the the group consisting of deuterium, halogen, amino, cyano, hydroxyl, C₁-C₆alkyl, C₁-C₆haloalkyl, C₃-C₆ cycloalkyl, C₃-C₆ halocycloalkyl, C₁-C₆ alkoxy and C₁-C₆ haloalkoxy;
or B is selected from the group consisting of 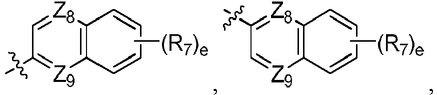 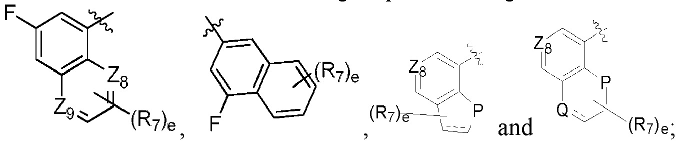
wherein, - - - is a single bond or a double bond;
Z₈ and Z₉ are each independently selected from CR₁₁ or N;
P is independently selected from O, NH or S;
when - - - is a double bond, Q is independently selected from CR₁₁ or N; when - - - is a single bond, Q is independently selected from O, S, CR₁₁R₁₂ or NH;
R₇ is each independently selected from the group consisting of halogen, amino, cyano, hydroxy, C₁-C₆ alkyl, C₃-C₆ cycloalkyl, C₁-C₆ alkoxy, C₁-C₆ alkylamino , monosubstituted or polysubstituted C₁-C₆ alkyl, monosubstituted or polysubstituted C₁-C₆ alkoxy and monosubstituted or polysubstituted C₃-C₆ cycloalkyl; the substituents of the monosubstituted or polysubstituted C₁-C₆ alkyl, monosubstituted or polysubstituted C₁-C₆ alkoxy and monosubstituted or polysubstituted C₃-C₆ cycloalkyl are independently selected from the group consisting of deuterium, halogen, amino, cyano, hydroxy, C₁-C₆ alkyl, C₁-C₆ haloalkyl, C₁-C₆alkoxy and C₁-C₆haloalkoxy;
R₁₁ and R₁₂ are each independently selected from the group consisting of H, hydroxy, halogen, amino, cyano, C₁-C₆ alkyl, C₁-C₆ haloalkyl, C₁-C₆ alkoxy and C₁-C₆ haloalkoxy;
e is 0, 1, 2, 3 or 4;
C is 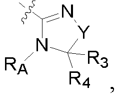 where Y is independently selected from the group consisting of O, NR_{A} and CR₁R₂;
wherein R₁ and R₂ are each independently selected from the group consisting of hydrogen, halogen, amino, cyano, hydroxy, C₁-C₆ alkyl, C₁-C₆ alkoxy, C₃-C₆ cycloalkyl, monosubstituted or polysubstituted C₁-C₆ alkyl, monosubstituted or polysubstituted C₁-C₆ alkoxy and monosubstituted or polysubstituted C₃-C₆ cycloalkyl; the substituents of the monosubstituted or polysubstituted C₁-C₆ alkyl, the monosubstituted or polysubstituted C₁-C₆ alkoxy and the monosubstituted or polysubstituted C₃-C₆ cycloalkyl are independently selected from the group consisting of deuterium, halogen, amino, cyano and hydroxyl;
R₃ and R₄ are each independently selected from the group consisting of hydrogen, amino, hydroxy, C₁-C₆ alkyl, C₃-C₆ cycloalkyl, phenyl, 5-6 membered heteroaryl, monosubstituted or polysubstituted C₁-C₆ alkyl, monosubstituted or polysubstituted C₁-C₆ alkoxy, monosubstituted or polysubstituted C₃-C₆ cycloalkyl, monosubstituted or polysubstituted phenyl and monosubstituted or polysubstituted 5-6 membered heteroaryl; the substituents of the monosubstituted or polysubstituted C₁-C₆ alkyl, the monosubstituted or polysubstituted C₁-C₆ alkoxy, the monosubstituted or polysubstituted C₃-C₆cycloalkyl, the monosubstituted or polysubstituted phenyl and the monosubstituted or polysubstituted 5-6 membered heteroaryl are independently selected from the group consisting of halogen, amino, cyano, hydroxy, C₁-C₆ alkyl, C₁-C₆ haloalkyl, C₁-C₆ alkoxy and C₁-C₆ haloalkoxy;
or R₃ and R₄ together with the C atom attached to them form substituted or unsubstituted 3-7 membered cycloalkane, substituted or unsubstituted 3-7 membered aza-cycloalkane, substituted or unsubstituted 3-7 membered oxa-cycloalkane or substituted or unsubstituted 3-7 membered thio-cycloalkane or oxo(=O); wherein the substituted means being substituted by one or more groups selected from the group consisting of C₁-C₆ alkyl, -C(O)C₁-C₆ alkyl, -C(O)OC₁-C₆ alkyl, -S(O)₂C₁-C₆ alkyl, and -S(O)C₁-C₆ alkyl; 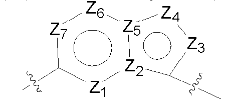 moiety is 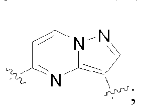
R₆ and R_{A} are each independently selected from the group consisting of hydrogen, C₁-C₆ alkyl, C₃-C₆ cycloalkyl, monosubstituted or polysubstituted C₁-C₆ alkyl and monosubstituted or polysubstituted C₃-C₆ cycloalkyl; the substituents of the monosubstituted or polysubstituted C₁-C₆ alkyl and the monosubstituted or polysubstituted C₃-C₆ cycloalkyl are independently selected from the group consisting of halogen, amino, cyano and hydroxy; and
each heteroaryl containing 1, 2 or 3 ring heteroatoms selected from N, O or S.

2. The compound of claim 1, or the tautomer thereof, or the pharmaceutically acceptable salt thereof, or the deuterated compound thereof: 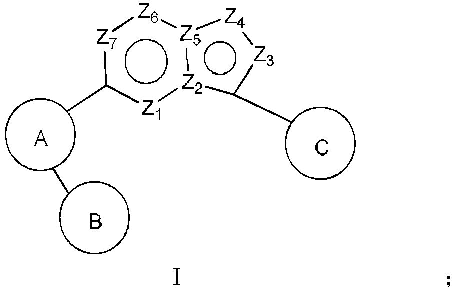 in formula I:
A is 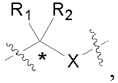 wherein, * denotes R configuration, and X is NR₆ and O;
B is selected from phenyl and 5-6 membered heteroaryl, wherein the H on any carbon atom of B is optionally substituted by the following substituents: halogen, hydroxyl, amino, cyano, C₁-C₆ alkyl, halo C₁-C₆ alkyl, C₁-C₆ alkylamino and C₁-C₆ alkoxy;
or B is 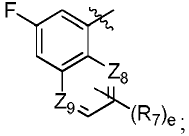
wherein, Z₈ and Z₉ are each independently selected from CR₁₁ or N;
R₇ is each independently selected from the group consisting of halogen, amino, cyano, hydroxy, C₁-C₆ alkyl, C₁-C₆ alkoxy, monosubstituted or polysubstituted C₁-C₆ alkyl and monosubstituted or polysubstituted C₁-C₆ alkoxy; the substituents of the monosubstituted or polysubstituted C₁-C₆ alkyl and monosubstituted or polysubstituted C₁-C₆ alkoxy are independently selected from the group consisting of deuterium, halogen, amino, cyano, hydroxy, C₁-C₆ alkyl, halo C₁-C₆ alkyl, C₁-C₆ alkoxy and halo C₁-C₆ alkoxy;
R₁₁ is each independently selected from the group consisting of H, hydroxy, halogen, amino, cyano, C₁-C₆ alkyl, halo C₁-C₆ alkyl, C₁-C₆ alkoxy and halo C₁-C₆ alkoxy;
e is 0, 1, 2, 3 or 4;
C is optionally selected from 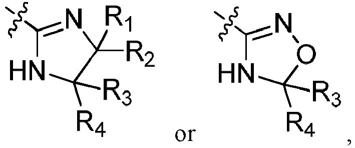 wherein the R₁, R₂, R₃, and R₄ are each independently selected from the group consisting of hydrogen, halogen, amino, cyano, hydroxyl, C₁-C₆ alkyl, C₁-C₆ alkoxy and monosubstituted or polysubstituted C₁-C₆ alkyl; the substituents of the monosubstituted or polysubstituted C₁-C₆ alkyl are independently selected from the group consisting of halogen, amino, cyano and hydroxy, or R₃ and R₄ together with the C atom attached to them form substituted or unsubstituted 3-7 membered cycloalkane, substituted or unsubstituted 3-7 membered aza-cycloalkane, substituted or unsubstituted 3-7 membered oxa-cycloalkane, or substituted or unsubstituted 3-7 membered thio-cycloalkane, wherein the substituted means being substituted by one or more groups selected from the group consisting of C₁-C₆ alkyl, -C(O)C₁-C₆ alkyl, -C(O)OC₁-C₆ alkyl, -S(O)₂C₁-C₆ alkyl, and -S(O)C₁-C₆ alkyl; 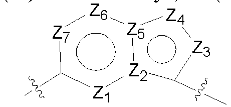 moiety is 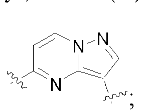
R₆ is each independently selected from the group consisting of hydrogen, C₁-C₆ alkyl, and monosubstituted or polysubstituted C₁-C₆ alkyl; the substituents of the monosubstituted or polysubstituted C₁-C₆ alkyl are independently selected from the group consisting of halogen, amino, cyano and hydroxy.

3. The compound of claim 1, or the tautomer thereof, or the pharmaceutically acceptable salt thereof, or the deuterated compound thereof: 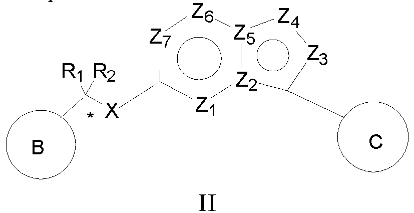 in formula II,
* denotes R configuration;
X is selected from NR₆ or O;
R₁ and R₂ are different and independently selected from the group consisting of hydrogen, halogen, amino, cyano, hydroxyl, C₁-C₆ alkyl and halo C₁-C₆ alkyl;
R₆ is independently selected from the group consisting of hydrogen, C₁-C₆ alkyl, and monosubstituted or polysubstituted C₁-C₆ alkyl, and the substituents of monosubstituted or polysubstituted C₁-C₆ alkyl are independently selected from the group consisting of halogen, amino, cyano and hydroxy; 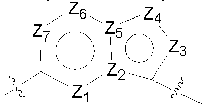 moiety is 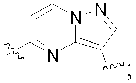
B and C are as defined in claim 1.

4. The compound of claim 1, or the tautomer thereof, or the pharmaceutically acceptable salt thereof, or the deuterated compound thereof, wherein B is independently selected from the group consisting of 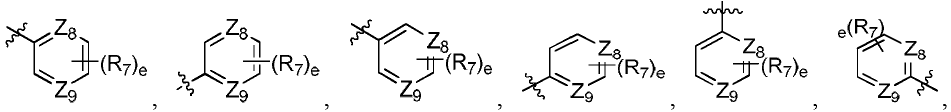 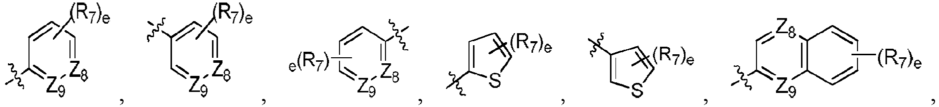 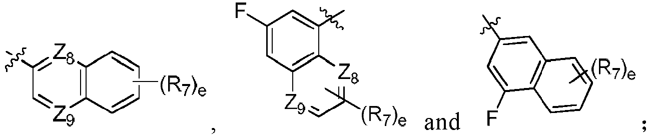
Z₈ and Z₉ are each independently selected from CR₁₁ or N;
each R₇ is independently selected from the group consisting of halogen, amino, cyano, hydroxy, C₁-C₆ alkyl, C₁-C₆ alkoxy, C₁-C₆ alkylamino, monosubstituted or polysubstituted C₁-C₆ alkyl, and monosubstituted or polysubstituted C₁-C₆ alkoxy; the substituents of the monosubstituted or polysubstituted C₁-C₆ alkyl, and the monosubstituted or polysubstituted C₁-C₆ alkoxy are independently selected from the group consisting of deuterium, halogen, amino, cyano, hydroxyl, C₁-C₆ alkyl, halo C₁-C₆ alkyl C₁-C₆ alkoxy and halo C₁-C₆ alkoxy;
R₁₁ is each independently selected from the group consisting of **H,** hydroxy, halogen, amino, cyano, C₁-C₆ alkyl, halo C₁-C₆alkyl, C₁-C₆ alkoxy, and halo C₁-C₆ alkoxy;
e is 0, **1,** 2, 3 or 4.

5. The compound of claim **1,** or the tautomer thereof, or the pharmaceutically acceptable salt thereof, or the deuterated compound thereof, wherein C is 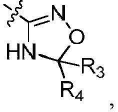 wherein, R₃ and R₄ are as defined in claim 1.

6. The compound of claim 1, or the tautomer thereof, or the pharmaceutically acceptable salt thereof, or the deuterated compound thereof, wherein the compound is selected from the following compounds:

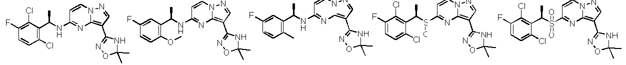

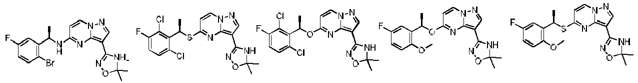

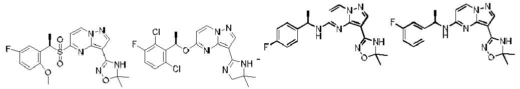

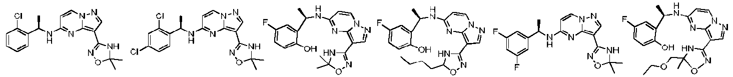

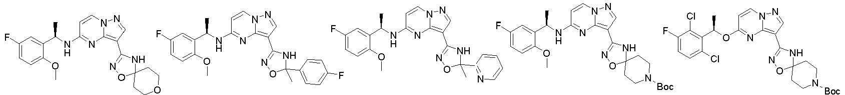

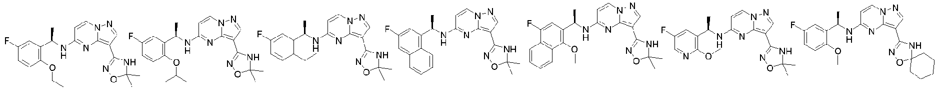

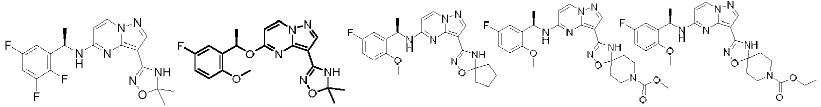

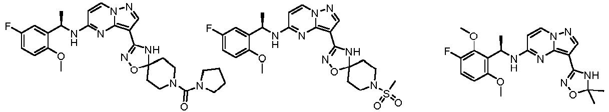

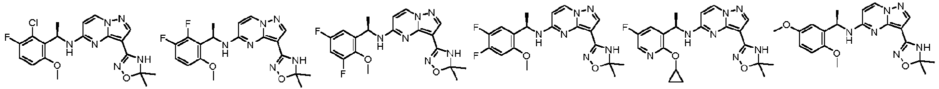

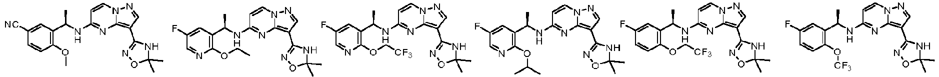

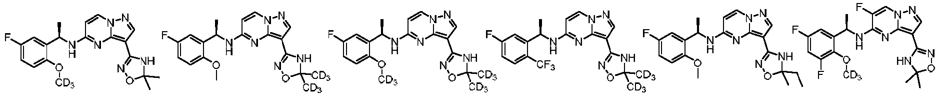

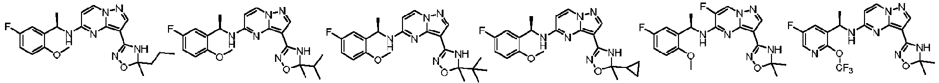

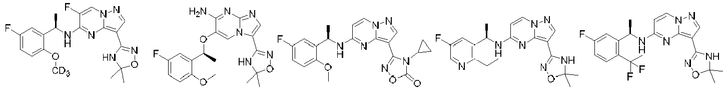

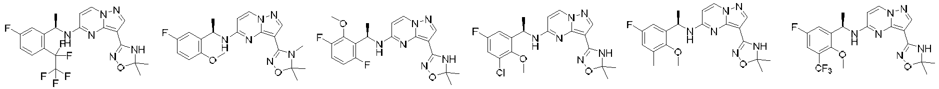

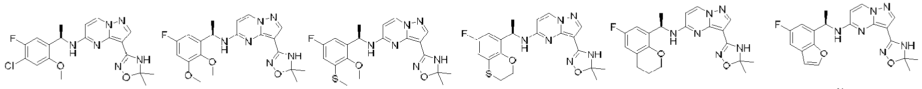

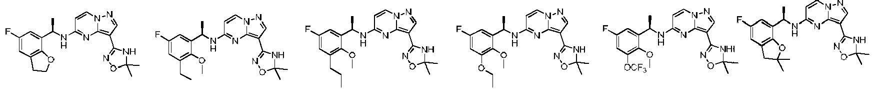

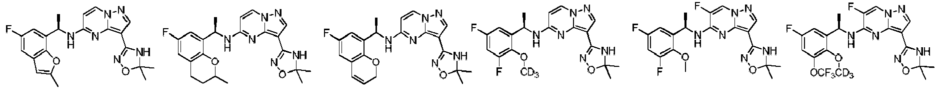

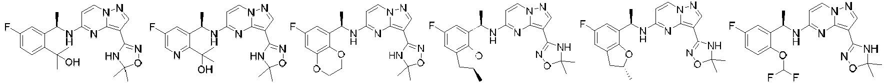

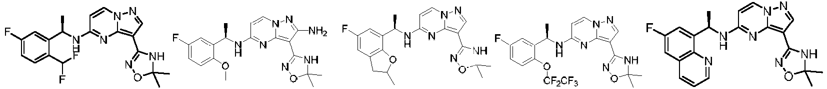

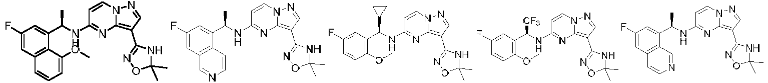

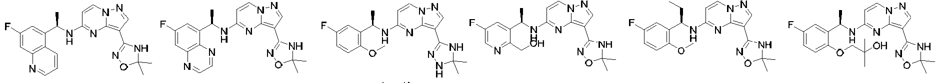

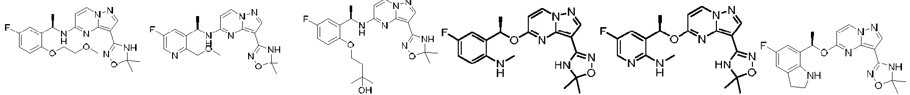

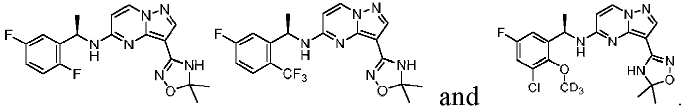

7. A pharmaceutically acceptable salt of the compound of formula I of claim 1, wherein the pharmaceutically acceptable salt is an inorganic acid salt or an organic acid salt, wherein the inorganic acid salt is selected from the group consisting of hydrochloride, hydrobromide, hydroiodate, sulfate, bisulfate, nitrate, phosphate and acid phosphate; the organic acid salt is selected from formate, acetate, trifluoroacetate, propionate, pyruvate, hydroxyacetate, oxalate, malonate, fumarate, maleate, lactate, malate, citrate, tartrate, methanesulfonate, ethane sulfonate, hydroxyethanesulfonate, benzenesulfonate, salicylate, picrate, glutamate, ascorbate, camphorate, and camphor sulfonate.

8. A pharmaceutical composition comprising a therapeutically effective amount of the compound of formula I of claim 1, or the tautomer thereof, or the pharmaceutically acceptable salt thereof, or the deuterated compound thereof, and one or more pharmaceutically acceptable carriers, diluents or excipients.

9. The compound of formula I of claim 1, or the tautomer thereof, or the pharmaceutically acceptable salt thereof, or the deuterated compound thereof or the pharmaceutical composition comprising the compound represented by formula I for use in preventing and/or treating the diseases mediated by ROS1, NTRK, and ALK, or drug-resistant kinases thereof.

10. The compound for use of claim 9, wherein the disease mediated by ROS1, NTRK, and ALK, or drug-resistant kinases thereof is selected from cancer, sarcoma or pain.

11. The compound for use of claim 10, wherein the cancer is any one of breast cancer, cervical cancer, colon cancer, lung cancer, stomach cancer, rectal cancer, pancreatic cancer, brain cancer, skin cancer, oral cancer, prostate cancer, bone cancer, kidney cancer, ovarian cancer, bladder cancer, liver cancer, fallopian tumor, peritoneal tumor, melanoma, glioma, glioblastoma, head and neck cancer, mastoid nephroma, leukemia, lymphoma, myeloma and thyroid tumor.

## Patentansprüche

1. Verbindung, die durch Formel I dargestellt wird, oder Tautomer davon oder pharmazeutisch annehmbares Salz davon oder deuterierte Verbindung davon: 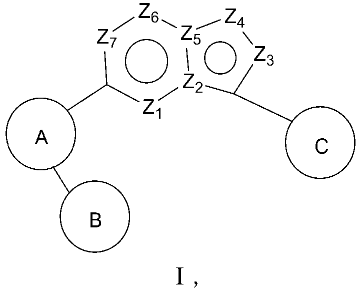 wobei in Formel I
A = 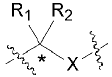 ist, wobei * eine R-Konfiguration bezeichnet, und X unabhängig aus der Gruppe ausgewählt ist, die aus NR₆, O, CR₁R₂, S, S(O) und S(O)₂ besteht;
B aus der Gruppe ausgewählt ist, die aus Phenyl und fünf- bis sechsgliedrigem Heteroaryl besteht; wobei H an einem beliebigen Kohlenstoffatom des Phenyls und des fünf- bis sechsgliedrigen Heteroaryls gegebenenfalls durch die folgenden Substituenten substituiert ist: Halogen, Hydroxy, Amino, Cyano, C₁-C₆-Alkyl, C₃-C₆-Cycloalkyl, C₁-C₆-Alkylamino, C₁-C₆-Alkoxy, C₃-C₆-Cycloalkoxy, einfach substituiertes oder mehrfach substituiertes C₁-C₆-Alkyl, einfach substituiertes oder mehrfach substituiertes C₁-C₆-Alkoxy, einfach substituiertes oder mehrfach substituiertes C₃-C₆-Cycloalkyl und einfach substituiertes oder mehrfach substituiertes C₃-C₆-Cycloalkoxy; wobei die Substituenten des einfach substituierten oder mehrfach substituierten C₁-C₆-Alkyl, des einfach substituierten oder mehrfach substituierten C₁-C₆-Alkoxy, des einfach substituierten oder mehrfach substituierten C₃-C₆-Cycloalkyl und des einfach substituierten oder mehrfach substituierten C₃-C₆-Cycloalkoxy unabhängig aus der Gruppe ausgewählt sind, die aus Deuterium, Halogen, Amino, Cyano, Hydroxy, C₁-C₆-Alkyl, C₁-C₆-Halogenalkyl, C₃-C₆-Cycloalkyl, C₃-C₆-Halogencycloalkyl, C₁-C₆-Alkoxy und C₁-C₆-Halogenalkoxy besteht;
oder B aus der Gruppe ausgewählt ist, die aus 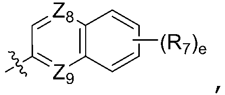 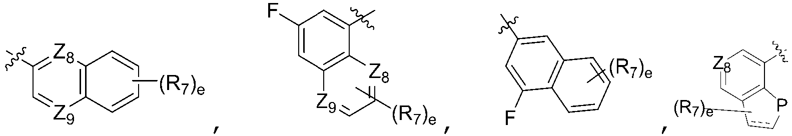 und 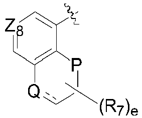 besteht;
wobei - - - eine Einfachbindung oder eine Doppelbindung ist;
Z₈ und Z₉ jeweils unabhängig aus CR₁₁ oder N ausgewählt sind;
P unabhängig aus O, NH oder S ausgewählt ist;
wenn - - - eine Doppelbindung ist, dann Q unabhängig aus CR₁₁ oder N ausgewählt ist; und wenn - - - eine Einfachbindung ist, dann Q unabhängig aus O, S, CR₁₁R₁₂ oder NH ausgewählt ist;
R₇ jeweils unabhängig aus der Gruppe ausgewählt ist, die aus Halogen, Amino, Cyano, Hydroxy, C₁-C₆-Alkyl, C₃-C₆-Cycloalkyl, C₁-C₆-Alkoxy, C₁-C₆-Alkylamino, einfach substituiertem oder mehrfach substituiertem C₁-C₆-Alkyl, einfach substituiertem oder mehrfach substituiertem C₁-C₆-Alkoxy und einfach substituiertem oder mehrfach substituiertem C₃-C₆-Cycloalkyl besteht; wobei die Substituenten des einfach substituierten oder mehrfach substituierten C₁-C₆-Alkyl, des einfach substituierten oder mehrfach substituierten C₁-C₆-Alkoxy und des einfach substituierten oder mehrfach substituierten C₃-C₆-Cycloalkyl unabhängig aus der Gruppe ausgewählt sind, die aus Deuterium, Halogen, Amino, Cyano, Hydroxy, C₁-C₆-Alkyl, C₁-C₆-Halogenalkyl, C₁-C₆-Alkoxy und C₁-C₆-Halogenalkoxy besteht;
R₁₁ und R₁₂ jeweils unabhängig aus der Gruppe ausgewählt sind, die aus H, Hydroxy, Halogen, Amino, Cyano, C₁-C₆-Alkyl, C₁-C₆-Halogenalkyl, C₁-C₆-Alkoxy und C₁-C₆-Halogenalkoxy besteht;
e = 0, 1, 2, 3 oder 4 ist;
C = 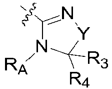 ist, wobei Y unabhängig aus der Gruppe ausgewählt ist, die aus O, NR_{A} and CR₁R₂ besteht;
wobei R₁ und R₂ jeweils unabhängig aus der Gruppe ausgewählt sind, die aus Wasserstoff, Halogen, Amino, Cyano, Hydroxy, C₁-C₆-Alkyl, C₁-C₆-Alkoxy, C₃-C₆-Cycloalkyl, einfach substituiertem oder mehrfach substituiertem C₁-C₆-Alkyl, einfach substituiertem oder mehrfach substituiertem C₁-C₆-Alkoxy und einfach substituiertem oder mehrfach substituiertem C₃-C₆-Cycloalkyl besteht; wobei die Substituenten des einfach substituierten oder mehrfach substituierten C₁-C₆-Alkyl, des einfach substituierten oder mehrfach substituierten C₁-C₆-Alkoxy und des einfach substituierten oder mehrfach substituierten C₃-C₆-Cycloalkyl unabhängig aus der Gruppe ausgewählt sind, die aus Deuterium, Halogen, Amino, Cyano und Hydroxy besteht;
R₃ und R₄ jeweils unabhängig aus der Gruppe ausgewählt sind, die aus Wasserstoff, Amino, Hydroxy, C₁-C₆-Alkyl, C₃-C₆-Cycloalkyl, Phenyl, fünfbis sechsgliedrigem Heteroaryl, einfach substituiertem oder mehrfach substituiertem C₁-C₆-Alkyl, einfach substituiertem oder mehrfach substituiertem C₁-C₆-Alkoxy, einfach substituiertem oder mehrfach substituiertem C₃-C₆-Cycloalkyl, einfach substituiertem oder mehrfach substituiertem Phenyl und einfach substituiertem oder mehrfach substituiertem fünf- bis sechsgliedrigen Heteroaryl besteht; wobei die Substituenten des einfach substituierten oder mehrfach substituierten C₁-C₆-Alkyl, des einfach substituierten oder mehrfach substituierten C₁-C₆-Alkoxy, des einfach substituierten oder mehrfach substituierten C₃-C₆-Cycloalkyl, des einfach substituierten oder mehrfach substituierten Phenyl und des einfach substituierten oder mehrfach substituierten fünf- bis sechsgliedrigen Heteroaryl unabhängig aus der Gruppe ausgewählt sind, die aus Halogen, Amino, Cyano, Hydroxy, C₁-C₆-Alkyl, C₁-C₆-Halogenalkyl, C₁-C₆-Alkoxy und C₁-C₆-Halogenalkoxy besteht;
oder R₃ und R₄ zusammen mit dem C-Atom, an das sie gebunden sind, substituiertes oder unsubstituiertes drei- bis siebengliedriges Cycloalkan, substituiertes oder unsubstituiertes drei- bis siebengliedriges Azacycloalkan, substituiertes oder unsubstituiertes drei- bis siebengliedriges Oxacycloalkan oder substituiertes oder unsubstituiertes drei- bis siebengliedriges Thiocycloalkan oder Oxo (=O) bilden; wobei "substituiert" bedeutet, mit einer oder mehreren Gruppen substituiert zu sein, die aus der Gruppe ausgewählt sind, die aus C₁-C₆-Alkyl, -C(O)C₁-C₆-Alkyl, -C(O)OC₁-C₆-Alkyl, -S(O)₂C₁-C₆-Alkyl und -S(O)C₁-C₆-Alkyl besteht;
es sich bei der Struktureinheit 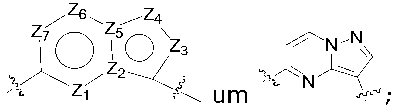 handelt;
R₆ und R_{A} jeweils unabhängig aus der Gruppe ausgewählt sind, die aus Wasserstoff, C₁-C₆-Alkyl, C₃-C₆-Cycloalkyl, einfach substituiertem oder mehrfach substituiertem C₁-C₆-Alkyl und einfach substituiertem oder mehrfach substituiertem C₃-C₆-Cycloalkyl besteht; wobei die Substituenten des einfach substituierten oder mehrfach substituierten C₁-C₆-Alkyl und des einfach substituierten oder mehrfach substituierten C₃-C₆-Cycloalkyl unabhängig aus der Gruppe ausgewählt sind, die aus Halogen, Amino, Cyano und Hydroxy besteht; und
jedes Heteroaryl 1, 2 oder 3 Ringheteroatome enthält, die aus N, O oder S ausgewählt sind.

2. Verbindung gemäß Anspruch 1 oder Tautomer davon oder pharmazeutisch annehmbares Salz davon oder deuterierte Verbindung davon: 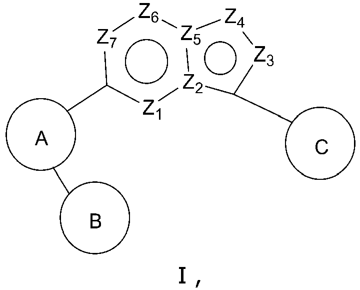 wobei in Formel I:
A = 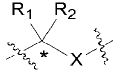 ist, wobei * eine R-Konfiguration bezeichnet, und X = NR₆ und O ist;
B aus Phenyl und fünf- bis sechsgliedrigem Heteroaryl ausgewählt ist, wobei H an einem beliebigen Kohlenstoffatom von B gegebenenfalls durch die folgenden Substituenten substituiert ist: Halogen, Hydroxy, Amino, Cyano, C₁-C₆-Alkyl, Halogen-C₁-C₆-alkyl, C₁-C₆-Alkylamino und C₁-C₆-Alkoxy;
oder B = 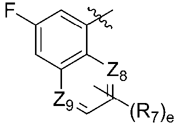 ist;
wobei Z₈ und Z₉ jeweils unabhängig aus CR₁₁ oder N ausgewählt sind;
R₇ jeweils unabhängig aus der Gruppe ausgewählt ist, die aus Halogen, Amino, Cyano, Hydroxy, C₁-C₆-Alkyl, C₁-C₆-Alkoxy, einfach substituiertem oder mehrfach substituiertem C₁-C₆-Alkyl und einfach substituiertem oder mehrfach substituiertem C₁-C₆-Alkoxy besteht; wobei die Substituenten des einfach substituierten oder mehrfach substituierten C₁-C₆-Alkyl und des einfach substituierten oder mehrfach substituierten C₁-C₆-Alkoxy unabhängig aus der Gruppe ausgewählt sind, die aus Deuterium, Halogen, Amino, Cyano, Hydroxy, C₁-C₆-Alkyl, Halogen-C₁-C₆-alkyl, C₁-C₆-Alkoxy und Halogen-C₁-C₆-alkoxy besteht;
R₁₁ jeweils unabhängig aus der Gruppe ausgewählt ist, die aus H, Hydroxy, Halogen, Amino, Cyano, C₁-C₆-Alkyl, Halogen-C₁-C₆-alkyl, C₁-C₆-Alkoxy und Halogen-C₁-C₆-alkoxy besteht;
e = 0, 1, 2, 3 oder 4 ist;
C gegebenenfalls aus 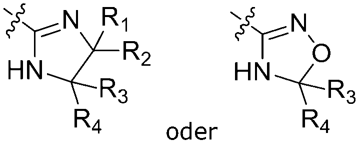 ausgewählt ist, wobei die R₁, R₂, R₃ und R₄ jeweils unabhängig aus der Gruppe ausgewählt sind, die aus Wasserstoff, Halogen, Amino, Cyano, Hydroxy, C₁-C₆-Alkyl, C₁-C₆-Alkoxy und einfach substituiertem oder mehrfach substituiertem C₁-C₆-Alkyl besteht; wobei die Substituenten des einfach substituierten oder mehrfach substituierten C₁-C₆-Alkyl unabhängig aus der Gruppe ausgewählt sind, die aus Halogen, Amino, Cyano und Hydroxy besteht, oder R₃ und R₄ zusammen mit dem C-Atom, an das sie gebunden sind, substituiertes oder unsubstituiertes drei- bis siebengliedriges Cycloalkan, substituiertes oder unsubstituiertes drei- bis siebengliedriges Azacycloalkan, substituiertes oder unsubstituiertes drei- bis siebengliedriges Oxacycloalkan oder substituiertes oder unsubstituiertes drei- bis siebengliedriges Thiocycloalkan bilden; wobei "substituiert" bedeutet, mit einer oder mehreren Gruppen substituiert zu sein, die aus der Gruppe ausgewählt sind, die aus C₁-C₆-Alkyl, -C(O)C₁-C₆-Alkyl, -C(O)OC₁-C₆-Alkyl, -S(O)₂C₁-C₆-Alkyl und -S(O)C₁-C₆-Alkyl besteht;
es sich bei der Struktureinheit 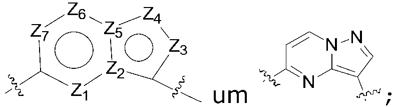 handelt;
R₆ jeweils unabhängig aus der Gruppe ausgewählt ist, die aus Wasserstoff, C₁-C₆-Alkyl und einfach substituiertem oder mehrfach substituiertem C₁-C₆-Alkyl besteht; wobei die Substituenten des einfach substituierten oder mehrfach substituierten C₁-C₆-Alkyl unabhängig aus der Gruppe ausgewählt sind, die aus Halogen, Amino, Cyano und Hydroxy besteht.

3. Verbindung gemäß Anspruch 1 oder Tautomer davon oder pharmazeutisch annehmbares Salz davon oder deuterierte Verbindung davon: 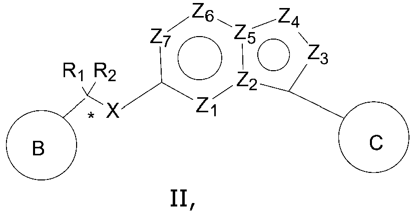
wobei in Formel (II)
* eine R-Konfiguration bezeichnet;
X aus NR₆ oder O ausgewählt ist;
R₁ und R₂ verschieden sind und unabhängig aus der Gruppe ausgewählt sind, die aus Wasserstoff, Halogen, Amino, Cyano, Hydroxy, C₁-C₆-Alkyl und Halogen-C₁-C₆-alkyl besteht;
R₆ unabhängig aus der Gruppe ausgewählt ist, die aus Wasserstoff, C₁-C₆-Alkyl und einfach substituiertem oder mehrfach substituiertem C₁-C₆-Alkyl besteht, und wobei die Substituenten des einfach substituierten oder mehrfach substituierten C₁-C₆-Alkyl unabhängig aus der Gruppe ausgewählt sind, die aus Halogen, Amino, Cyano und Hydroxy besteht;
es sich bei der Struktureinheit 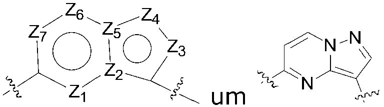 handelt;
B und C wie in Anspruch 1 definiert sind.

4. Verbindung gemäß Anspruch 1 oder Tautomer davon oder pharmazeutisch annehmbares Salz davon oder deuterierte Verbindung davon, wobei B unabhängig aus der Gruppe ausgewählt ist, die aus Folgenden besteht: 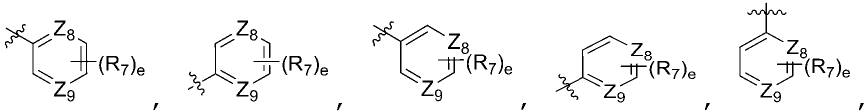 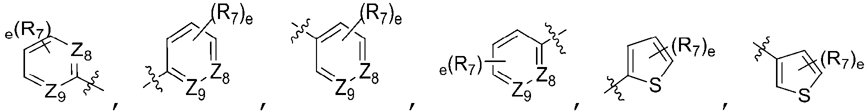 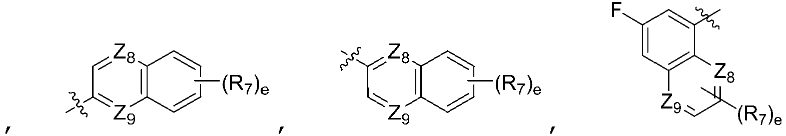 und 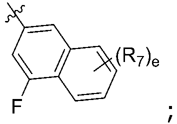
Z₈ und Z₉ jeweils unabhängig aus CR₁₁ oder N ausgewählt sind;
R₇ jeweils unabhängig aus der Gruppe ausgewählt ist, die aus Halogen, Amino, Cyano, Hydroxy, C₁-C₆-Alkyl, C₁-C₆-Alkoxy, C₁-C₆-Alkylamino, einfach substituiertem oder mehrfach substituiertem C₁-C₆-Alkyl und einfach substituiertem oder mehrfach substituiertem C₁-C₆-Alkoxy besteht; wobei die Substituenten des einfach substituierten oder mehrfach substituierten C₁-C₆-Alkyl und des einfach substituierten oder mehrfach substituierten C₁-C₆-Alkoxy unabhängig aus der Gruppe ausgewählt sind, die aus Deuterium, Halogen, Amino, Cyano, Hydroxy, C₁-C₆-Alkyl, Halogen-C₁-C₆-alkyl, C₁-C₆-Alkoxy und Halogen-C₁-C₆-alkoxy besteht;
R₁₁ jeweils unabhängig aus der Gruppe ausgewählt ist, die aus H, Hydroxy, Halogen, Amino, Cyano, C₁-C₆-Alkyl, Halogen-C₁-C₆-alkyl, C₁-C₆-Alkoxy und Halogen-C₁-C₆-alkoxy besteht;
e = 0, 1, 2, 3 oder 4 ist.

5. Verbindung gemäß Anspruch 1 oder Tautomer davon oder pharmazeutisch annehmbares Salz davon oder deuterierte Verbindung davon, wobei C = 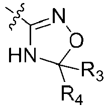 ist,
wobei R³ und R⁴ wie in Anspruch 1 definiert sind.

6. Verbindung gemäß Anspruch 1 oder Tautomer davon oder pharmazeutisch annehmbares Salz davon oder deuterierte Verbindung davon, wobei die Verbindung aus den folgenden Verbindungen ausgewählt ist:

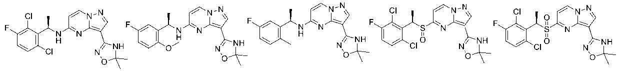

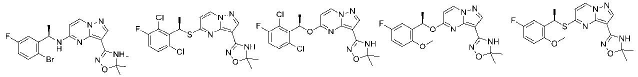

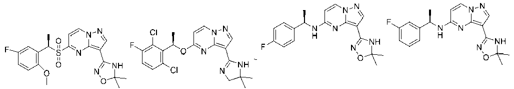

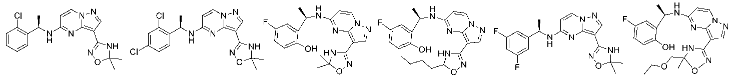

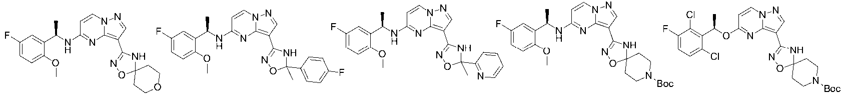

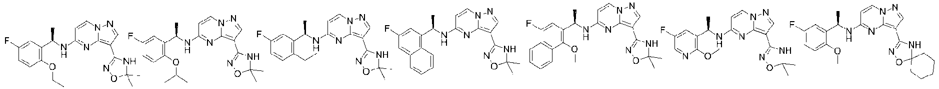

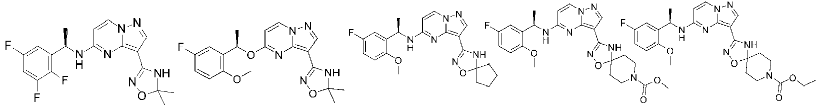

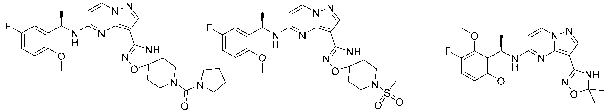

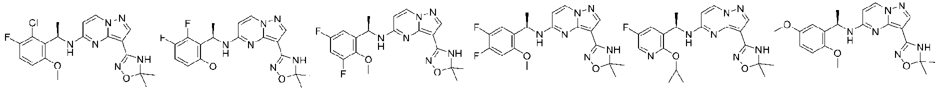

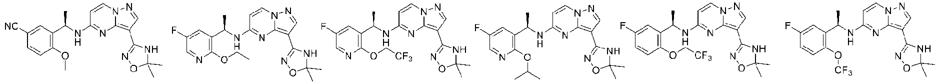

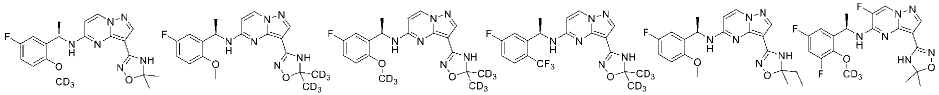

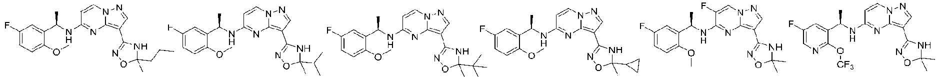

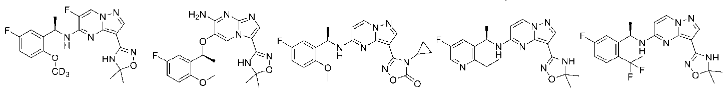

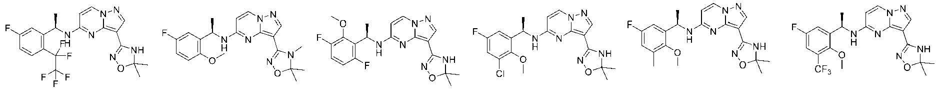

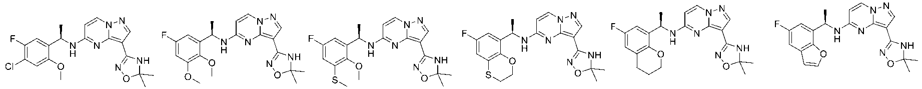

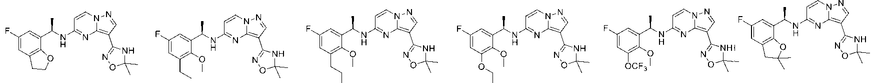

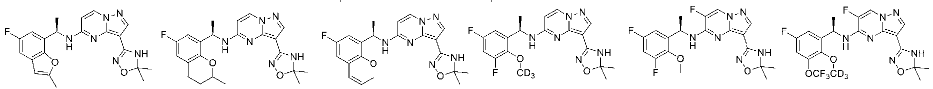

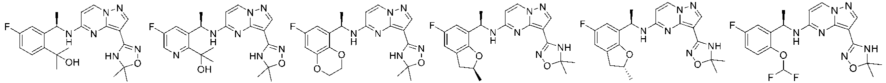

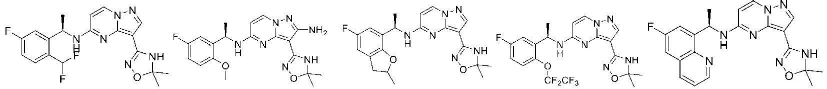

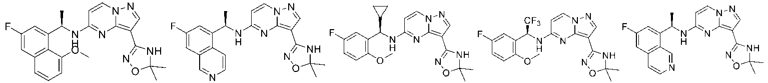

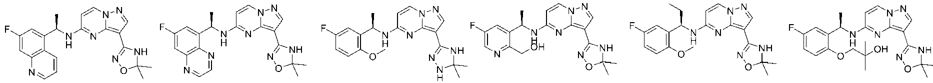

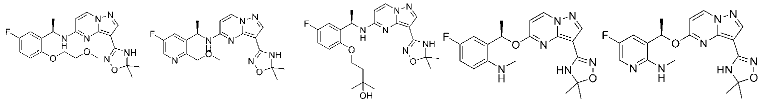

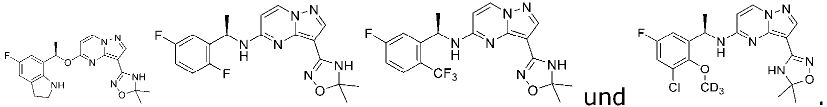

7. Pharmazeutisch annehmbares Salz der Verbindung der Formel I gemäß Anspruch 1, wobei das pharmazeutisch annehmbare Salz ein Salz einer anorganischen Säure oder ein Salz einer organischen Säure ist, wobei das Salz einer anorganischen Säure aus der Gruppe ausgewählt ist, die aus Hydrochlorid, Hydrobromid, Hydroiodat, Sulfat, Hydrogensulfat, Nitrat, Phosphat und saurem Phosphat besteht; das Salz der organischen Säure aus Formiat, Acetat, Trifluoracetat, Propionat, Pyruvat, Hydroxyacetat, Oxalat, Malonat, Fumarat, Maleat, Lactat, Malat, Citrat, Tartrat, Methansulfonat, Ethansulfonat, Hydroxyethansulfonat, Benzolsulfonat, Salicylat, Pikrat, Glutamat, Ascorbat, Camphorat und Camphersulfonat ausgewählt ist.

8. Pharmazeutische Zusammensetzung, umfassend eine therapeutisch wirksame Menge der Verbindung der Formel I gemäß Anspruch 1 oder das Tautomer davon oder das pharmazeutisch annehmbare Salz davon oder die deuterierte Verbindung davon und ein oder mehrere pharmazeutisch annehmbare Träger, Verdünnungsmittel oder Arzneimittelhilfsstoffe.

9. Verbindung der Formel I gemäß Anspruch 1 oder Tautomer davon oder pharmazeutisch annehmbares Salz davon oder deuterierte Verbindung davon oder pharmazeutische Zusammensetzung, die die durch Formel I dargestellte Verbindung umfasst, zur Verwendung bei der Prävention und/oder Behandlung der Krankheiten, die durch ROS1, NTRK und ALK oder arzneimittelresistente Kinasen davon vermittelt werden.

10. Verbindung zur Verwendung gemäß Anspruch 9, wobei die Krankheit, die durch ROS1, NTRK und ALK oder arzneimittelresistente Kinasen davon vermittelt wird, aus Krebs, Sarkom oder Schmerzen ausgewählt ist.

11. Verbindung zur Verwendung gemäß Anspruch 10, wobei der Krebs einer der folgenden ist: Brustkrebs, Gebärmutterhalskrebs, Darmkrebs, Lungenkrebs, Magenkrebs, Rektumkrebs, Bauchspeicheldrüsenkrebs, Hirnkrebs, Hautkrebs, Mundkrebs, Prostatakrebs, Knochenkrebs, Nierenkrebs, Eierstockkrebs, Blasenkrebs, Leberkrebs, Eileitertumor, Peritonealtumor, Melanom, Gliom, Glioblastom, Kopf- und Halskrebs, Mastoidnephrom, Leukämie, Lymphom, Myelom und Schilddrüsentumor.

## Revendications

1. Composé représenté par la formule I, ou un tautomère de celui-ci, ou un sel pharmaceutiquement acceptable de celui-ci, ou un composé deutéré de celui-ci : [INSÉRER STRUCTURE CHIMIQUE - Formule I avec cycles A, B, C et positions Z1-Z7] 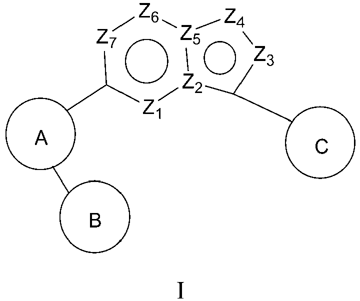 dans la formule I :
A est 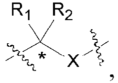 dans laquelle * désigne la configuration R, et X est indépendamment choisi dans le groupe constitué de NR
6, O, CR1R2, S, S(O) et S(O)2 ;
B est choisi dans le groupe constitué de phényle, hétéroaryle à 5-6 chaînons ; dans lequel H sur tout atome de carbone du phényle et de l'hétéroaryle à 5-6 chaînons est facultativement substitué par les substituants suivants : halogène, hydroxy, amino, cyano, alkyle en C1-C6, cycloalkyle en C3-C6, alkylamino en C1-C6, alcoxy en C1-C6, cycloalcoxy en C3-C6, alkyle en C1-C6 monosubstitué ou polysubstitué, alcoxy en C1-C6 monosubstitué ou polysubstitué, cycloalkyle en C3-C6 monosubstitué ou polysubstitué et cycloalcoxy en C3-C6 monosubstitué ou polysubstitué ; les substituants de l'alkyle en C1-C6 monosubstitué ou polysubstitué, de l'alcoxy en C1-C6 monosubstitué ou polysubstitué, du cycloalkyle en C3-C6 monosubstitué ou polysubstitué et du cycloalcoxy en C3-C6 monosubstitué ou polysubstitué sont indépendamment choisis dans le groupe constitué de deutérium, halogène, amino, cyano, hydroxyle, alkyle en C1-C6, haloalkyle en C1-C6, cycloalkyle en C3-C6, halocycloalkyle en C3-C6, alcoxy en C1-C6 et haloalcoxy en C1-C6 ;
ou B est choisi dans le groupe constitué de 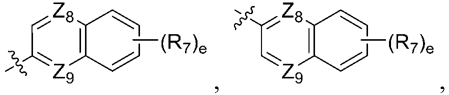 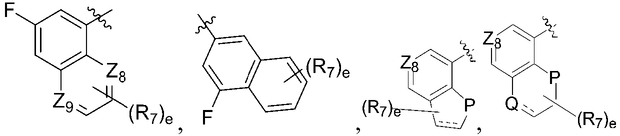
dans lesquels, - - - est une liaison simple ou une liaison double ;
Z8 et Z9 sont chacun indépendamment choisis parmi CR11 ou N ;
P est indépendamment choisi parmi O, NH ou S ;
lorsque - - - est une liaison double, Q est indépendamment choisi parmi CR11 ou N ; lorsque - - - est une liaison simple, Q est indépendamment choisi parmi O, S, CR11R12 ou NH ;
R7 est chacun indépendamment choisi dans le groupe constitué de halogène, amino, cyano, hydroxy, alkyle en C1-C6, alcoxy en C1-C6, alkylamino en C1-C6, alkyle en C1-C6 monosubstitué ou polysubstitué, et alcoxy en C1-C6 monosubstitué ou polysubstitué, et cycloalkyle en C3-C6 monosubstitué ou polysubstitué ; les substituants de l'alkyle en C1-C6 monosubstitué ou polysubstitué, de l'alcoxy en C1-C6 monosubstitué ou polysubstitué et du cycloalkyle en C3-C6 monosubstitué ou polysubstitué sont indépendamment choisis dans le groupe constitué de deutérium, halogène, amino, cyano, hydroxyle, alkyle en C1-C6, haloalkyle en C1-C6, alcoxy en C1-C6 et haloalcoxy en C1-C6 ;
R11 et R12 sont chacun indépendamment choisis dans le groupe constitué de H, hydroxy, halogène, amino, cyano, alkyle en C1-C6, haloalkyle en C1-C6, alcoxy en C1-C6 et haloalcoxy en C1-C6 ;
e est 0, 1, 2, 3 ou 4 ;
C est 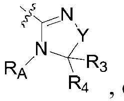 dans lequel Y est indépendamment choisi dans le groupe constitué de O, NRA et CR1R2 ;
dans lequel R1 et R2 sont chacun indépendamment choisis dans le groupe constitué de hydrogène, halogène, amino, cyano, hydroxy, alkyle en C1-C6, alcoxy en C1-C6, cycloalkyle en C3-C6, alkyle en C1-C6 monosubstitué ou polysubstitué, alcoxy en C1-C6 monosubstitué ou polysubstitué et cycloalkyle en C3-C6 monosubstitué ou polysubstitué ; les substituants de l'alkyle en C1-C6 monosubstitué ou polysubstitué, de l'alcoxy en C1-C6 monosubstitué ou polysubstitué et du cycloalkyle en C3-C6 monosubstitué ou polysubstitué sont indépendamment choisis dans le groupe constitué de deutérium, halogène, amino, cyano et hydroxyle ;
R3 et R4 sont chacun indépendamment choisis dans le groupe constitué de hydrogène, amino, hydroxy, alkyle en C1-C6, cycloalkyle en C3-C6, phényle, hétéroaryle à 5-6 chaînons, alkyle en C1-C6 monosubstitué ou polysubstitué, alcoxy en C1-C6 monosubstitué ou polysubstitué, cycloalkyle en C3-C6 monosubstitué ou polysubstitué, phényle monosubstitué ou polysubstitué et hétéroaryle à 5-6 chaînons monosubstitué ou polysubstitué ; les substituants de l'alkyle en C1-C6 monosubstitué ou polysubstitué, de l'alcoxy en C1-C6 monosubstitué ou polysubstitué, du cycloalkyle en C3-C6 monosubstitué ou polysubstitué, du phényle monosubstitué ou polysubstitué et de l'hétéroaryle à 5-6 chaînons monosubstitué ou polysubstitué sont indépendamment choisis dans le groupe constitué de halogène, amino, cyano, hydroxy, alkyle en C1-C6, haloalkyle en C1-C6, alcoxy en C1-C6 et haloalcoxy en C1-C6 ;
ou R3 et R4 forment ensemble avec l'atome de C auquel ils sont rattachés un cycloalcane substitué ou non substitué à 3-7 chaînons, un aza-cycloalcane substitué ou non substitué à 3-7 chaînons, un oxa-cycloalcane substitué ou non substitué à 3-7 chaînons ou un thio-cycloalcane substitué ou non substitué à 3-7 chaînons ou un oxo(=O) ; dans lesquels substitué signifie substitué par un ou plusieurs groupes choisis dans le groupe constitué de alkyle en C1-C6, -C(O)C1-C6 alkyle, -S(O)2C1-C6 alkyle, et -S(O)C1-C6 alkyle ; 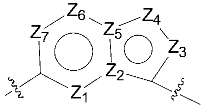 le fragment est 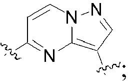
R6 et RA sont chacun indépendamment choisis dans le groupe constitué de hydrogène, alkyle en C1-C6, alkyle en C1-C6 monosubstitué ou polysubstitué et cycloalkyle en C3-C6 monosubstitué ou polysubstitué ; les substituants de l'alkyle en C1-C6 monosubstitué ou polysubstitué et du cycloalkyle en C3-C6 monosubstitué ou polysubstitué sont indépendamment choisis dans le groupe constitué de halogène, amino, cyano et hydroxy ; et
chaque hétéroaryle comprenant 1, 2 ou 3 hétéroatomes cycliques choisis parmi N, O ou S.

2. Composé selon la revendication 1, ou le tautomère de celui-ci, ou le sel pharmaceutiquement acceptable de celui-ci, ou le composé deutéré de celui-ci : 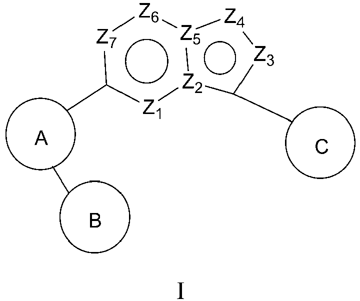 dans la formule I :
A est 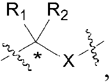 dans laquelle * désigne la configuration R, et X est NR6 et O ;
B est choisi parmi phényle et hétéroaryle à 5-6 chaînons, dans lequel H sur tout atome de carbone de B est facultativement substitué par les substituants suivants : halogène, hydroxyle, amino, cyano, alkyle en C1-C6, haloalkyle en C1-C6, alkylamino en C1-C6 et alcoxy en C1-C6 ;
ou B est 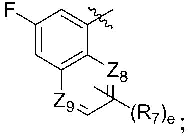
dans lequel Z8 et Z9 sont chacun indépendamment choisis parmi CR11 ou N ;
chaque R7 est indépendamment choisi dans le groupe constitué de halogène, amino, cyano, hydroxy, alkyle en C1-C6, alcoxy en C1-C6, alkylamino en C1-C6, alkyle en C1-C6 monosubstitué ou polysubstitué et alcoxy en C1-C6 monosubstitué ou polysubstitué ; les substituants de l'alkyle en C1-C6 monosubstitué ou polysubstitué et de l'alcoxy en C1-C6 monosubstitué ou polysubstitué sont indépendamment choisis dans le groupe constitué de deutérium, halogène, amino, cyano, hydroxy, alkyle en C1-C6, haloalkyle en C1-C6, alcoxy en C1-C6 et haloalcoxy en C1-C6 ;
R11 est chacun indépendamment choisi dans le groupe constitué de H, hydroxy, halogène, amino, cyano, alkyle en C1-C6, haloalkyle en C1-C6, alcoxy en C1-C6 et haloalcoxy en C1-C6 ;
e est 0, 1, 2, 3 ou 4 ;
C est facultativement choisi parmi 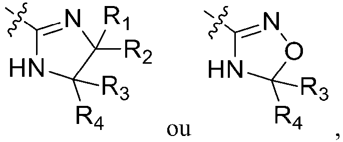 dans lequel R1, R2, R3, et R4 sont chacun indépendamment choisis dans le groupe constitué de hydrogène, halogène, amino, cyano, hydroxyle, alkyle en C1-C6, alcoxy en C1-C6 et alkyle en C1-C6 monosubstitué ou polysubstitué ; les substituants de l'alkyle en C1-C6 monosubstitué ou polysubstitué sont indépendamment choisis dans le groupe constitué de halogène, amino, cyano et hydroxy, ou
R3 et R4 forment ensemble avec l'atome de C auquel ils sont rattachés un cycloalcane substitué ou non substitué à 3-7 chaînons, un aza-cycloalcane substitué ou non substitué à 3-7 chaînons, un oxa-cycloalcane substitué ou non substitué à 3-7 chaînons, ou un thio-cycloalcane substitué ou non substitué à 3-7 chaînons, dans lesquels substitué signifie substitué par un ou plusieurs groupes choisis dans le groupe constitué de alkyle en C1-C6, -C(O)C1-C6 alkyle, -S(O)2C1-C6 alkyle, et -S(O)C1-C6 alkyle ; 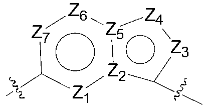 fragment est 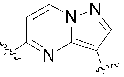
R6 est chacun indépendamment choisi dans le groupe constitué de hydrogène, alkyle en C1-C6, et alkyle en C1-C6 monosubstitué ou polysubstitué ; les substituants de l'alkyle en C1-C6 monosubstitué ou polysubstitué sont indépendamment choisis dans le groupe constitué de halogène, amino, cyano et hydroxy.

3. Composé selon la revendication 1, ou le tautomère de celui-ci, ou le sel pharmaceutiquement acceptable de celui-ci, ou le composé deutéré de celui-ci : 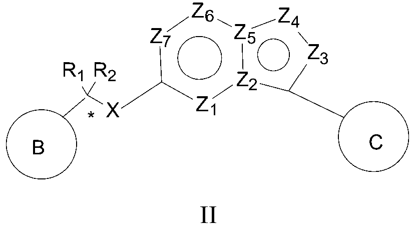 dans la formule II,
* désigne la configuration R ;
X est choisi parmi NR6 ou O ;
R1 et R2 sont différents et indépendamment choisis dans le groupe constitué de hydrogène, halogène, amino, cyano, hydroxyle, alkyle en C1-C6 et haloalkyle en C1-C6 ;
R6 est indépendamment choisi dans le groupe constitué de hydrogène, alkyle en C1-C6, et alkyle en C1-C6 monosubstitué ou polysubstitué, et les substituants de l'alkyle en C1-C6 monosubstitué ou polysubstitué sont indépendamment choisis dans le groupe constitué de halogène, amino, cyano et hydroxy ; 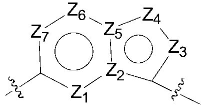 fragment est 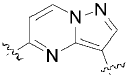
B et C sont tels que définis dans la revendication 1.

4. Composé selon la revendication 1, ou le tautomère de celui-ci, ou le sel pharmaceutiquement acceptable de celui-ci, ou le composé deutéré de celui-ci, dans lequel B est indépendamment choisi dans le groupe constitué de 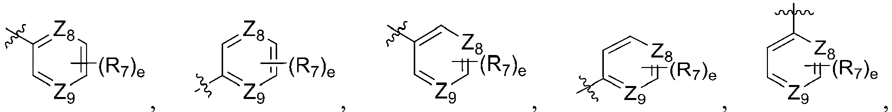 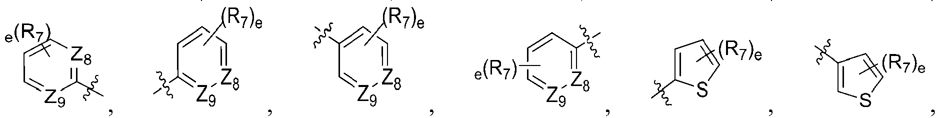 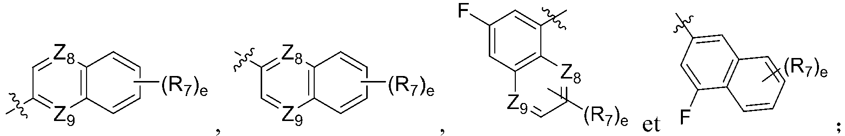
Z8 et Z9 sont chacun indépendamment choisis parmi CR11 ou N ;
chaque R7 est indépendamment choisi dans le groupe constitué de halogène, amino, cyano, hydroxy, alkyle en C1-C6, alcoxy en C1-C6, alkylamino en C1-C6, alkyle en C1-C6 monosubstitué ou polysubstitué et alcoxy en C1-C6 monosubstitué ou polysubstitué ; les substituants de l'alkyle en C1-C6 monosubstitué ou polysubstitué, et de l'alcoxy en C1-C6 monosubstitué ou polysubstitué sont indépendamment choisis dans le groupe constitué de deutérium, halogène, amino, hydroxyle, alkyle en C1-C6, haloalkyle en C1-C6, alcoxy en C1-C6 et haloalcoxy en C1-C6 ;
R11 est chacun indépendamment choisi dans le groupe constitué de H, hydroxy, halogène, amino, cyano, alkyle en C1-C6, haloalkyle en C1-C6, alcoxy en C1-C6, et haloalcoxy en C1-C6 ;
e est 0, 1, 2, 3 ou 4.

5. Composé selon la revendication 1, ou le tautomère de celui-ci, ou le sel pharmaceutiquement acceptable de celui-ci, ou le composé deutéré de celui-ci, dans lequel C est 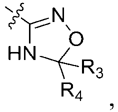 dans lequel R3 et R4 sont tels que définis dans la revendication 1.

6. Composé selon la revendication 1, ou le tautomère de celui-ci, ou le sel pharmaceutiquement acceptable de celui-ci, ou le composé deutéré de celui-ci, dans lequel le composé est choisi parmi les composés suivants :

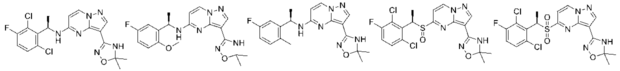

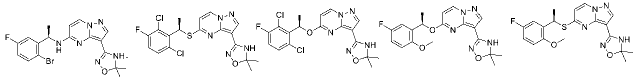

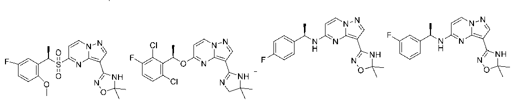

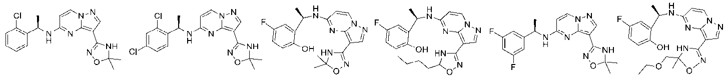

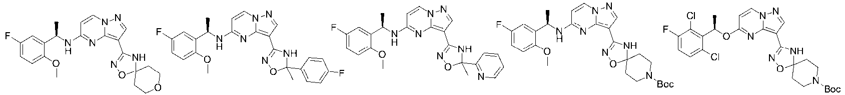

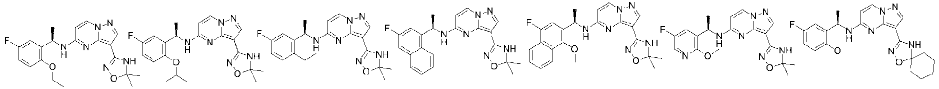

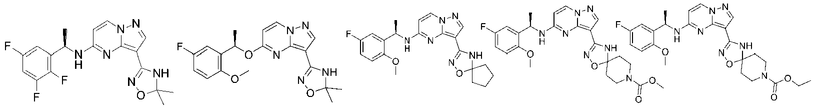

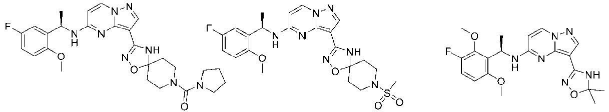

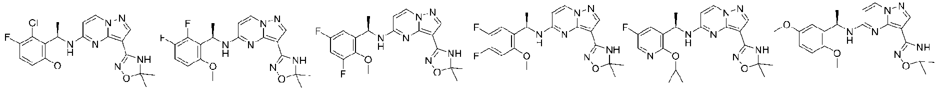

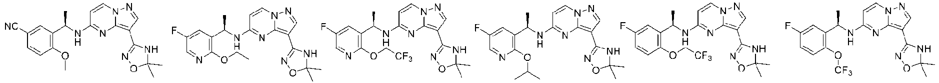

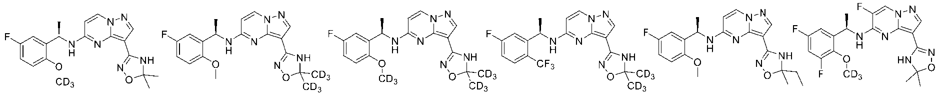

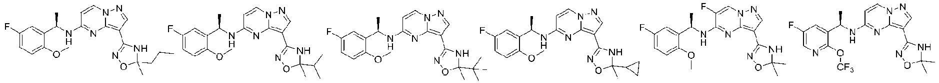

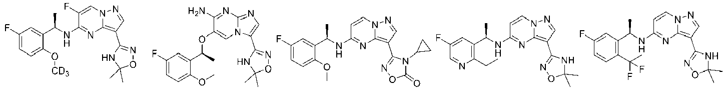

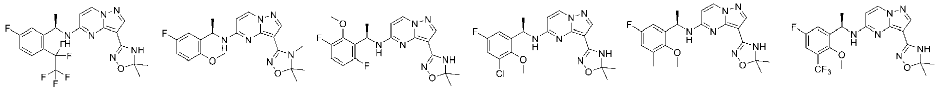

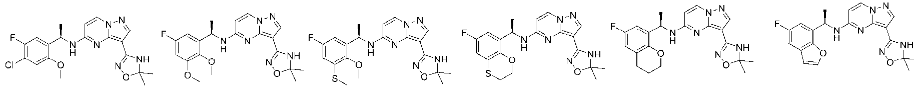

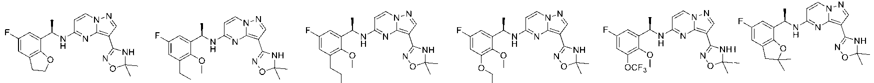

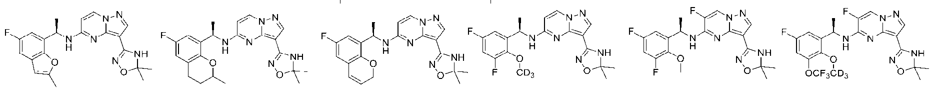

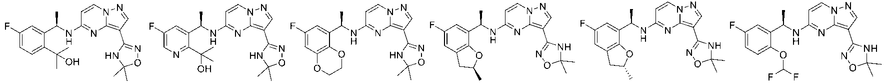

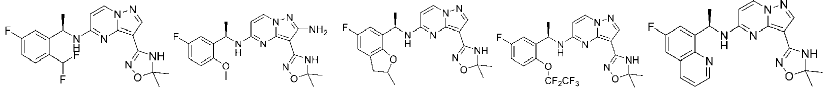

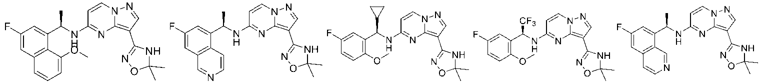

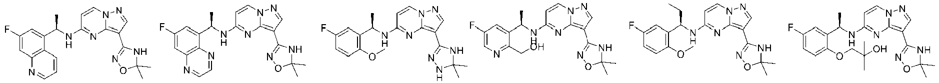

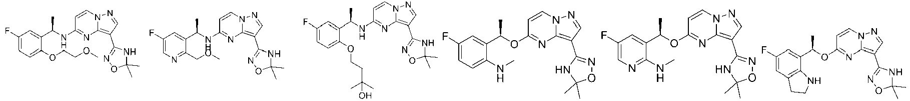

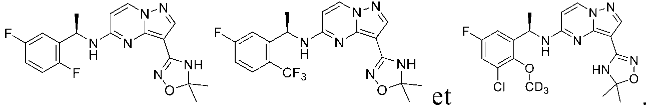

7. Sel pharmaceutiquement acceptable du composé de formule I selon la revendication 1, dans lequel le sel pharmaceutiquement acceptable est un sel d'acide inorganique ou un sel d'acide organique, dans lequel le sel d'acide inorganique est choisi dans le groupe constitué de chlorhydrate, bromhydrate, iodhydrate, sulfate, bisulfate, nitrate, phosphate et phosphate acide ; le sel d'acide organique est choisi parmi formiate, acétate, trifluoroacétate, propionate, pyruvate, hydroxyacétate, oxalate, malonate, fumarate, maléate, lactate, malate, citrate, tartrate, méthanesulfonate, éthanesulfonate, hydroxyéthanesulfonate, benzènesulfonate, salicylate, picrate, glutamate, ascorbate, camphorate et camphorsulfonate.

8. Composition pharmaceutique comprenant une quantité thérapeutiquement efficace du composé de formule I selon la revendication 1, ou le tautomère de celui-ci, ou le sel pharmaceutiquement acceptable de celui-ci, ou le composé deutéré de celui-ci, et un ou plusieurs véhicules, diluants ou excipients pharmaceutiquement acceptables.

9. Composé de formule I selon la revendication 1, ou le tautomère de celui-ci, ou le sel pharmaceutiquement acceptable de celui-ci, ou le composé deutéré de celui-ci, ou la composition pharmaceutique comprenant le composé représenté par la formule I, pour une utilisation dans la prévention et/ou le traitement des maladies médiées par ROS1, NTRK et ALK, ou les kinases résistantes aux médicaments de ceux-ci.

10. Composé pour utilisation selon la revendication 9, dans lequel la maladie médiée par ROS1, NTRK et ALK, ou les kinases résistantes aux médicaments de ceux-ci, est choisie parmi le cancer, le sarcome ou la douleur.

11. Composé pour utilisation selon la revendication 10, dans lequel le cancer est l'un quelconque parmi le cancer du sein, le cancer du col de l'utérus, le cancer du côlon, le cancer du poumon, le cancer de l'estomac, le cancer rectal, le cancer du pancréas, le cancer du cerveau, le cancer de la peau, le cancer buccal, le cancer de la prostate, le cancer des os, le cancer du rein, le cancer de l'ovaire, le cancer de la vessie, le cancer du foie, la tumeur des trompes de Fallope, la tumeur péritonéale, le mélanome, le gliome, le glioblastome, le cancer de la tête et du cou, le néphrome mastoïde, la leucémie, le lymphome, le myélome et la tumeur thyroïdienne.
